# EUROPEAN PATENT SPECIFICATION

(11) **EP 0 355 827 B1**
(45) Date of publication and mention of the grant of the patent: **02.01.1997**
(21) Application number: 89115635.8
(22) Date of filing: 24.08.1989
(51) Int. Cl.: C07D 409/12, A61K 31/415, C07D 405/12, C07D 417/12, C07D 403/12, C07D 413/12, C07D 401/12, C07D 235/28, C07D 333/62, C07D 333/34, C07D 307/82

(54) **Hydantoin derivatives**
Hydantoin-Derivate
Dérivés d'hydantoine

(30) Priority: 24.08.1988 US 235557; 25.02.1989 JP 43422/89
(43) Date of publication of application: 28.02.1990
(73) Proprietor: MOCHIDA PHARMACEUTICAL CO., LTD., Shinjuku-ku Tokyo 160 (JP)
(72) Inventor: Mochida, Ei, Tokyo (JP); Murakami, Kimihiro, Suntou-gun Shizuoka-ken (JP); Kato, Kazuo, Mishima-shi Shizuoka-ken (JP); Kato, Katsuaki, Tokyo (JP); Okuda, Jun, Nagoya-shi Aichi-ken (JP); Miwa, Ichitomo A-305, Miyukiyama-Park-Mansion, Nagogya-shi Aichi-ken (JP)
(74) Representative: Hansen, Bernd, Dr.Dipl.-Chem.

(56) References cited:
- EP-A- 0 173 497
- EP-A- 0 187 387
- EP-A- 0 305 947
- FR-M- 6 097
- US-A- 3 384 643
- US-A- 4 575 507
- US-A- 4 743 611
- CHEMICAL ABSTRACTS, vol. 97, no. 1, 5th July 1982, pages 657, abstract no. 6774e, Columbus, Ohio, US; A.M. EL-NAGGAR et al.: "Synthesis of thiophene-2-sulfonyl - amino acid and dipeptide derivatives", & EGYPT. J. CHEM. 1981, 23(4), 273-9
- CHEMICAL ABSTRACTS, vol. 95, no. 15, 12th October 1981, page 658, abstract no. 132725m, Columbus, Ohio, US; M.A. EL-MAGHRABY et al.: "Synthesis of new heterocyclic sulfonamides", & INDIAN J. CHEM., SECT. B 1981, 20B(3), 256-7

## Description

### BACKGROUND OF THE INVENTION

The present invention relates to novel hydantoin derivatives, processes for producing hydantoin derivatives, pharmaceutical compositions containing at least one of said hydantoin derivatives as aldose reductase inhibitors and novel intermediate compounds in the synthesis of said hydantoin derivatives.

Cataract, peripheral neuropathy, retinopathy and nephropathy associated with diabetes mellitus result from abnormal accumulation of polyol metabolites converted from sugars by aldose reductase. For example, sugar cataract results from damage of lens provoked by change in osmotic pressure induced by abnormal accumulation of polyol metabolites converted from glucose or galactose by aldose reductase in lens [see J. H. Kinoshita et al., Biochim. Biophys. Acta, 158, 472 (1968) and cited references in the report ]. And some reports were submitted about undesirable effect of abnormal accumulation of polyol metabolites in lens, peripheral nerve cord and kidney of the diabetic animals [ see A. Pirie et al. Exp. Eye Res., 3, 124 (1964) ; L. T. Chylack Jr. et al., Invest. Opthal., 8, 401 (1969) ; J. D. Ward et al., Diabetologia, 6, 531 (1970) ]. Consequently, it is important to inhibit aldose reductase as strongly as possible for treating and/or preventing diabetic complications mentioned above. Although several compounds have been offered as aldose reductase inhibitors, none of them is fully sufficient in inhibitory activity against the enzyme. Therefore, it has been desired to develop new compounds having a stronger inhibitory activity against aldose reductase.

### SUMMARY OF THE INVENTION

An object of the present invention is to provide novel hydantoin derivatives and salts, solvates and solvates of salts thereof.

Another object of the present invention is to provide processes for producing said novel hydantoin derivatives.

A further object of the present invention is to provide pharmaceutical compositions comprising at least one of said novel hydantoin derivatives having an inhibitory activity against aldose reductase.

A further object of the present invention is to provide novel intermediate compounds in the synthesis of said novel hydantoin derivatives.

The present inventors previously found that sulfonylhydantoin derivatives had a strong inhibitory activity against aldose reductase and accomplished an invention on aldose reductase inhibitors (JP kokai 58 109418, 62 67075, 62 201873 and 1 61465). And M. S. Malamas et al. U.S. Pat. No. 4,743,611 disclosed naphthalenesulfonyl hydantoin derivatives useful as aldose reductase inhibitors. And Ohishi et al. disclosed benzofuranylsulfonyl glycine derivatives useful as drugs of treatment of diabetic complications (JP Kokai 62 155269).

Furthermore, the present inventors have made extensive researches on a series of compounds having an inhibitory activity against aldose reductase and found novel hydantoin derivatives having an extremely strong inhibitory activity against aldose reductase. They are extremely useful for the treatment and/or prevention of various forms of diabetic complications based on the accumulation of polyol metabolites.

### DETAILED DESCRIPTION OF THE INVENTION

As a result of extensive investigations concerning development of hydantoin derivatives having a satisfactory inhibitory activity against aldose reductase, the present inventors have found that novel hydantoin derivatives represented by the formula (I) satisfy this requirement and have accomplished the present invention.

The present invention is based on the selection of a hydantoin which is bonded through a sulfonyl group to various substituents at the 1-position of the hydantoin skeleton.

The present invention is directed to novel hydantoin derivatives represented by the formula (I): 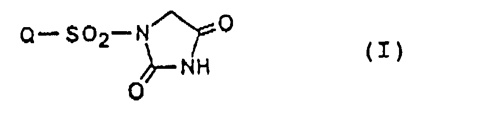 and non-toxic salts, solvates and solvates of non-toxic salts thereof; wherein Q represents a mono- or a fused heterocyclic group which may be substituted by one or more substituents which are same or different and selected from a group consisting of a halogen atom, a lower alkyl group, a nitro group, a cyano group, an optionally protected carboxy group, an optionally protected carboxymethyl group, a halogenated lower alkyl group, a lower alkylthio group, a lower alkylcarbonyl group, a lower alkoxy group, a lower alkylsulfinyl group, a lower alkylsulfonyl group, an optionally protected hydroxy group, an optionally protected amino group, a carbamoyl group and a phenyl group.

The present invention is also directed to the process for preparing above-mentioned hydantoin derivatives.

The present invention is further directed to pharmaceutical compositions characterized by containing at least one of these hydantoin derivatives as active component(s).

The present invention is further directed to novel intermediate compounds in the synthesis of above-mentioned hydantoin derivatives.

Compounds of the present invention and non-toxic salts, solvates and solvates of non-toxic salts thereof represents a satisfactory inhibitory activity against aldose reductase and a preventing activity against cataracts and neuropathy in experimental animal models.

Compounds of the present invention and non-toxic salts, solvates and non-toxic salts thereof are free of central nervous system side effects such as anticonvulsant activity and low toxicity, so useful for the treatment and/or prevention of various forms of diabetic complications such as neuropathy, autonomic disease, cataract, retinopathy, neuropathy and microvascular disease.

In the hydantoin derivatives of the present invention represented by the general formula (I), it is known that the hydantoin moiety exhibits tautomerism as shown below:

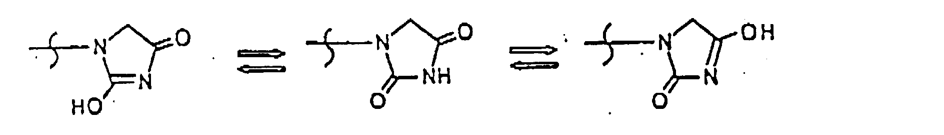

Since these tautomeric isomers are generally deemed to be the same substance, the compounds of the present invention represented by the formula (I) also include all of these tautomeric isomers.

The compounds represented by the formula (I) may form salts with base. Typical examples of salts with base of the compounds represented by the formula (I) include pharmaceutically acceptable salts such as alkali metal salts (such as sodium salts, potassium salts, etc.), alkaline earth metal salts (such as magnesium salts, calcium salts, etc.), salts with organic bases (such as ammonium salts, benzylamine salts, diethylamine salts, etc.) or salts of amino acids (such as arginine salts, lysine salts, etc.). These salts of the compounds represented by the formula (I) may be mono-salts or di-salts which may be salts of the hydantoin moiety and/or salts of the carboxy group contained in the Q group.

The compounds represented by the formula (I) may also form acid addition salts. Typical example of acid addition salts of the compounds represented by the formula (I) include pharmaceutically acceptable salts, such as salts of inorganic acids (such as hydrochlorides, hydrobromides, sulfates, phosphates, etc.), salts of organic acids (such as acetates, citrates, maleates, tartrates, benzoates, ascorbate, ethenesulfonates, toluenesulfonates, etc.) or salts of amino acids (such as aspartates, glutamates, etc.). These salts of the compounds represented by the formula (I) may be salts of the heterocyclic moiety in the Q group.

In the compounds of the present invention represented by the formula (I), term "lower" can be defined more specifically as a straight or branched chain having 1 to 4 carbon atoms.

In the compounds of the present invention represented by the formula (I), the heterocyclic group can be defined as a mono heterocyclic group such as pyrrolyl, pyrrolinyl, imidazolyl, pyrazolyl, triazolyl, tetrazolyl, oxazolyl, isoxazolyl, oxadiazolyl, thiazolyl, isothiazolyl, thiazolinyl, thiadiazolyl, thiatriazolyl, thienyl, furyl, pyrrolidinyl, imidazolidinyl, thiazolidinyl, pyridyl or its N-oxide, pyrazinyl, pyrimidinyl, pyridazinyl, piperidyl, piperazinyl, morpholinyl, triazinyl, etc., or a fused heterocyclic group such as indolyl, isoindolyl, benzimidazolyl, quinolyl, isoquinolyl, quinazolinyl, cinnolinyl, phthalazinyl, quinoxalinyl, indazolyl, benzotriazolyl, benzoxazolyl, benzoxadiazolyl, benzothiazolyl, benzothiadiazolyl, benzisoxazolyl, benzisothiazolyl, benzothienyl (benzo[b]thienyl or benzo[c]thienyl), tetrahydrobenzothienyl, benzofuranyl (benzo[b]furanyl or isobenzofuranyl), chromenyl, chromanyl, coumarinyl, chromonyl, triazolopyridyl, tetrazolopyridyl, purinyl, thiazolopyrimidinyl, triazolopyrimidinyl, thiadiazolopyrimidinyl, thiazolopyridazinyl, naphthyridinyl, xanthenyl, phenoxathiinyl, phenoxazinyl, phenothiazinyl, carbazolyl, etc., preferably indolyl, benzimidazolyl, benzotriazolyl, benzothiazolyl, benzisoxazolyl, benzisothiazolyl, benzothienyl, tetrahydrobenzothienyl, benzofuranyl, coumarinyl, chromonyl, more preferably benzo[b]thienyl or benzo[b]furanyl. The above-mentioned heterocyclic groups may be substituted with a group such as a lower alkyl group (such as methyl, ethyl, isopropyl, tert-butyl, etc.), a lower alkylcarbonyl group (such as acetyl, propanoyl, butanoyl, etc.), a lower alkoxy group (such as methoxy, ethoxy, isopropoxy, tert-butoxy, etc.), a phenyl group, a cyano group, a carbamoyl group, an optionally protected carboxy group, an optionally protected carboxymethyl group, a nitro group, a halogenated lower alkyl group (such as trifluoromethyl, pentafluoroethyl, etc.), an optionally protected hydroxy group, an optionally protected amino group, (such as acyl amino, etc.), a lower alkylthio group, a lower alkylsulfinyl group, a lower alkyl sulfonyl group or a halogen atom (such as fluoro, chloro, bromo, iodo etc.), or combination of any of these groups.

In a mono- heterocyclic group, a compound unsubstituted or substituted with 1 or 2 substituents which are the same or different and selected from a group consisting of a halogen atom or a phenyl group, is preferable.

In a fused heterocyclic group, a compound unsubstituted or substituted with 1 to 3 substituents which are the same or different and selected from a group consisting of a halogen atom, a lower alkyl group, a halogenated lower alkyl group, a lower alkylthio group or a cyano group, is preferable.

When a fused heterocyclic group is a benzo[b]-furan-2-yl group which may be substituted, the said substituents are preferably 1 to 3 halogen atoms.

The compounds of the present invention represented by the formula (I) can be produced by the processes described as follows.
Namely;

The starting material of sulfonyl halide represented by the formula (II):

Q-SO₂-Y (II)

wherein Q represents a mono- or a fused heterocyclic group which may be substituted by one or more substituents which are same or different and selected from a group consisting of a halogen atom, a lower alkyl group, a nitro group, a cyano group, an optionally protected carboxy group, an optionally protected carboxymethyl group, a halogenated lower alkyl group, a lower alkylthio group, a lower alkylcarbonyl group, a lower alkoxy group, a lower alkylsulfinyl group, a lower alkylsulfonyl group, an optionally protected hydroxy group, an optionally protected amino group, a carbamoyl group and a phenyl group and Y represents a halogen atom, is prepared as follows.

A compound Q-H wherein Q has the same significance as defined above and H represents a hydrogen atom is reacted with a base (such as n-butyllithium or lithium diisopropylamide, etc.) and sulfur dioxide and then reacted with a halogenating reagent (such as chlorine, bromine, phosphorus pentachloride, thionyl chloride, N-chlorosuccinimide or N-bromosuccinimide, etc.) to obtain an objective compound.

Further, Q-H wherein Q has the same significance as defined above is reacted with a halosulfonic acid (preferably chlorosulfonic acid, etc.) to obtain directly an objective compound.

Further, a sulfonic acid derivative of Q-H ( Q-SO₃H) wherein Q has the same significance as defined above is reacted with sodium bicarbonate to give a corresponding salt, and then reacted with a halogenating reagent (such as phosphorus pentachloride, thionyl chloride or thionyl bromide, etc.) to obtain an objective compound.

Further, a S-benzyl derivative of Q-H ( Q-S-CH₂C₆H₅ ) wherein Q has the same significance as defined above is reacted with a halogenating reagent (such as chlorine, etc.) to obtain an objective compound.

Further, an amine derivative of Q-H ( Q-NH₂ ) wherein Q has the same significance as defined above is reacted with a nitrite salt (such as sodium nitrite, etc.), and then reacted with sulfur dioxide and a halogenating reagent (such as copper (I) chloride or copper (II) chloride, etc.) to obtain an objective compound.

The sulfonyl halide derivative, obtained above mentioned procedure is reacted with a glycine derivative represented by the formula (III):

NH₂CH₂CO-R (III)

wherein R represents a hydroxy group, an alkoxy group or an amino group which may be substituted by an alkoxycarbonyl group, to give the corresponding sulfonylglycine derivative represented by the formula (IV):

Q-SO₂NHCH₂CO-R (IV)

wherein Q and R have the same significance as defined above. Compounds of this kind are for example known from Int. J. Pept. Protein Res. 19 (1982) 408; Egypt J. Chem. 23 (1981) 273; Indian J. Chem. Sect. B 20B (1981) 256; Yakugaku Zasshi 106 (1986) 1008; Farmaco. Ed.Sci. 41 (1986) 41; J.Med.Chem. 30 (1987) 678; J.Heterocycl.Chem. 21 (1984) 1017; EP-A-0 103 142; Egypt J. Chem. 23 (1981) 273; EP-A-0 21 058; DE-A-25 37 070; DE-A-28 38 851; EP-A-0 187 387; J.Serb.Chem.Soc. 52 (1987) 529; Farmaco Ed.Sci. 41 (1986) 729; and Indian J. Chem. Sect. B 15B (1977) 245. Such a condensation reaction is carried out generally in an aqueous solution, in an organic solvent (such as dichloromethane, chloroform, dioxane, tetrahydrofuran, acetonitrile, ethyl acetate, acetone, N,N-dimethylformamide, etc.) or in a mixed solvent of an aqueous solution and an organic solvent, preferably in the presence of deacidifying agent. As the deacidifying agent, triethylamine, diethylaniline, pyridine, etc. is employed in the organic solvent system, and in the aqueous system, aqueous alkali (such as sodium carbonate, sodium bicarbonate, potassium carbonate, sodium hydroxide, etc.) is employed. The condensation reaction is carried out at temperatures ranging from about -20 to 80°C, preferably 0°C to room temperature.

When R represents an amino group in the formula (IV), the sulfonylglycine derivative is represented by the formula (V):

Q-SO₂NHCH₂CONH₂ (V)

wherein Q has the same significance as defined above.

The sulfonylglycine derivative represented by the formula (V) is cyclized using a haloformic acid ester (such as methyl chloroformate, ethyl chloroformate, etc.) in the presence of a base (such as sodium hydride, potassium hydride, butyllithium, etc.) to give the corresponding hydantoin derivative of the present invention represented by the formula (I). The cyclization reaction is carried out generally in an inert solvent (such as N,N-dimethylformamide, dimethylsulfoxide, ethyl ether, tetrahydrofuran, dioxane, dichloromethane, etc.) and at temperatures ranging from about -20 to 120°C, preferably 0 to 80°C.

When R represents an amino group protected with an alkoxycarbonyl group, the sulfonylglycine derivative is cyclized in the presence of a base (such as sodium hydride etc.) to give the corresponding hydantoin derivative of the present invention represented by the formula (I).

When R represents a hydroxy group or an alkoxy group in the formula (IV), the sulfonylglycine derivative is represented by the formula (VI):

Q-SO₂NHCH₂CO-R¹ (VI)

wherein Q has the same significance as defined above and R¹ represents a hydroxy group or an alkoxy group. The sulfonylglycine derivative represented by the formula (VI) is cyclized with a thiocyanate derivative (such as ammonium thiocyanate, potassium thiocyanate, etc.) in the presence of an acid anhydride (such as acetic anhydride, propionic anhydride, etc.) and, if necessary and desired, a base (such as pyridine, triethylamine, etc.) to give the corresponding 2-thiohydantoin derivative. If necessary and desired, the cyclization reaction is carried out after hydrolysis of ester when R¹ represents an alkoxy group. The cyclization reaction is carried out generally in an inert solvent (such as pyridine, triethylamine, N,N-dimethylformamide, dimethylsulfoxide, etc.) and at temperatures ranging from 0 to 120 °C, preferably room temperature to 100°C. Further, the 2-thiohydantoin derivative obtained by said cyclization is oxidized using oxidizing agent (such as nitric acid, chlorine, iodine chloride, potassium permanganate, hydrogen peroxide, dimethylsulfoxide-sulfuric acid, etc.) to give the corresponding hydantoin derivatives of the present invention represented by the formula (I).

To demonstrate the utility of the compounds of the present invention, experimental examples of representative compounds are shown below.

Compounds in the present invention
- Compound 1:: 1-(benzo(b]thien-2-ylsulfonyl)hydantoin
- Compound 2:: 1-(3-chlorobenzo[b]thien-2-yl-sulfonyl)hydantoin
- Compound 3:: 1-(5-chlorobenzo[b]thien-2-yl-sulfonyl)hydantoin
- Compound 4:: 1-(benzo[b]furan-2-ylsulfonyl)hydantoin
- Compound 5:: 1-(5-chlorobenzo[b]furan-2-ylsulfonyl)hydantoin
- Compound 6:: 1-(5-bromobenzo[b]furon-2-ylsulfonyl)hydantoin
- Compound 7:: 1-(benzothiazol-2-ylsulfonyl)hydantoin
- Compound 8:: 1-(coumarin-6-ylsulfonyl)hydantoin
- Compound 9:: 1-(2,5-dichlorothien-3-ylsulfonyl)hydantoin
- Compound 10:: 1-(4,5-dibromothien-2-ylsulfonyl)hydantoin
- Compound 11:: 1-(6-chlorobenzo[b]thien-2-yl-sulfonyl)hydantoin
- Compound 12:: 1-(7-chlorobenzo[b]thien-2-yl-sulfonyl)hydantoin
- Compound 13:: 1-(3-isopropylbenzo[b]thien-2-yl-sulfonyl)hydantoin
- Compound 14:: 1-(3-trifluoromethylbenzo[b]thien-2-ylsulfonyl)hydantoin
- Compound 15:: 1-(3-bromobenzo[b]thien-2-yl-sulfonyl)hydantoin
- Compound 16:: 1-(3-methoxybenzo[b]thien-2-yl-sulfonyl)hydantoin
- Compound 17:: 1-(3-methylsulfonylbenzo[b]thien-2-ylsulfonyl)hydantoin
- Compound 18:: 1-(3-cyanobenzo[b]thien-2-yl- sulfonyl)hydantoin
- Compound 19:: 1-(3-bromo-7-fluorobenzo[b]thien-2-ylsulfonyl)hydantoin
- Compound 20:: 1-(2-chlorobenzo[b]thien-3-yl-sulfonyl)hydantoin
- Compound 21:: 1-(4-iodobenzo[b]furan-2-ylsulfonyl)hydantoin
- Compound 22:: 1-(4,6-dichlorobenzo[b]furan-2-yl-sulfonyl)hydantoin
- Compound 23:: 1-(3-bromobenzo[b]furen-2-ylsulfonyl)hydantoin
- Compound 24:: 1-(5-fluorobenzo[b]thien-2-yl-sulfonyl)hydantoin
- Compound 25:: 1-(4-chlorobenzo[b]thien-2-yl-sulfonyl)hydantoin
- Compound 26:: 1-(benzo[b]isothiazol-3-ylsulfonyl)hydantoin
- Compound 27:: 1-(5-nitrobenzo[b]thien-2-yl-sulfonyl)hydantoin
- Compound 28:: 1-(5-carboxybenzo[b]thien-2-yl-sulfonyl)hydantoin
- Compound 29:: 1-(4,5-dichlorobenzo[b]furan-2-yl-sulfonyl)hydantoin
- Compound 30:: 1-(5,6-dichlorobenzo[b]furan-2-yl-sulfonyl)hydantoin
- Compound 31:: 1-(3-bromo-4,6-dichlorobenzo[b]furan-2-ylsulfonyl)hydantoin
- Compound 32:: 1-(3-chlorobenzo[b]furan-2-ylsulfonyl)hydantoin
- Compound 33:: 1-(7-fluorobenzo[b]furan-2-ylsulfonyl)hydantoin
- Compound 34:: 1-(3-bromo-7-fluorobenzo[b]furan-2-yl-sulfonyl)hydantoin

Reference compounds
- Compound A:: sorbinil : [(S)-6-Fluoro-2,3-dihydrospiro(4H-1-benzopyran-4,4'-imidazolidine)-2', 5'-dione] ( synthesized by the method of R. S. sarges et al. : see J. Med. Chem., 28, 1716 (1985) )

### Experimental Example 1

The inhibitory activities of hydantoin derivatives on bovine lens aldose reductase were measured according to the procedure of Inagaki et al. (K. Inagaki et al., Arch. Biochem. Biophys., 216, 337 (1982)) with slight modifications. Assays were performed in 0.1 M phosphate buffer (pH 6.2) containing 0.4 M ammonium sulfate, 10 mM DL-glyceraldehyde, 0.16 mM nicotinamide adenine dinucleotide phosphate, reduced form ( NADPH ) and aldose reductase (0.010-0.016 units) in a total volume of 1.0 ml. To this mixture was added 10 µl of the solution of each hydantoin derivative to be tested, and the decrease in absorbance at 340 nm was measured with a spectrophotometer.

The concentrations of typical hydantoin derivatives of the present invention required to produce 50% inhibition are shown in table 1.

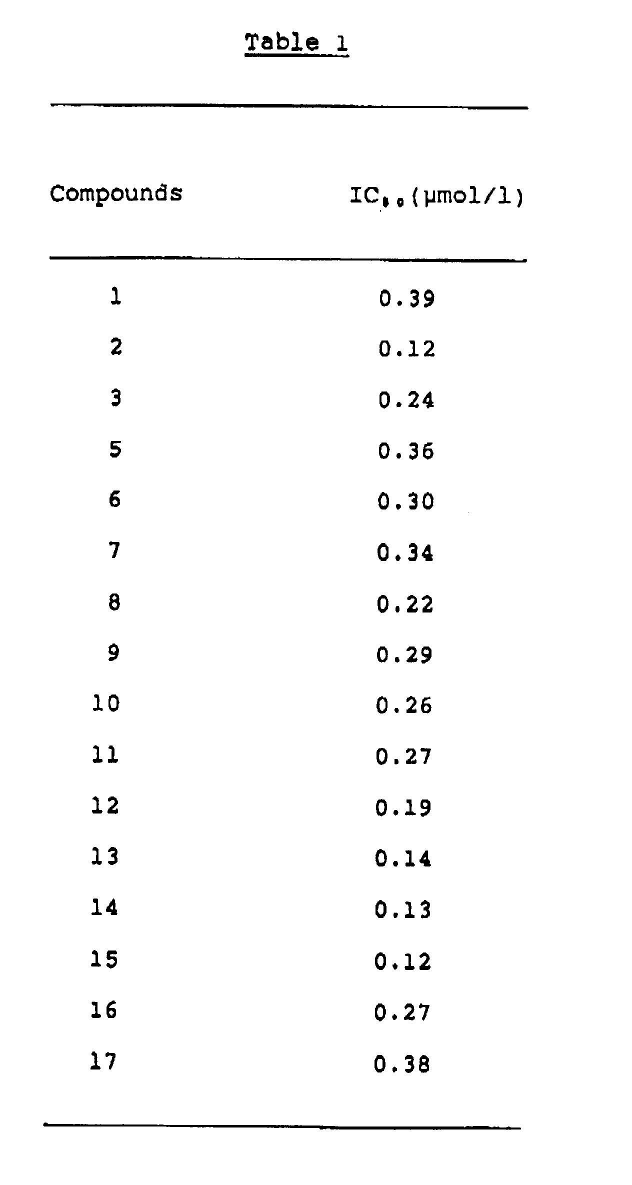

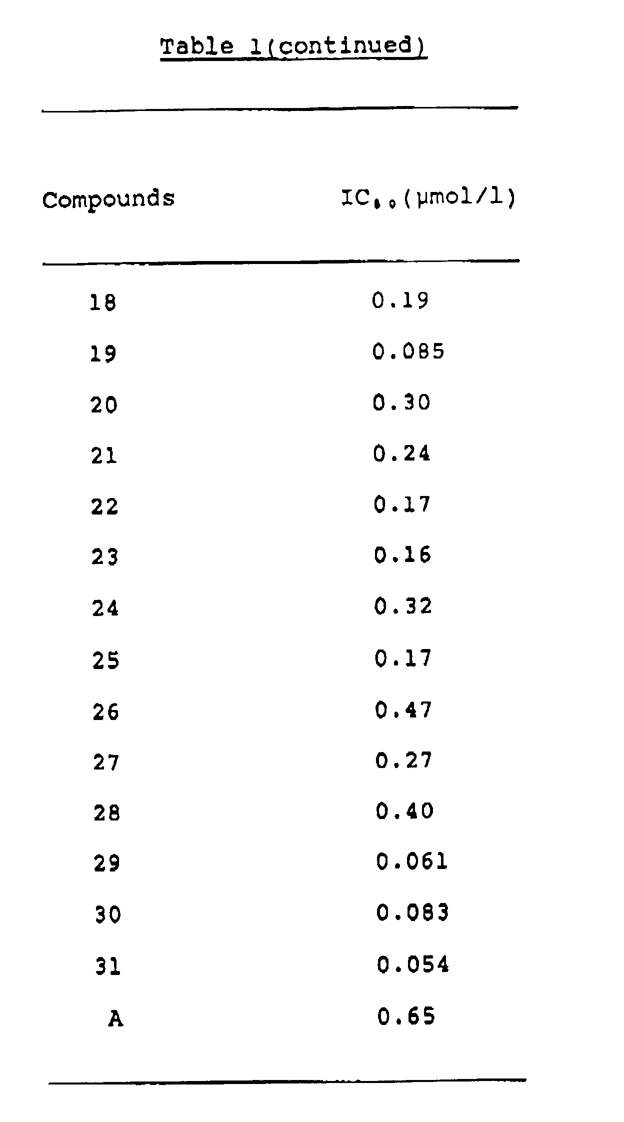

Compounds 1 to 31 of the present invention showed stronger inhibitory activities against aldose reductase than reference compound A did. Above all, several compounds were ten times or more potent than reference compound A.

### Experimental Example 2

Hydantoin derivatives of the present invention were examined for acute toxicity. Groups of 5 ICR strain mice were orally administered with compound 1, 2, 4, 5, 6, 9, 23, 25, 29, 30, 32, 33 or 34 of the present invention in a dose of 1 g/kg, and no change was observed in any of the groups over the one-week period after the administration.

Since the compounds of the present invention have strong inhibitory activities against aldose reductase, show lower toxicity and show stronger preventing activities against cataracts and neuropathy in animal models than known compounds, pharmaceutical compositions containing at least one of these compounds as active component(s) are useful for the treatment and/or prevention of diabetic complications based on the accumlation of polyol metabolites.

The hydantoin derivatives provided by the present invention can be employed as pharmaceutical compositions, for example, in the form of pharmaceutical compositions containing hydantoin derivatives together with appropriate pharmaceutically acceptable carrier or medium such as sterilized water, edible oils, non-toxic organic solvents or non-toxic solubilizer such as glycerin or propylene glycol. They may be mixed with excipients, binders, lubricants, coloring agents, corrigents, emulsifying agents or suspending agents such as Tween 80 or arabic gum to prepare tablets, capsules, powders, granules, subtilized granules, syrups, eye drops, suppositories, ointments, inhalants, aqueous or oily solutions or emulsion or suspensions for injections. These agents can be administered either orally or parenterally (such as intravenous administration, intramuscular administration, subcutaneous administration, intrarectal administration, percutaneous administration or permucosal administration etc.), and the amount of administration may be in the range of 1 to 3000 mg/day, preferably 10 to 500 mg/day when the preparation is tablets, capsules, powders, injections, suppositories, syrups, inharants or ointments, 1 µg to 10 mg/day, preferably 10 µg to 1 mg/day when the preparation is eye drops, and 1 to 10 % composition when the preparation is ointments, and may also be adjusted according to the patient conditions and can administered once or divided 2 to 6 times or by instillation, etc.

Hereafter the present invention will be described with references to the examples below but is not deemed to be limited thereof.

### Example 1

### preparation of 1-(benzo[b]thien-2-ylsulfonyl)hydantoin (compound 1).

### Step 1

### Preparation of benzo[b]thien-2-ylsulfonyl chloride.

To a solution of benzo[b]thiophen (38.3 g) in anhydrous ether (180 ml) was added dropwise 1.6 M solution of n-butyllithium in hexane (220 ml) under ice-cooling and nitrogen atmosphere. After refluxing for 40 minutes, into the solution was bubbled sulfur dioxide for 2.75 hours with stirring at -30°C. Then the solution was stirred for 1 hour and the formed precipitate was separated by filtration to give lithium benzo[b]thien-2-ylsulfinate. Into the suspension of the product in concentrated hydrochloric acid (400 ml) and water (100 ml) was bubbled chlorine gas for 1.5 hours with stirring at -5°C. The resulting solution was poured into ice-water (500 ml) and extracted with dichloromethane (1.5 1 x 2) and the organic layer was washed with saturated aqueous NaCl solution. After drying over anhydrous magnesium sulfate, dichloromethane was removed in vacuo, and the residue was purified by silica gel column chromatography to give 40.4 g of the objective compound.
- IR (KBr, cm⁻¹):: 1495, 1384, 1189, 1168, 1155
- NMR (CDCl₃, ppm):: 7.49 - 7.68 (2H, m), 7.86 - 8.03 (2H, m), 8.14 (1H, s)

### Step 2

### Preparation of N-(benzo[b]thien-2-ylsulfonyl)glycine.

To a solution of potassium carbonate (28.9 g) and glycine (15.7 g) in water (450 ml) was added benzo[b]thien-2-ylsulfonyl chloride (40.4 g) at room temperature and the mixture was stirred under reflux for 30 minutes. After cooling to room temperature, the resulting solution was acidified with 2 M hydrochloric acid to a pH in the range of 1 to 2 and the formed precipitate was separated by filtration to give 36.3 g of the objective compound.
Melting point: 171.3 - 172.4°C
- IR (KBr, cm⁻¹):: 3267, 1735, 1352, 1258, 1115
- NMR (DMSO-d₆, ppm):: 3.73 (2H, d, J - 6.0 Hz), 7.39 - 7.61 (2H, m), 7.77 - 8.13 (3H, m), 8.51 (1H, d, J = 6.0 Hz), 12.68 (1H, bs)

### Step 3

### Preparation of 1-(benzo[b]thien-2-ylsulfonyl)-2-thiohydantoin.

Anhydrous pyridine (12.6 ml), ammonium thiocyanate (10.1 g) and acetic anhydride (40 ml) were added to the product obtained in Step 2 (16.3 g) and the mixture was heated with stirring for 15 minutes at 80°C. After cooling to room temperature, the resulting solution was poured into ice-water (300 ml) and the formed precipitate was separated by filtration to give 8.6 g of the objective compound.
Melting point: 218.6°C (decomposition)
- IR (KBr, cm⁻¹):: 1759, 1374, 1255, 1171, 1157
- NMR (DMSO-d₆, ppm):: 4.74 (2H, s), 7.35 - 7.69 (2H, m), 8.04 - 8.21 (2H, m), 8.45 (1H, s), 12.72 (1H, bs)

### Step 4

### Preparation of 1-(benzo[b]thien-2-ylsulfonyl)hydantoin.

To a suspension of iodine monochloride (7.12 ml) in 1 M hydrochloric acid (200 ml) were added successively the product obtained in step 3 (8.50 g) and dichloromethane (200 ml). The mixture was stirred for 20 minutes at room temperature. After adding sodium bicarbonate (6.85 g), the reaction mixture was stirred for 15 minutes and extracted twice with ethyl acetate (1 l + 300 ml). The organic layer was washed with successive saturated aqueous sodium bisulfite solution and saturated aqueous NaCl solution. After drying over anhydrous magnesium sulfate, ethyl acetate was removed in vacuo and the residue was washed with dichloromethane to give 4.83 g of the objective compound.
Melting point: 251.8 - 254 2°C
- IR (KBr, cm⁻¹):: 3245, 1803, 1740, 1376, 1352, 1167
- NMR (DMSO-d₆, ppm):: 4.48 (2H, s), 7.51 - 7.63 (2H, m), 8.05 - 8.20 (2H, m), 8.33 (1H, s), 11.71 (1H, bs)

### Example 2

### Preparation of 1-(benzo[b]furan-2-ylsulfonyl)hydantoin (compound 4).

### Step 1

### Preparation of benzo[b]furan-2-ylsulfonyl chloride.

Starting from benzo[b]furan, the objective compound was obtained in a manner similar to Step 1 of Example 1.
IR (KBr, cm⁻¹): 1533, 1389, 1244, 1193, 1166
NMR (CDCl₃, ppm): 7.32 - 7.82 (5H, m)

### Step 2

### Preparation of N-(benzo[b]furan-2-ylsulfonyl)glycine.

Starting from benzo[b]furan-2-ylsulfonyl chloride, the objective compound was obtained in a manner similar to Step 2 of Example 1.
Melting point: 177.0 - 178.2°C
- IR (KBr, cm⁻¹):: 3289, 1724, 1347, 1162
- NMR (DMSO-d₆, ppm):: 3.77 (2H, d, J = 6.3 Hz), 7.35 - 7.81 (5H, m), 8.72 (1H, t, J = 6.3 Hz), 12.69 (1H, bs)

### Step 3

### Preparation of 1-(benzo[b]furan-2-ylsulfonyl)-2-thiohydantoin.

To a suspension of the product obtained in Step 2 (37.0 g) and ammonium thiocyanate (24.3 g) in acetic anhydride (100 ml) was added dropwise anhydrous pyridine (30.5 ml) and the mixture was heated with stirring for 1.5 hours at 70 - 80°C. After cooling to room temperature, the resulting solution was poured into ice (800 g) and the formed precipitate was separated. The precipitate was washed with water and dried to give 18.5 g of the objective compound.
Melting point: 213.0°C (decomposition)
- IR (KBr, cm⁻¹):: 3080, 1759, 1386, 1255, 1167, 1086
- NMR (DMSO-d₆, ppm):: 4.76 (2H, s), 7.34 - 8.04 (5H, m), 12.81 (1H, bs)

### Step 4

### Preparation of 1-(benzo[b]furan-2-ylsulfonyl) hydantoin.

Starting from the product obtained in Step 3, the objective compound was obtained in a manner similar to Step 4 of Example 1.
Melting point: 255.9 - 256.4°C
- IR (KBr, cm⁻¹):: 1803, 1735, 1398, 1360, 1166
- NMR (DMSO-d₆, ppm):: 4.49 (2H, s), 7.33 - 8.08 (5H, m), 11.79 (1H, bs)

Compounds of Example 3 to 39 prepared in a manner similar to Example 1 are summarized in the following table 2 together with corresponding IR and NMR data and melting points.

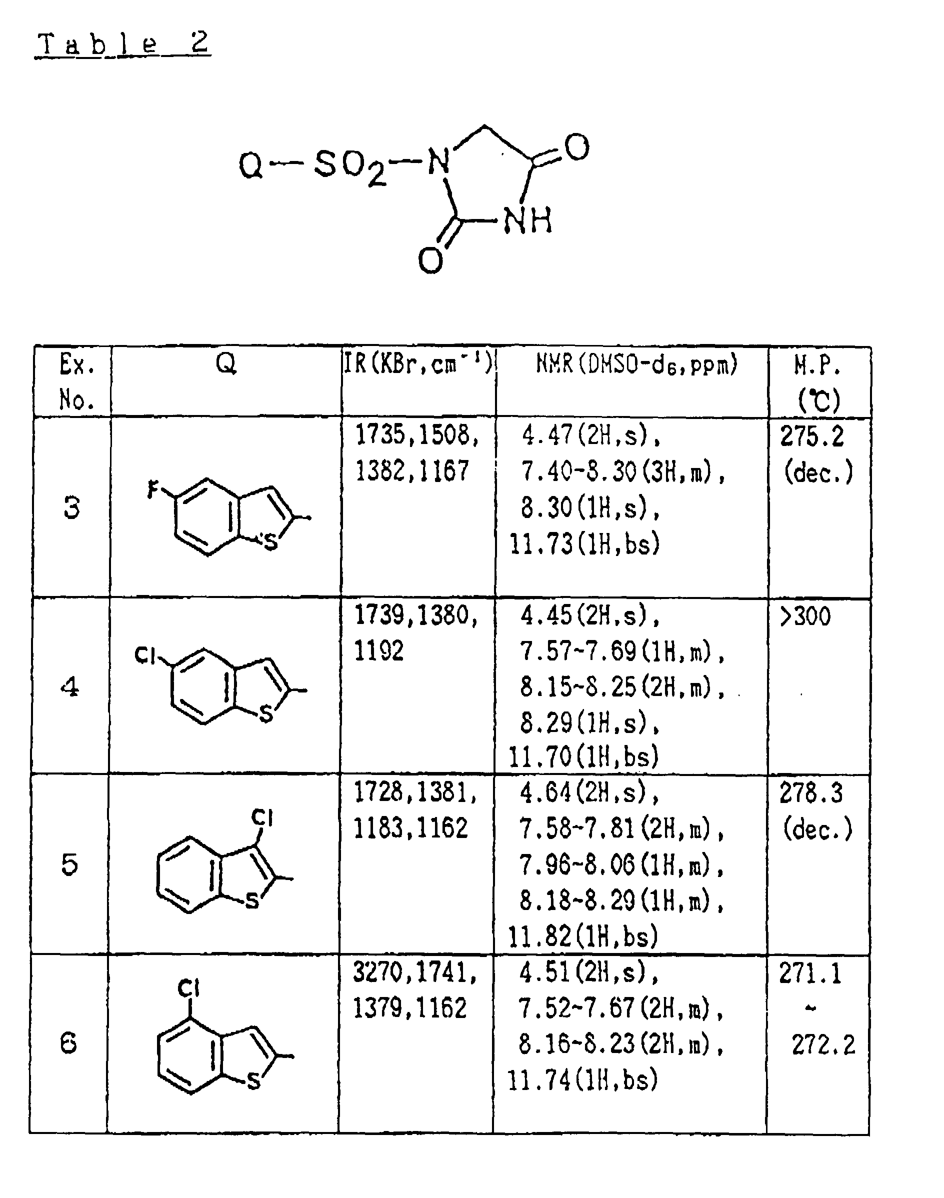

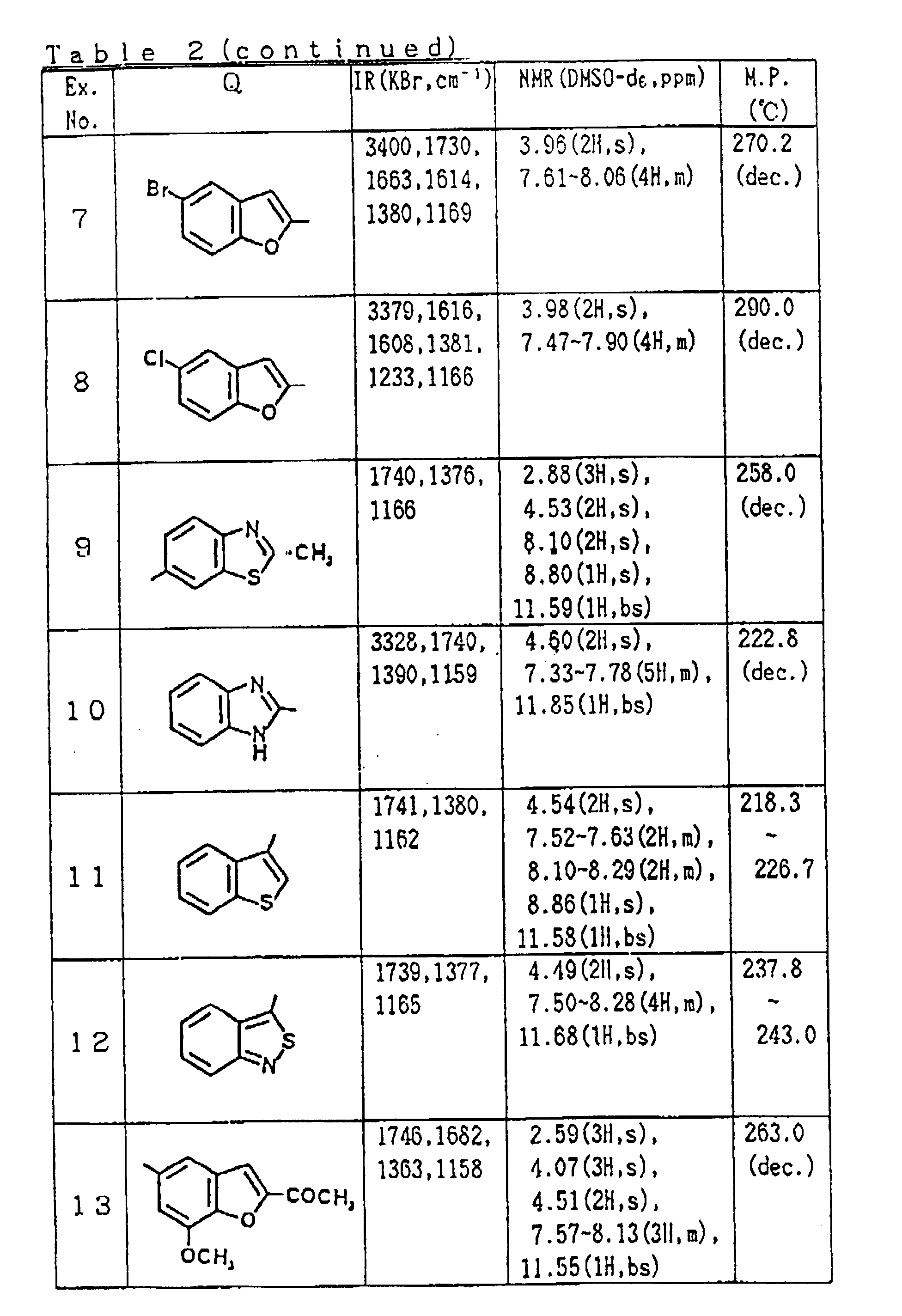

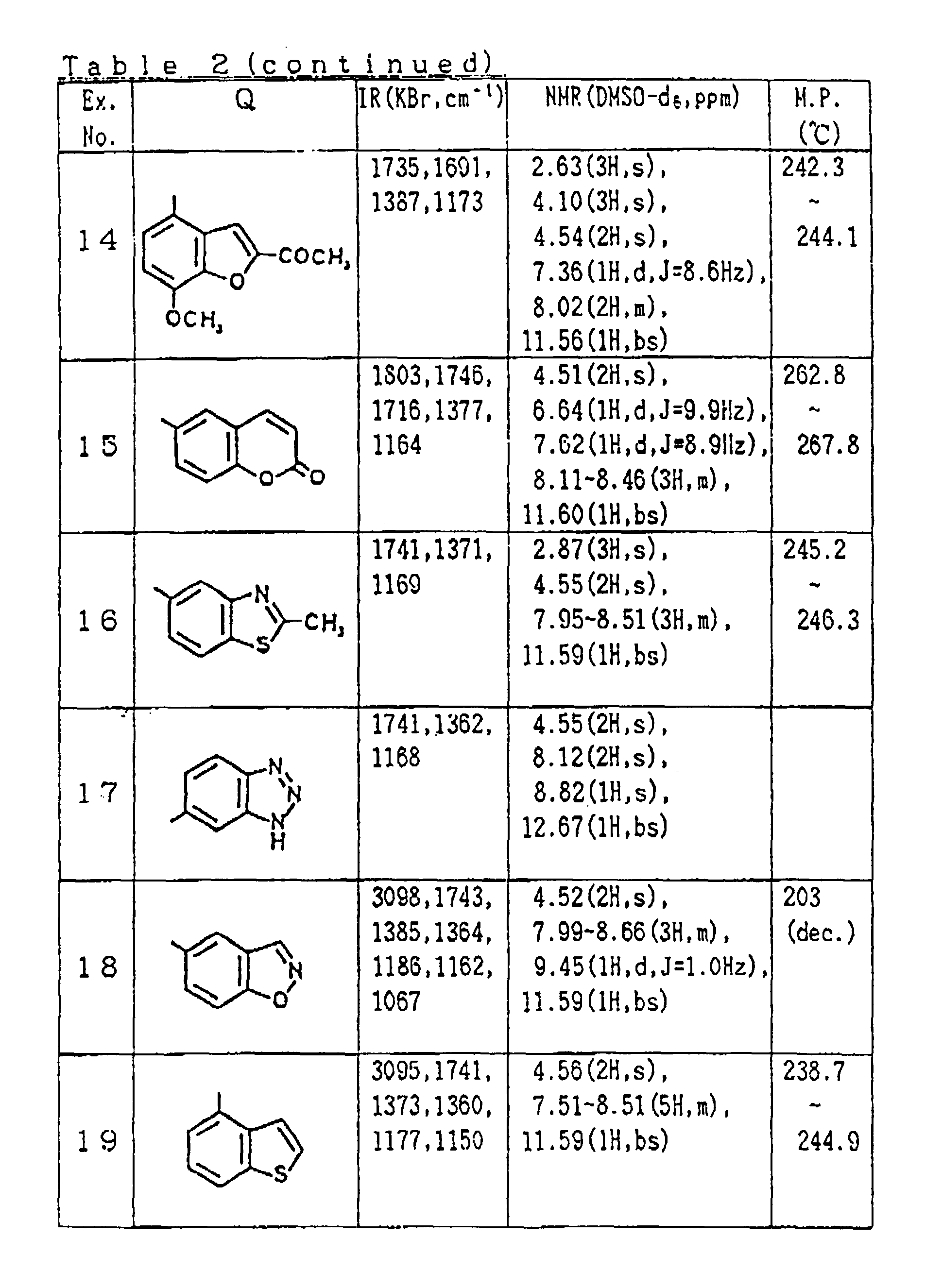

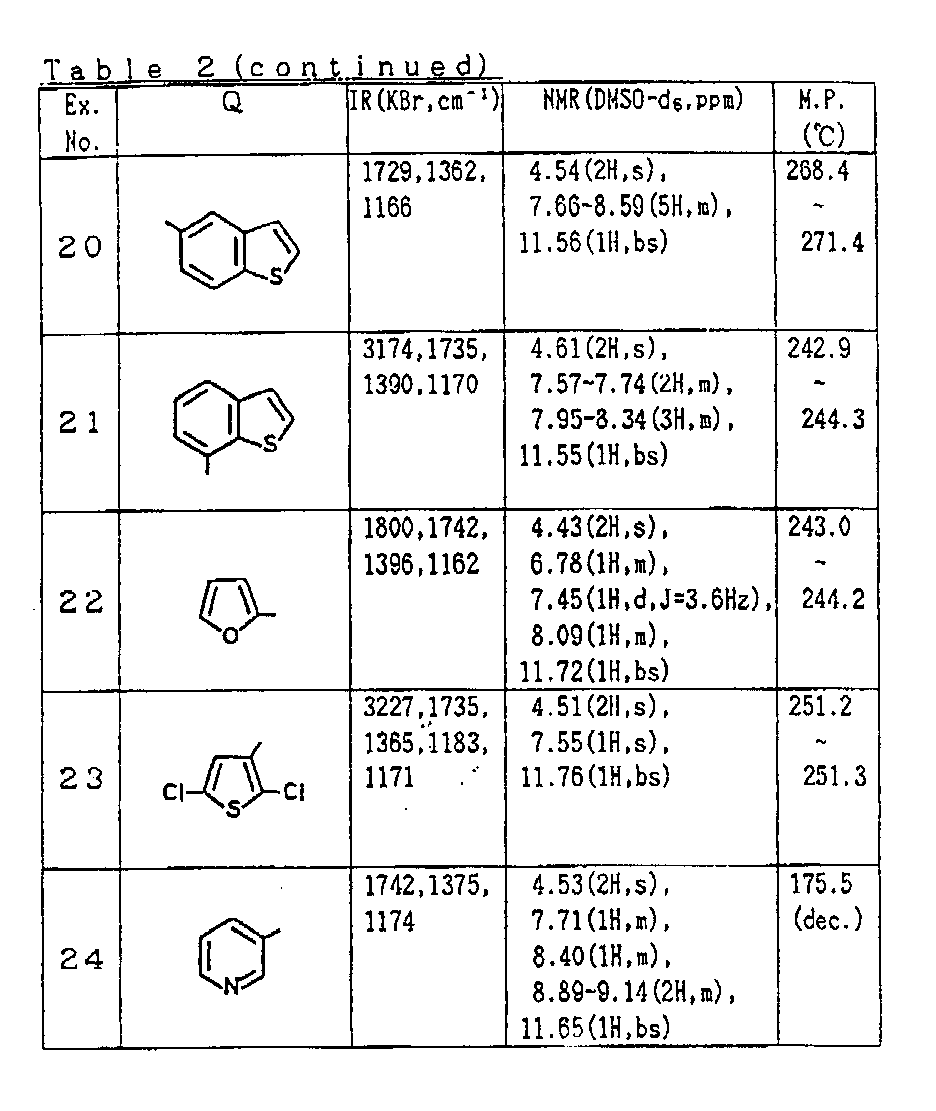

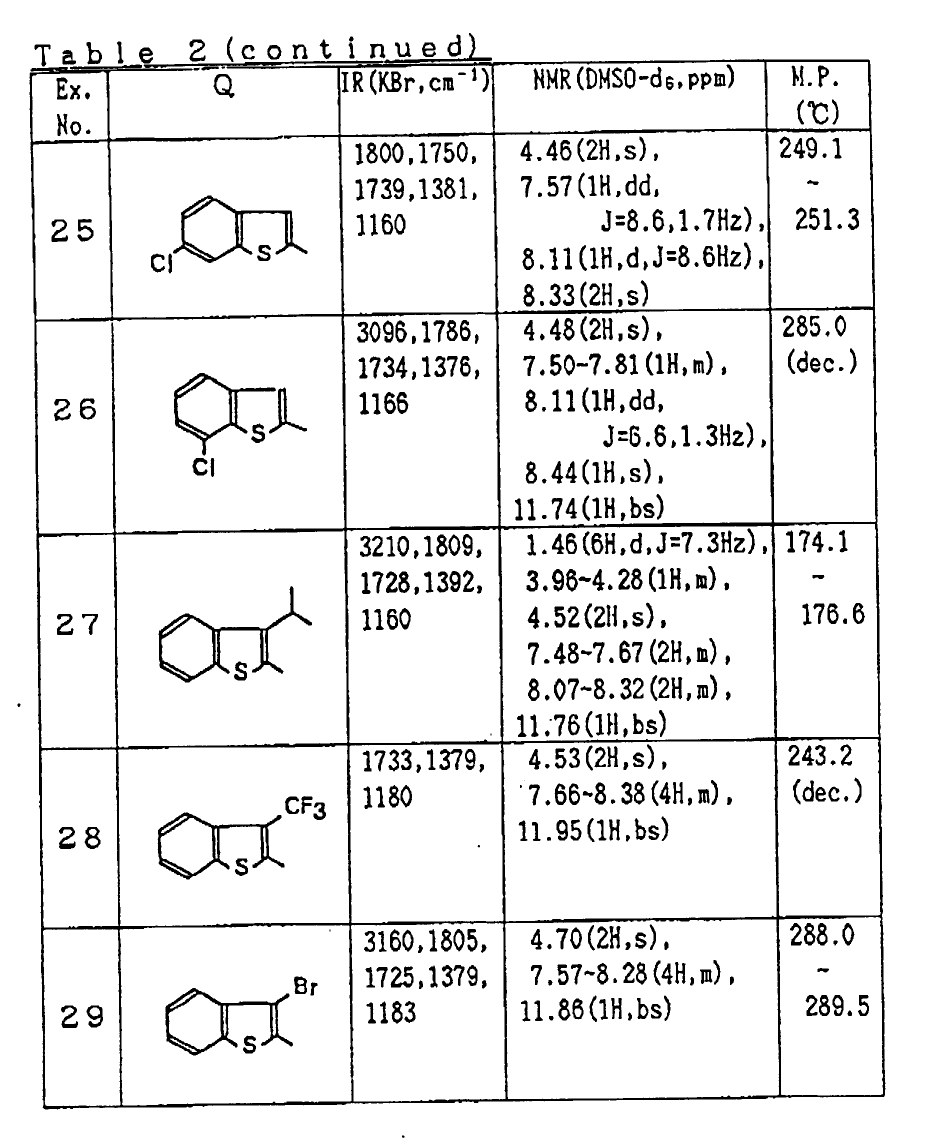

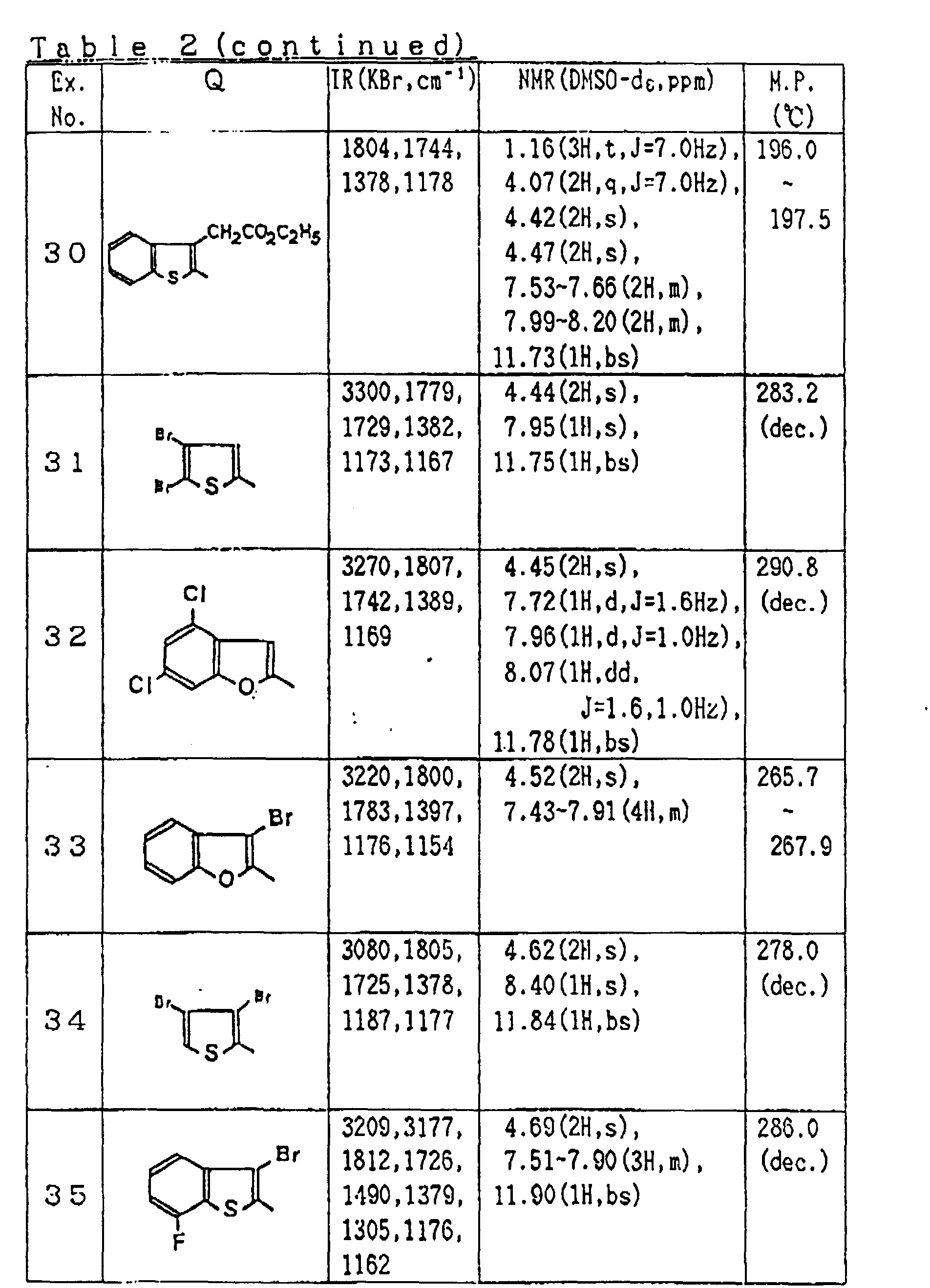

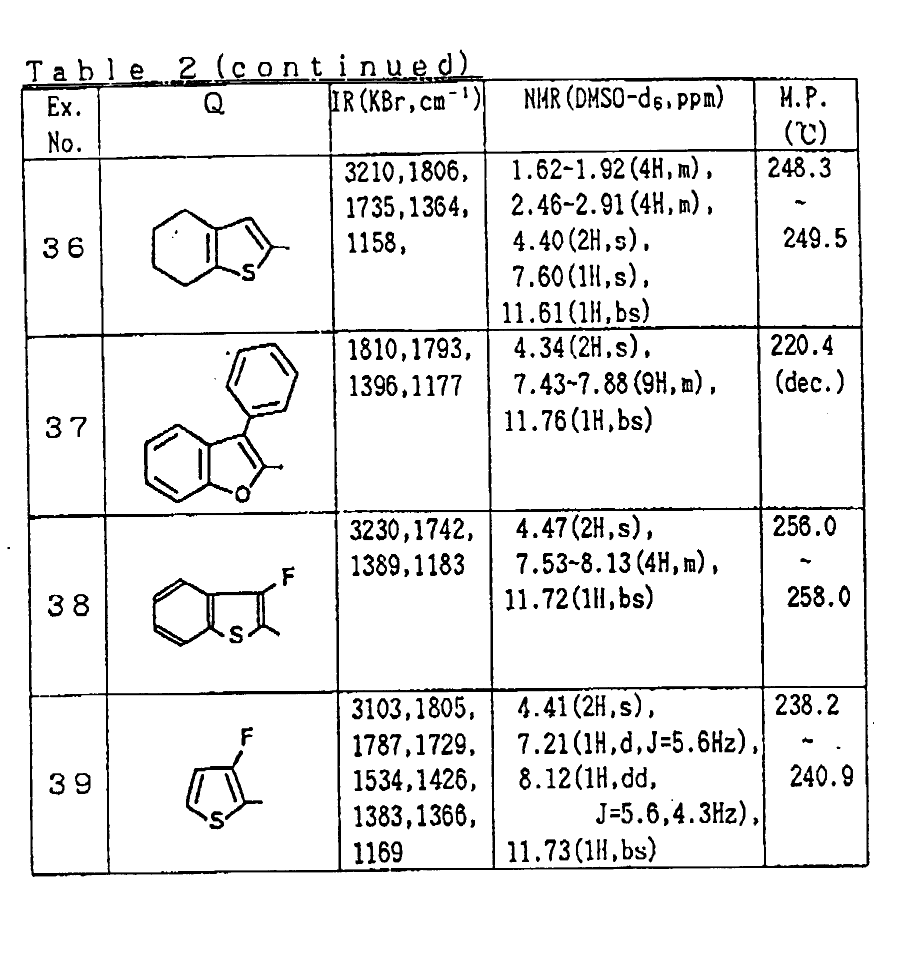

### Example 40

### Preparation of 1-(4,5-diphenylthien-2-yl-sulfonyl)hydantoin.

### Step 1

### Preparation of 4,5-diphenylthien-2-ylsulfonyl chloride.

Starting from 2,3-diphenylthiophen, the objective compound was obtained in a manner similar to Step 1 of Example 1.
- IR (KBr, cm⁻¹):: 1382, 1172, 1038, 698, 583
- NMR (CDCl₃, ppm):: 7.27 - 7.33 (10H, m), 7.89 (1H, s)

### Step 2

### Preparation of N-(4,5-diphenylthien-2-yl-sulfonyl)glycine ethyl ester.

To a suspension of 4,5-diphenylthien-2-yl-sulfonyl chloride (36.5 g) and glycine ethyl ester hydrochloride (30.4 g) in dichloromethane (320 ml) was added slowly triethylamine (30.3 ml) under ice-cooling and the mixture was stirred for 160 minutes at room temperature. Water (200 ml) was added to the resulting solution and the mixture was extracted with dichloromethane. The organic layer was washed with successive 1 M hydrochloric acid, water and saturated aqueous NaCl solution. After drying over anhydrous magnesium sulfate, dichloromethane was removed in vacuo and the residue was reprecipitated from ethyl acetate and hexane to give 41.1 g of the objective compound.
Melting point: 151.2 - 152.7°C
- IR (KBr, cm⁻¹):: 3266, 1734, 1354, 1231, 1215, 1164, 1127
- NMR (DMSO-d₆, ppm):: 1.12 (3H, t, J = 7.1 Hz), 3.88 (2H, d, J = 6.3 Hz), 4.04 (2H, q, J = 7.1 Hz), 6.84 - 7.44 (10H, m), 7.67 (1H, s), 8.57 (1H, t, J = 6.3 Hz)

### Step 3

### Preparation of N-(4,5-diphenylthien-2-yl-sulfonyl)glycine.

A solution of sodium hydroxide (12.4 g) in water (73 ml) was added to a solution of the product obtained in Step 2 (41.4 g) in tetrahydrofuran (730 ml) and the mixture was stirred for 25 minutes at 60°C. After removing the solvent, water (300 ml) was added to the residue and the resulting solution was acidified with concentrated hydrochloric acid to a pH 1 under ice-cooling. The acidified solution was extracted with ethyl acetate (800 ml) and the organic layer was washed with successive water and saturated aqueous NaCl solution. After drying over anhydrous sodium sulfate, ethyl acetate was removed in vacuo and the residue was reprecipitated from ethyl acetate and hexane to give 37.6 g of the objective compound.
Melting point: 172.2 - 174.4°C
- IR (KBr, cm⁻¹):: 3268, 1736, 1353, 1159
- NMR (DMSO-d₆, ppm):: 3.78 (2H, d, J - 5.9 Hz), 7.12 - 7.42 (10H, m), 7.67 (1H, s), 8.39 (1H, t, J = 5.9 Hz), 12.78 (1H, bs)

### Step 4

### Preparation of 1-(4,5-diphenylthien-2-yl-sulfonyl)-2-thiohydantoin.

Starting from the product obtained in Step 3, the objective compound was obtained in a manner similar to Step 3 of Example 1.
Melting point: 213.2 - 215.4°C
- IR (KBr, cm⁻¹):: 1752, 1446, 1376, 1168, 1083
- NMR (DMSO-d₆, ppm):: 4.77 (2H, s), 7.32 - 7.46 (10H, m), 8.12 (1H, s), 12.73 (1H, bs)

### Step 5

### Preparation of 1-(4,5-diphenylthien-2-yl-sulfonyl)hydantoin.

Starting from the product obtained in Step 4, the objective compound was obtained in a manner similar to Step 4 of Example 1.
Melting point: 242.5 - 243.9°C
- IR (KBr, cm⁻¹):: 1737, 1386, 1165
- NMR (DMSO-d₆, ppm):: 4.53 (2H, s), 7.32 - 7.45 (10H, m), 8.00 (1H, s), 11.72 (1H, bs)

Compounds of Example 41 and 44 prepared in a manner similar to Example 40 are summarized in the following table 3 together with corresponding IR and NMR data and melting points.

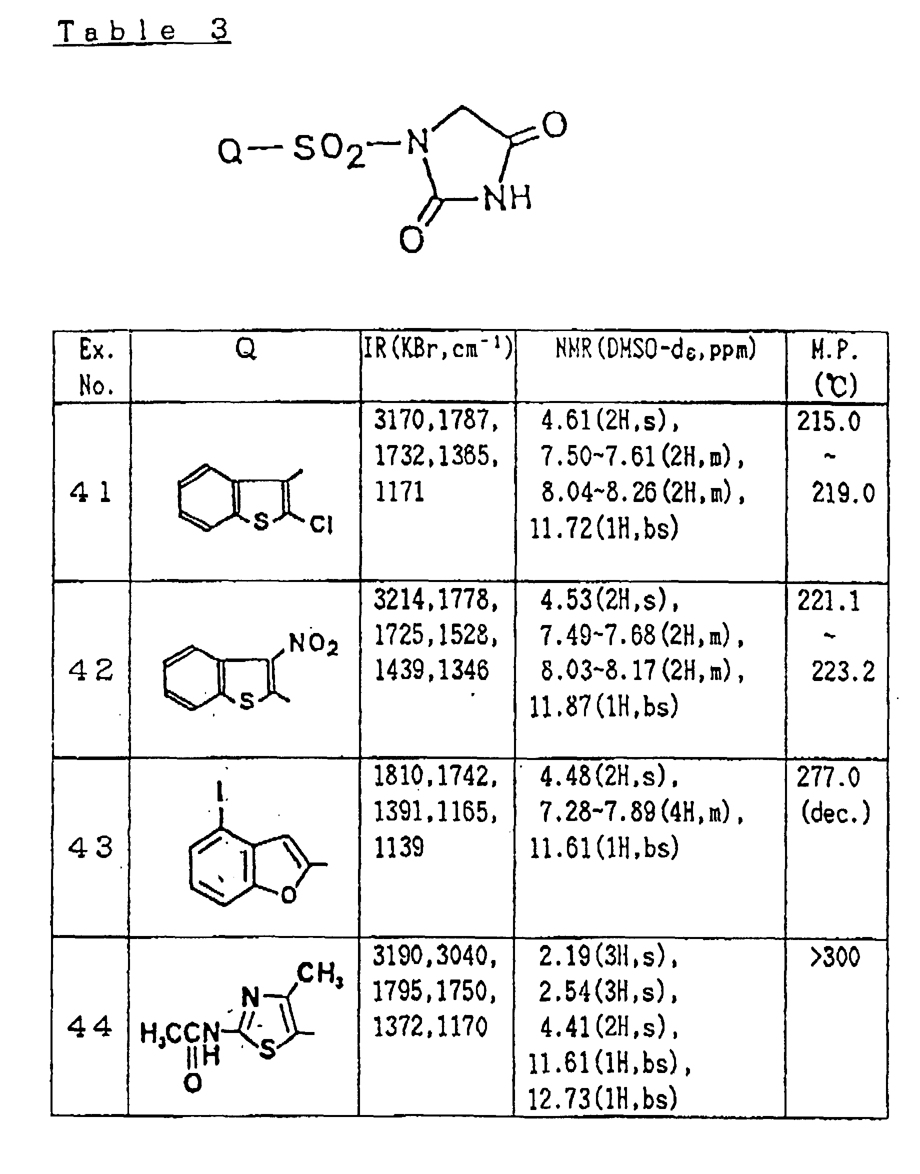

### Example 45

### Preparation of 1-(5-nitrobenzo[b]thien-2-yl-sulfonyl)hydantoin. (compound 27).

### Step 1

### Preparation of 5-nitrobenzo[b]thien-2-yl-sulfonyl chloride.

To a solution of 5-nitrobenzo[b]thiophen (60 g) in anhydrous tetrahydrofuran (2 l) was added dropwise a solution of lithium diisopropylamide comprising 1.6 M n-butyllithium in hexane (240 ml) and diisopropylamine (57.8 ml) and anhydrous ether (170 ml) with stirring at -70°C under nitrogen atmosphere. After stirring for 30 minutes, into the solution was bubbled sulfur dioxide for 90 minutes with stirring at -70°C. Then the solution was stirred for 1 hour at room temperature and the formed precipitate was separated by filtration to give lithium 5-nitrobenzo[b]-thien-2-ylsulfinate. Into the suspension of the product in concentrated hydrochloric acid (500 ml) and water (125 ml) was bubbled chlorine gas for 3 hours with sufficiently stirring at below 0°C. After stirring for 1 hour at room temperature, the resulting suspension was extracted with dichloromethane (400 ml x 2) and the organic layer was washed with successive water and saturated aqueous NaCl solution. After drying over anhydrous sodium sulfate, dichloromethane was removed in vacuo, and the residue was purified by silica gel column chromatography to give 21 g of the objective compound.
- IR (KBr, cm⁻¹):: 1602, 1519, 1378, 1340, 1172
- NMR (CDCl₃, ppm):: 8.10 (1H, d, J= 8.9 Hz), 8.31 (1H, s), 8.46 (1H, dd, J = 8.9, 2.0 Hz), 8.90 (1H, d, J = 2.0 Hz)

### Step 2

### Preparation of N-(5-nitrobenzo[b]thien-2-yl-sulfonyl)glycine.

Starting from 5-nitrobenzo[b]thien-2-ylsulfonyl chloride, the objective compound was obtained in a manner similar to Step 2 of Example 1.
Melting point: 187.2 - 194.8°C
- IR (KBr, cm⁻¹):: 3325, 1734, 1530, 1377, 1351, 1159
- NMR (DMSO-d₆, ppm):: 3.76 (2H, d, J = 5.9 Hz), 8.22 (1H, s), 8.32 - 8.91 (4H, m), 12.72 (1H, bs)

### Step 3

### Preparation of 1-(5-nitrobenzo[b]thien-2-yl-sulfonyl)-2-thiohydantoin.

Starting from the product obtained in Step 2, the objective compound was obtained in a manner similar to Step 3 of Example 1.
Melting point: 217.4°C (decomposition)
- IR (KBr, cm⁻¹):: 1762, 1521, 1470, 1389, 1347, 1248, 1173, 1087
- NMR (DMSO-d₆, ppm):: 4.73 (2H, s), 8.25 - 9.09 (4H, m), 12.78 (1H, bs)

### Step 4

### Preparation of 1-(5-nitrobenzo[b]thien-2-yl-sulfonyl)hydantoin.

A mixture of the product obtained in Step 3 (1.66 g) and 50% (w/v) nitric acid (35 ml) was heated with stirring for 6 hours at 60°C and the resulting solution was poured into ice-water (150 ml). The formed precipitate was separated by filtration and washed with acetone to give 0.47 g of the objective compound.
Melting point: 282.4°C (decomposition)
- IR (KBr, cm⁻¹):: 3100, 1737, 1522, 1385, 1349 1176
- NMR (DMSO-d₆, ppm):: 4.47 (2H, s), 8.22 - 9.05 (4H, m), 11.70 (1H, bs)

### Example 46

### Preparation of 1-(5-cyanobenzo[b]thien-2-yl-sulfonyl)hydantoin.

### Step 1

### Preparation of 5-cyanobenzo[b]thien-2-yl-sulfonyl chloride.

Starting from benzo[b]thien-5-ylcarbonitrile, the objective compound was obtained in a manner similar to Step 1 of Example 45.
- IR (KBr, cm⁻¹):: 2236, 1500, 1376, 1171, 577
- NMR (DMSO-d₆, ppm):: 7.56 (1H, s), 7.70 (1H, dd, J = 8.9, 2.0 Hz), 8.15 (1H, d, J = 8.9 Hz ), 8.37 (1H, d, J = 2.0 Hz )

### Step 2

### Preparation of N-(5-cyanobenzo[b]thien-2-yl-sulfonyl)glycine.

Starting from 5-cyanobenzo[b]thien-2-ylsulfonyl chloride, the objective compound was obtained in a manner similar to Step 2 of Example 1.
- IR (KBr, cm⁻¹):: 3289, 2235, 1714, 1350, 1153
- NMR (DMSO-d₆, ppm):: 3.75 (2H, d, J = 5,6 Hz), 7.87 (1H, dd, J = 8.6, 1.3 Hz), 8.06 (1H, s), 8.34 (1H, d, J = 8.6 Hz), 8.56 (1H, d, J = 1.3 Hz), 8.70 (1H, t, J = 5.6 Hz), 12.69 (1H, bs)

### Step 3

### Preparation of 1-(5-cyanobenzo[b]thien-2-yl-sulfonyl)-2-thiohydantoin.

Starting from the product obtained in Step 2, the objective compound was obtained in a manner similar to Step 3 of Example 1.
- IR (KBr, cm⁻¹):: 2231, 1762, 1451, 1243, 1173, 1077
- NMR (DMSO-d₆, ppm):: 4.73 (2H, s), 7.95 (1H, dd, J = 8.6, 1.7 Hz), 8.41 (1H, d, J - 8.6 Hz), 8.53 (1H, s), 8.63 (1H, d, J = 1.7 Hz), 12.72 (1H, bs)

### Step 4

### Preparation of 1-(5-cyanobenzo[b]thien-2-yl-sulfonyl)hydantoin.

A mixture of the product obtained in step 3 (0.39 g) and 50% (w/v) nitric acid (8.2 ml) was heated with stirring for 5 minutes at 80°C, then for 30 minutes at room temperature and the resulting solution was poured into ice-water (35 ml). The formed precipitate was separated by filtration and washed with acetone (100 ml) to give 0.11 g of the objective compound.
Melting point: 276.3°C (decomposition)
- IR (KBr, cm⁻¹):: 3100, 2231, 1740, 1386, 1172
- NMR (DMSO-d₆, ppm):: 4.47 (2H, s), 7.95 (1H, dd, J = 8.6, 1.7 Hz), 8.41 (1H, s), 8.42 (1H, d, J = 8.6 Hz), 8.65 (1H, d, J = 1.7 Hz), 11.75(1H, bs)

### Example 47

### Preparation of 1-(5-carboxybenzo[b]thien-2-yl-sulfonyl)hydantoin (compound 28).

To the suspension of the product obtained in Step 4 of Example 46 (0.1 g) in water (1.5 ml) was added slowly concentrated sulfuric acid (1.5 ml) and acetic acid (1.5 ml) under ice-cooling and the mixture was stirred under reflux for 2 hours. After cooling to room temperature, the formed precipitate was separated by filtration and washed with acetone (20 ml). The washings were concentrated in vacuo and the residue was triturated with ether (2 ml) to give 0.02 g of the objective compound.
Melting point: >300°C
- IR (KBr, cm⁻¹):: 1743, 1690,1380, 1163
- NMR (DMSO-d₆, ppm):: 4.46 (2H, s), 8.07 (1H, dd, J = 8.6, 1.7 Hz), 8.28 (1H, d, J = 8.6 Hz), 8.48 (1H, s), 8.69(1H, d, J = 1.7 Hz)

### Example 48

### Preparation of 1-(indol-2-ylsulfonyl)hydantoin. Step 1

### Preparation of 1-benzenesulfonylindol-2-yl-sulfonyl chloride.

To a solution of lithium diisopropylamide comprising 1.6 M n-butyllithium in hexane (422 ml), diisopropylamine (101 ml) and anhydrous ether (260 ml) was added dropwise a solution of 1-benzenesulfonylindole (150 g) in anhydorous ether (2060 ml) with stirring at 0°C. After stirring for 15 minutes at 0°C, the solution was poured into sulfuryl chloride (125 ml) at -50°C and stirred for 2 hours. The resulting solution was poured into ice-water (2.5 l) and stirred sufficiently and then the organic layer was extracted. The aqueous layer was extracted with ethyl acetate (2 l) and the combined organic layer was washed with successive water and saturated aqueous NaCl solution. After drying over anhydrous sodium sulfate, ether and ethyl acetate were removed in vacuo and the residue was triturated with ether to give 146 g of the objective compound.
IR (KBr, cm⁻¹): 1513, 1387, 1378, 1245, 1188
NMR (CDCl₃, ppm): 7.29 - 8.36 (10H, m)

### Step 2

### Preparation of N-(1-benzenesulfonylindol-2-yl-sulfonyl)glycine ethyl ester.

Starting from 1-benzenesulfonylindol-2-ylsulfonyl chloride, the objective compound was obtained in a manner similar to Step 2 of Example 40.
- IR (KBr, cm⁻¹):: 3335, 1746, 1346, 1338, 1171
- NMR (DMSO-d₆, ppm):: 1.11 (3H, t, J = 7.3 Hz), 3.94 (2H, d, J = 5.6 Hz), 4.06 (2H, q, J = 7.3 Hz), 6.38 (1H, t, J = 5.6 Hz), 7.14 - 8.32 (10H, m)

### Step 3

### Preparation of N-(indol-2-ylsulfonyl)glycine.

A solution of sodium hydroxide (1.6 g) in water (7 ml) was added to a solution of the product obtained in Step 2 (4.22 g) in tetrahydrofuran (70 ml) at room temperature and the mixture was stirred for 5 minutes at 65 - 75°C. After removing tetrahydofuran in vacuo, a solution of sodium hydroxide (0.4 g) in water (23 ml) was added to the residue and the mixture was stirred for 5 hours at 65 - 75°C. After cooling to room temperature, the resulting solution was washed with ether, acidified with 6 M hydrochloric acid to a pH 1 under ice-cooling and extracted with ethyl acetate (15 ml × 3). The organic layer was washed with successive water and saturated aqueous NaCl solution. After drying over anhydrous sodium sulfate, ethyl acetate was removed in vacuo and the residue was triturated with ethyl acetate and hexane to give 1.66 g of the objective compound.
Melting point: 170.2 - 171.9°C
- IR (KBr, cm⁻¹):: 3328, 1707, 1340, 1155, 1145
- NMR (DMSO-d₆, ppm):: 3.73 (2H, d, J = 6.3 Hz), 6.94 - 7.70 (5H, m), 8.05 (1H, t, J = 6.3 Hz), 11.90 (1H, bs), 12.67 (1H, bs)

### Step 4

### Preparation of 1-(indol-2-ylsulfonyl)-2-thiohydantoin.

Starting from the product obtained in Step 3, the objective compound was obtained in a manner similar to Step 3 of Example 1.
Melting point: 209.2 - 210.4°C
- IR (KBr, cm⁻¹):: 3131, 3103, 1755, 1473, 1367, 1249, 1197, 1165, 1147, 1079
- NMR (DMSO-d₆, ppm):: 4.81 (2H, s), 7.08 - 7.78 (5H, m), 12.33 (1H, bs), 12.66 (1H, bs)

### Step 5

### Preparation of 1-(indol-2-ylsulfonyl)hydantoin.

Starting from the product obtained in Step 4, the objective compound was obtained in a manner similar to Step 4 of Example 1.
Melting point: 287.1°C (decomposition)
- IR (KBr, cm⁻¹):: 3290, 1787, 1725, 1389, 1365, 1156
- NMR (DMSO-d₆, ppm):: 4.67 (2H, s), 7.29 - 7.58 (5H, m), 11.67 (1H, bs), 12.63 (1H, bs)

### Example 49

### Preparation of 1-(2-carboxychromon-6-ylsulfonyl)hydantoin.

### Step 1

### Preparation of 2-methoxycarbonylchromon-6-yl-sulfonyl chloride.

To a solution of methyl 6-aminochromon-2-carboxylate (20 g) in water (132 ml) was added concentrated sulfuric acid (26.4 ml) and then sodium nitrite (9.0 g) at 0°C. After stirring for 30 minutes, to the solution was added sulfur dioxide (19.7 ml), acetic acid (112 ml), concentrated hydrochloric acid (26 ml) and copper (II) chloride dihydrate (11.2 g) and then the mixture was stirred for 15 minutes. The formed precipitate was separated by filtration and dissolved in dichloromethane (600 ml) and the resulting solution was washed with saturated aqueous NaCl solution. After drying over anhydrous sodium sulfate, dichloromethane was removed in vacuo to give 22 g of the objective compound.
- IR (KBr, cm⁻¹):: 1744, 1661, 1381, 1287, 1174, 600
- NMR (DMSO-d₆, ppm):: 6.96 (1H, s), 7.70 (1H, d, J = 8.6 Hz), 8.04 (1H, dd, J = 8.6, 2.0 Hz), 8.25 (1H, d, J = 2.0 Hz)

### Step 2

### Preparation of N-(2-methoxycarbonylchromon-6-yl-sulfonyl)glycine.

To a suspension of 2-methoxycarbonylchromon-6-yl-sulfonyl chloride (20.0 g) in acetone (600 ml) was added slowly a solution of glycine (6.15 g), sodium hydroxide (3.28 g) and sodium bicarbonate (6.11 g) in water (300 ml) and the mixture was stirred for 85 minutes at room temperature. After adjusting a pH of the resulting solution to ca. 6 with 6 M hydrochloric acid, acetone was removed in vacuo and insoluble matters were filtered off. The filtrate was acidified with 2 M hydrochloric acid to a pH 1 under ice-cooling. The acidified solution was extracted with ethyl acetate (350 ml × 3) and the organic layer was washed with successive water and saturated aqueous NaCl solution. After drying over anhydrous sodium sulfate, ethyl acetate was removed in vacuo and the residue was purified by silica gel column chromatography to give 5.45 g of the objective compound.
Melting point: 210.6 - 212.8°C
- IR (KBr, cm⁻¹):: 3327, 1746, 1716, 1659, 1288, 1266, 1165
- NMR (DMSO-d₆, ppm):: 3.67 (2H, d, J = 5.9 Hz), 3.96 (3H, s), 7.04 (1H, s), 7.89 - 8.42 (4H, m)

### Step 3

### Preparation of 1-(2-methoxycarbonylchromon-6-yl-sulfonyl)-2-thiohydantoin.

Starting from the product obtained in Step 2, the objective compound was obtained in a manner similar to Step 3 of Example 1.
Melting point: 217.4°C (decomposition)
- IR (KBr, cm⁻¹):: 1746, 1660, 1443, 1374, 1282, 1260, 1174
- NMR (DMSO-d₆, ppm):: 3.96 (3H, s), 4.84 (2H, s), 7.07 (1H, s), 7.97 - 8.71 (3H, m), 12.68 (1H, bs)

### Step 4

### Preparation of 1-(2-methoxycarbonylchromon-6-yl-sulfonyl)hydantoin.

Starting from the product obtained in step 3, the objective compound was obtained in a manner similar to step 4 of Example 1.
Melting point: >300°C
- IR (KBr, cm⁻¹):: 1751, 1741, 1664, 1617, 1375, 1177,
- NMR (DMSO-d₆, ppm):: 3.96 (3H, s), 4.52 (2H, s) 7.07 (1H, s), 7.98 - 8.64 (3H, m)

### Step 5

### Preparation of 1-(2-carboxychromon-6-ylsulfonyl)hydantoin.

A solution of the product obtained in Step 4 (2.27 g) in a saturated aqueous sodium bicarbonate solution (22.7 ml) was stirred for 2 hours at 40°C. The resulting solution was washed with ethyl acetate and acidified with 2 M hydrochloric acid to a pH 1 under ice-cooling and the formed precipitate was separated by filtration to give 0.82 g of the objective compound.
Melting point: 279.3°C (decomposition)
- IR (KBr, cm⁻¹):: 3220, 1751, 1663, 1376, 1172
- NMR (DMSO-d₆, ppm):: 4.54 (2H, s), 7.02 (1H, s), 7.95 - 8.61 (3H, m), 11.63 (1H, bs)

### Example 50

### Preparation of 1-(benzothiazol-2-ylsulfonyl)hydantoin (compound 7).

### Step 1

### Preparation of 2-benzylthiobenzothiazole.

To a solution of 2-benzothiazolthiol (250 g) in N,N-dimethylformamide (1 l) was added triethylamine (208 ml) under ice-cooling and dropwise a solution of benzyl bromide (178 ml) in N,N-dimethylformamide (300 ml) and the mixture was stirred for 40 minutes. The resulting solution was poured into water (10 l) and the formed precipitate was separated by filtration and dissolved in dichloromethane (3 l). After drying over anhydorpus magnesium sulfate, dichloromethane was removed in vacuo to give 378 g of the objective compound.

### Step 2

### Preparation of benzothiazol-2-ylsulfonyl chloride.

Into a mixture of 2-benzylthiobenzothiazole (100 g) and acetic acid (500 ml) in water (500 ml) was bubbled chlorine gas for 1.5 hours with stirring at -15°C. The resulting solution was poured into ice-water (1.5 l), the formed precipitate was separated by filtration to give 90.9 g of the objective compound.

### Step 3

### Preparation of N-(benzothiazol-2-ylsulfonyl)glycinamide.

To a suspension of glycinamide hydrochloride (43 g) in dioxane (1 l) was added benzothiazol-2-yl-sulfonyl chloride (90.9 g) under ice-cooling and a pH of the mixture was adjusted to 8 with saturated aqueous sodium bicarbonate solution. After stirring for 1.5 hours, the resulting solution was concentrated in vacuo. Water (1.5 l) was added to the residue and the solution was acidified with concentrated hydrochloric acid to a pH 2. The formed precipitate was separated by filtration to give 59.8 g of the objective compound.
Melting point: 179.7 - 181.8°C
- IR (KBr, cm⁻¹):: 3426, 1682, 1346, 1165
- NMR (DMSO-d₆, ppm):: 3.73 (2H, s), 7.08 (1H, bs), 7.36 (1H, bs), 7.52 - 8.29 (4H, m), 8.80 (1H, bs)

### Step 4

### Preparation of N²-(benzothiazol-2-yl-sulfonyl)-N²-methoxycarbonylglycinamide.

To a solution of the product obtained in Step 3 (102.3 g) in N,N-dimethylformamide (1.2 l) was added slowly 60% sodium hydride (16.7 g) under ice-cooling and the mixture was stirred for 1 hour at room temperature. Methyl chlorocarbonate (35.8 g) was added to the above mentioned mixture followed by stirring for 1 hour at room temperature. After removing the solvent, water (3.5 l) was added to the residue and the formed precipitate was separated by filtration to give 60.5 g of the objective compound.
Melting point: 153.1°C (decomposition)
- IR (KBr, cm⁻¹):: 3459, 3346, 1737, 1689, 1386, 1343, 1250, 1171
- NMR (DMSO-d₆, ppm):: 3.70 (3H, s), 4.51 (2H, s), 7.30 (1H, bs), 7.60 - 7.76 (3H, m), 8.20 - 8.39 (2H, m)

### Step 5

### Preparation of 1-(benzothiazol-2-ylsulfonyl)hydantoin.

To a solution of the product obtained in Step 4 (20.0 g) in N,N-dimethylformamide (200 ml) was added slowly 60% sodium hydride (2.67 g) and the mixture was stirred for 13.5 hours at 70°C. After removing the solvent, water (1 l) was added to the residue and the solution was extracted with ethyl acetate (1.5 l) and the organic layer was washed with saturated aqueous NaCl solution. After drying over anhydrous sodium sulfate, ethyl acetate was removed in vacuo and the residue was washed with acetone-chloroform (100 ml + 200 ml) to give 2.12 g of the objective compound.
Melting point; 260.4 - 261 9°C
- IR (KBr, cm⁻¹):: 3200, 3105, 1739, 1393, 1355, 1173
- NMR (DMSO-d₆, ppm):: 4.55 (2H, s), 7.61 - 7.81 (2H, m), 8.18 - 8.40 (2H, m), 11.88 (1H, bs)

Compounds of Example 51 and 52 prepared in a manner similar to Example 50 are summarized in the following table 4 together with corresponding IR and NMR data and melting points.

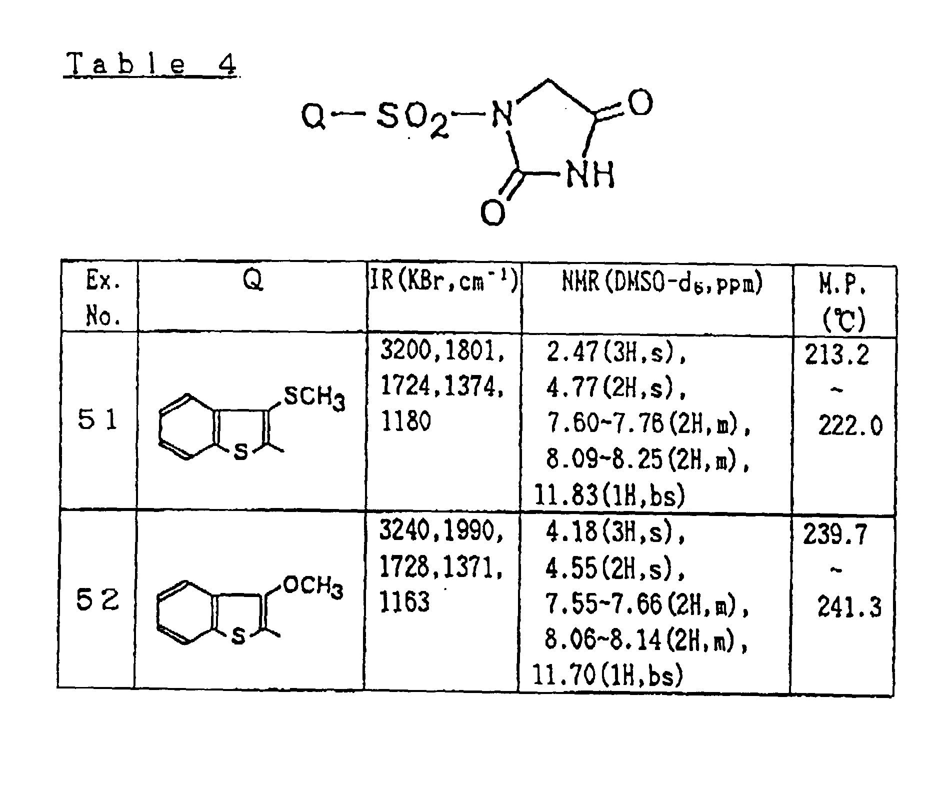

### Example 53

### Preparation of 1-(benzo[c]thien-1-ylsulfonyl)hydantoin.

### Step 1

### Preparation of N²-(benzo[c]thien-1-yl-sulfonyl)glycinamide.

To a solution of benzo[c]thiophen (5.5 g) in anhydrous ether (50 ml) was added 1.6 M solution of n-butyllithium in hexane (52.2 ml) at -20°C under nitrogen atmosphere. After stirring for 1 hour, into the solution was bubbled sulfur dioxide for 1 hour with stirring at -20°C. Ether was removed in vacuo and the residue was suspended in isopropanol (200 ml) and water (200 ml). To the suspension was added N-chlorosuccinimide (6.5 g) at 0°C. After stirring for 30 minutes at 0°C, N-chlorosuccinimide (1.63 g) was added and the mixture was stirred for additional 1 hour. The resulting solution was extracted with dichloromethane (1 l × 2) and the organic layer was washed with successive water and saturated aqueous NaCl solution. After drying over anhydrous sodium sulfate, dichloromethane was removed in vacuo under cooling. Using this residue and glycinamide hydrochloride, the objective compound was obtained in a manner similar to Step 3 of Example 50.
- NMR (DMSO-d₆, ppm):: 3.40 (2H, d, J = 6.9 Hz), 7.06 - 8.22 (5H, m), 8.49 (1H, s)

### Step 2

### Preparation of N²-(benzo[c]thien-1-ylsulfonyl)-N¹-methoxycarbonylglycinamide.

To a solution of the product obtained in Step 1 (0.45 g) in N,N-dimethylformamide (5 ml) was added slowly 60% sodium hydride (75 mg) under ice-cooling and the mixture was stirred for 30 minutes at room temperature. Methyl chlorocarbonate (0.14 ml) was added to the above mentioned mixture followed by stirring for 20 minutes at room temperature. 60% sodium hydride (75 mg) was added to the solution and the mixture was stirred for 1.5 hours at room temperature, then 15 minutes at 70°C. After cooling to room temperature, water (20 ml) was added to the resulting mixture and this aqueous solution was extracted with ethyl acetate (20 ml × 3). The organic layer was washed with successive water and saturated aqueous NaCl solution. After drying over anhydrous magnesium sulfate, ethyl acetate was removed in vacuo and the residue was purified by silica gel columun chromatography to give 0.18 g of the objective compound.
- NMR (CDCl₃, ppm):: 3.74 (3H, s), 4.24 (2H, d, J = 5.3 Hz), 5.92 (1H, t, J = 5.3 Hz), 7.17 - 8.31 (6H, m)

### Step 3

### Preparation of 1-(benzo[c]thien-1-ylsulfonyl)hydantoin.

To a solution of the product obtained in Step 2 (0.18 g) in N,N-dimethylformamide (3 ml) was added slowly 60% sodium hydride (48 mg) and the mixture was stirred for 2.5 hours at 70°C. After removing the solvent, ice water (20 ml) was added to the residue and a pH of the solution was adjusted to 4 with 1 M hydrochloric acid. The resulting solution was extracted with ethyl acetate (20 ml × 3) and the organic layer was washed with saturated aqueous NaCl solution. After drying over anhydrous magnesium sulfate, ethyl acetate was removed in vacuo and the residue was triturated with dichloromethane to give 0.03 g of the objective compound.
Melting point: 223.6 - 226.9°C
- IR (KBr, cm⁻¹):: 1736, 1378, 1185, 1162, 1152
- NMR (DMSO-d₆, ppm):: 4.51 (2H, s), 7.20 - 8.16 (4H, m), 8.82 (1H, s), 11.54 (1H, bs)

### Example 54

### Preparation of 1-(3-carboxymethylbenzo[b]-thien-2-ylsulfonyl)hydantoin.

A mixture of the product obtained in Step 4 of Example 30 (0.85 g) and 60% (w/v) nitric acid (9 ml) was heated with stirring for 140 minutes at 70°C. After cooling to room temperature, the formed precipitate was separated by filtration and washed with ether to give 0.21 g of the objective compound.
Melting point: 224.4°C (decomposition)
- IR (KBr, cm⁻¹):: 3220, 1800, 1736, 1718, 1374, 1170
- NMR (DMSO-d₆, ppm):: 4.32 (2H, s), 4.47 (2H, s), 7.55 - 7.65 (2H, m), 7.99 - 8.19 (2H, m), 11.71(1H, bs)

### Example 55

### Preparation of 1-(3-methylsulfinylbenzo[b]thien-2-ylsulfonyl)hydantoin.

To a suspension of the product obtained in Example 51 (0.65 g) in dichloromethane (26 ml) was added m-chloroperbenzoic acid (0.41 g) and the mixture was stirred for 1.5 hours at room temperature. The resulting solution was concentrated in vacuo and the residue was washed with ether (30 ml). The residue was purified by silica gel column chromatography to give 0.48 g of the objective compound.
Melting point: 215.0 - 221.0°C
- IR (KBr, cm⁻¹):: 1792, 1743, 1379, 1180
- NMR (DMSO-d₆, ppm):: 3.10 (3H, s), 4.57 (2H, s), 7.51 - 8.89 (4H,m), 11.81 (1H, bs)

### Example 56

### Preparation of 1-(3-methylsulfonylbenzo[b]thien-2-ylsulfonyl)hydantoin (compound 17).

To a suspension of the product obtained in Example 51 (0.65 g) in ethyl acetate (26 ml) was added m-chloroperbenzoic acid (0.82 g) and the mixture was stirred under reflux for 1.5 hours. Additional m-chloroperbenzoic acid (0.16 g) was added and the mixture was stirred under reflux for more 1.5 hours. The resulting solution was concentrated in vacuo and the residue was washed with successive methanol and ether to give 0.40 g of the objective compound.
Melting point: 224.0 - 245.0°C
- IR (KBr, cm⁻¹):: 1771, 1372, 1324, 1179
- NMR (DMSO-d₆, ppm):: 3.47 (3H, s), 4.63 (2H, s), 7.66 - 8.59 (4H,m), 11.90(1H, bs)

### Example 57

### Preparation of 1-(3-cyanobenzo[b]thien-2-yl-sulfonyl)hydantoin (compound 18).

To a mixture of the product obtained in Example 29 (11.3 g) end copper (I) cyanide (4.1 g) was added pyridine (42 ml). After stirring at 70 °C for 17 hours, a solution of Iron (III) chloride hexahydrate (15.7 g) in concentrated hydrochloric acid (3.9 ml) and water (23.6 ml) was added slowly to the solution and the resultant mixture was heated with stirring for 5 minutes at 50 °C. The formed precipitate was separated by filtration and the filtrate was extracted with ethyl acetate (300 ml) and the organic layer was washed with successive water and saturated aqueous NaCl solution and dried over anhydrous sodium sulfate. Above mentioned precipitate was extracted by ethanol and this ethanol solution was combined with above mentioned organic layer. The resulting solution was concentrated in vacuo and purified by silica gel column chromatography to give 1.21 g of the objective compound.
Melting point: 238.9 - 242.5°C
- IR (KBr, cm⁻¹):: 2233, 1807, 1746, 1736, 1388, 1167
- NMR (DMSO-d₆, ppm):: 4.51 (2H, s), 7.70 - 8.47 (4H, m), 11.83 (1H, bs)

### Example 58

### Preparation of 1-(3-hydroxybenzo[b]thien-2-yl-sulfonyl)hydantoin.

A mixture of the product obtained in Example 52 (2.5 g), acetic acid (7 ml) and 47% hydrobromic acid (8.9 ml) was stirred for 1 hour at room temperature and heated for 1 hour at 40 °C, for more 1 hour at 50°C. To the mixture was added additional acetic acid (7 ml) and 47% hydrobromic acid (8.9 ml) and heated with stirring for 1 hour at 60°C, for 2 hours at 80°C. The resulting solution was poured into water (300 ml) and extracted with ethyl acetate (1.2 l). After drying over anhydrous magnesium sulfate, ethyl acetate was removed in vacuo and the residue was dissolved in acetone (800 ml). After decoloring with activated charcoal, acetone was removed in vacuo and the residue was washed with successive ethyl acetate and ether to give 1.13 g of the objective compound.
Melting point; 171.8 °C (decomposition)
- IR (KBr, cm⁻¹):: 3260, 1800, 1735, 1358, 1185, 1164
- NMR (DMSO-d₆, ppm):: 4.60 (2H, s), 7.46 - 8.19 (4H, m), 11.70 (1H, bs)

### Example 59

### Preparation of 1-(3-carbamoylbenzo[b]thien-2-ylsulfonyl)hydantoin.

A mixture of the product obtained in Example 57 (0.84 g) and 80%(v/v) sulfuric acid (16.3 ml) was heated with stirring for 8 hours at 70°C and the resulting solution was poured into ice-water (200 ml). The formed precipitate was separated by filtration and washed with successive water, ethanol and acetone to give 0.16 g of the objective compound.
Melting point: 241.9 - 244.6°C
- IR (KBr, cm⁻¹):: 3412, 3197, 1795, 1741, 1376, 1162
- NMR (DMSO-d₆, ppm):: 4.51 (2H, s), 7.56 - 8.44 (6H, m), 11.73 (1H, bs)

### Example 60

### Preparation of 1-(3-carboxybenzo[b]thien-2-yl-sulfonyl)hydantoin.

To a suspension of the product obtained in Example 59 (0.60 g) in concentrated sulfuric acid (18 ml) was added sodium nitrite (2.4 g) under cooling at -15°C and the resulting suspension was stirred for 15 minutes at -15°C, for 30 minutes at 0°C and for 50 minutes at room temperature. To the mixture was added additional sodium nitrite (1.2 g) and stirred for 30 minutes at room temperature. After adjusting a pH of the resulting solution to ca. 9 with 0.1 M sodium bicarbonate, the resulting solution was washed with ethyl acetate and acidified with concentrated hydrochloric acid to a pH about 2 and extracted with ethyl acetate (200 ml). The organic layer was washed with successive water and saturated aqueous NaCl solution. After drying over anhydrous sodium sulfate, ethyl acetate was removed in vacuo and the residue was purified by silica gel column chromatography to give 0.18 g of the objective compound.
Melting point: 228.8 - 235.1°C
- IR (KBr, cm⁻¹):: 3450, 1739, 1735, 1380, 1175
- NMR (DMSO-d₆, ppm):: 4.73 (2H, s), 7.45 - 8.17 (4H, m), 11.76 (1H, bs)

### Example 61

### Preparation of 1-(3-chlorobenzo[b]furan-2-yl-sulfonyl)hydantoin (compound 32).

### Step 1

### Preparation of 3-chlorobenzo[b]furan-2-yl-sulfonyl chloride.

To a solution of 3-chlorobenzo[b]furan (11.4 g) in anhydrous ether (62 ml) was added dropwise 1.5 M lithium diisopropylamide mono(tetrahydrofuran) in hexane (62 ml) under nitrogen atmosphere at -70°C. After stirring for 30 minutes, into the solution was bubbled sulfur dioxide for 1 hour with stirring at -60°C. Then the solution was stirred for 1 hour at room temperature and the formed precipitate was separated by filtration to give lithium 3-chlorobenzo[b]furan-2-sulfinate. To the suspension of the product in dichloromethane (250 ml) was added N-chlorosuccinimide (11.0 g) at -50°C and stirred for 3 hours. After stirring for 2 hours under ice-cooling, insoluble matters were filtered off. Dichloromethane was removed in vacuo and the residue was purified by silica gel column chromatography to give 8.8 g of the objective compound.
Melting point: 60.6 - 68.2°C
- IR (KBr, cm⁻¹):: 1538, 1402, 1232, 1183, 1151, 1039
- NMR (CDCl₃, ppm):: 7.40 - 7.98 (4H, m)

### Step 2

### Preparation of N-(3-chlorobenzo[b]furan-2-yl-sulfonyl)glycine ethyl ester.

To a suspension of 3-chlorobenzo[b]furan-2-yl-sulfonyl chloride (8.6 g) and glycine ethyl ester hydrochloride (9.6 g) in dichloromethane (83 ml) was added slowly triethylamine (10.4 ml) under ice-cooling and then the resulting mixture was stirred for 30 minutes at room temperature. Water (150 ml) was added to the resultant solution and acidified with 1 M hydrochloric acid to a pH 2, and the acidified solution was extracted with ethyl acetate (300 ml). After drying over anhydrous magnesium sulfate, ethyl acetate was removed in vacuo to give 10.2 g of the objective compound.
Melting point: 104.5 - 110.8°C
- IR (KBr, cm⁻¹):: 3203, 1736, 1365, 1230, 1149
- NMR (DMSO-d₆, ppm):: 1.01 (3H, t, J = 7.1 Hz), 3.89 (2H, q, J = 7.1 Hz), 3.94 (2H, s), 7.50 - 7.73 (4H, m), 9.12 (1H, bs)

### Step 3

### Preparation of N-(3-chlorobenzo[b]furan-2-yl-sulfonyl)glycine.

To a solution of N-(3-chlorobenzo[b]furan-2-yl-sulfonyl)glycine ethyl ester (10.2 g) in tetrahydrofuran (160 ml) was added drpowise a solution of sodium hydroxide (4.9 g) in water (16 ml) under ice-cooling and the resulting solution was stirred for 1 hour. After stirring for 30 minutes at room temperature, tetrahydrofuran was removed in vacuo. Water (200 ml) was added to the residue and then acidified with concentrated hydrochloric acid under ice-cooling to a pH 1 and the acidified solution was extracted with ethyl acetate (500 ml). The organic layer was washed with saturated aqueous NaCl solution. After drying over anhydrous magnesium sulfate, ethyl acetate was removed in vacuo to give 9.2 g of the objective compound.
Melting point: 163.8 - 167.9°C
- IR (KBr, cm⁻¹):: 3236, 1709, 1369, 1232, 1153
- NMR (DHSO-d₆, ppm):: 3.84 (2H, d, J = 5.9 Hz), 7.38 - 7.81 (4H, m), 9.03 (1H, t, J = 5.9 Hz), 12.67 (1H, bs)

### Step 4

### Preparation of 1-(3-chlorobenzo[b]furan-2-yl-sulfonyl)-2-thiohydantoin.

To a mixture of N-(3-chlorobenzo[b]furan-2-yl-sulfonyl)glycine (9.2 g), ammonium thiocyanate (5.32 g) and acetic anhydride (18 ml) was added dropwise pyridine (6.68 ml) under ice-cooling and resulting mixture was stirred for 30 minutes at room temperature, for 30 minutes at 40°C and for 2 hours at 70 - 80°C. After cooling to room temperature, the resulting solution was poured into ice-water (300 ml) and the formed precipitate was separated by filtration and washed with water-ethanol to give 6.73 g of the objective compound.
Melting point: 195.4 - 204.7°C
- IR (KBr, cm⁻¹):: 3158, 1758, 1393, 1234, 1179
- NMR (DMSO-d₆, ppm):: 4.83 (2H, s), 7.56 - 7.90 (4H, m)

### Step 5

### Preparation of 1-(3-chlorobenzo[b]furan-2-yl-sulfonyl)hydantoin.

To a suspension of iodine monochloride (5.3 ml) in 1 M hydrochloric acid (160 ml) was added 1-(3-chlorobenzo[b]furan-2-ylsulfonyl)-2-thiohydantoin (6.7 g) and then dichloromethane (200 ml) dropwise. The mixture was stirred for 1.5 hours under ice-cooling and for 1.5 hours at room temperature. After adding saturated aqueous sodium sulfite solution, the reaction mixture was extracted with ethyl acetate (600 ml). The organic layer was washed with successive saturated aqueous sodium sulfite solution and saturated aqueous NaCl solution. After drying over anhydrous magnesium sulfate, ethyl acetate was removed in vacuo and the residue was washed with successive ether and ether-ethyl acetate to give 3.15 g of the objective compound.
Melting point: 246.6 - 256.8°C
- IR (KBr, cm⁻¹):: 3226, 1744, 1397, 1363, 1174, 1156
- NMR (DMSO-d₆, ppm):: 4.51 (2H, s), 7.54 - 7.89 (4H, m), 11.81 (1H, bs)

### Example 62

### Preparation of 1-(4-bromobenzo[b]furan-2-yl-sulfonyl)hydantoin.

### Step 1

### Preparation of (3-bromophenyloxy)acetaldehyde dimethyl acetal.

To a suspension of 60% sodium hydride (60 g) in N,N-dimethylformamide (1.4 l) was added dropwise 3-bromophenol (260 g) under ice-cooling. After stirring for 10 minutes, to the solution was added dropwise bromoacetaldehyde dimethyl acetal (318 g) and the mixture was heated with stirring for 3 hours at 90°C. After cooling, water was added to the resulting solution and acidified with 1 M hydrochloric acid and then extracted with ether (3 l). The organic layer was washed with successive water, saturated aqueous sodium bicarbonate solution and saturated aqueous NaCl solution. After drying over anhydrous sodium sulfate, ether was removed in vacuo and the residue was purified by silica gel column chromatography to give 363.3 g of the objective compound.
- IR (neat, cm⁻¹):: 2941, 2835, 1615, 1506, 1458
- NMR (CDCl₃, ppm):: 3.44 (6H, s), 3.96 (2H, d, J = 5.0 Hz), 4.69 (1H, t, J - 5.0 Hz), 6.77 - 7.26 (4H, m)

### Step 2

### Preparation of mixture of 4-bromobenzo[b]furan and 6-bromobenzo[b]furan.

Under ice-cooling, to phosphoric acid (413.5 ml) was added phosphorus pentoxide (344.2 g) and then chlorobenzene (870 ml). The resulting mixture was heated up to 125°C. To the mixture was added dropwise the solution of the product obtained in Step 1 (181.7 g) in chlorobenzene (150 ml) at 125°C and heated with stirring for 1 hour at 125°C. After cooling, the resulting mixture was poured into ice-water (2 l) and extracted with ether (2 l). The organic layer was washed with successive saturated aqueous sodium bicarbonate solution and saturated aqueous NaCl solution. After drying over anhydrous sodium sulfate, ether and chlorobenzene were removed in vacuo and the residue was purified by silica gel column chromatography to give 116 g of the objective compound.

### Step 3

### Preparation of 4-bromobenzo[b]furan-2-yl-sulfonyl chloride.

To a solution of the mixture obtained in Step 2 (100 g) in anhydrous ether (430 ml) was added dropwise 1.5 M lithium diisopropylamide mono(tetrahydrofuran) in cyclohexane (430 ml) under nitrogen atmosphere at -70°C. After stirring for 30 minutes, into the solution was bubbled sulfur dioxide for 1 hour with stirring at -60°C. Then the solution was stirred for 3 hours at room temperature and the formed precipitate was separated by filtration to give a mixture of lithium 4-bromobenzo[b]furan-2-sulfinate and lithium 6-bromobenzo[b]furan-2-sulfinate. To the suspension of the products in dichloromethane (2 l) was added N-chlorosuccinimide (96 g) at -50°C and stirred for 3 hours under ice-cooling. Insoluble matters were filtered off and dichloromethane was removed in vacuo and the residue was purified by silica gel column chromatography to give 14.1 g of the objective compound.
Melting point: 87.2°C
- IR (KBr, cm⁻¹):: 1603, 1578, 1389, 1175, 1165
- NMR (CDCl₃, ppm):: 7.43 - 7.67 (4H, m)

### Step 4

### Preparation of N-(4-bromobenzo[b]furan-2-yl-sulfonyl)glycine ethyl ester.

Starting from the product obtained in Step 3 (14.1 g), the objective compound (16.8 g) was obtained in a manner similar to Step 2 of Example 61.
Melting point: 115.6 - 117.3°C
- IR (KBr, cm⁻¹):: 3199, 1361, 1221, 1158
- NMR (CDCl₃, ppm):: 1.18 (3H, t, J = 7.1 Hz), 3.97 (2H, d, J = 5.3 Hz), 4.09 (2H, q, J = 7.1 Hz), 5.45 (1H, t, J = 5.3 Hz), 7.26 - 7.58 (4H,m)

### Step 5

### Preparation of N-(4-bromobenzo[b]furan-2-yl-sulfonyl)glycine.

Starting from the product obtained in Step 4 (16.8 g), the objective compound (14.4 g) was obtained in a manner similar to step 3 of Example 61.
Melting point: 180.0 - 182.1°C
- IR (KBr, cm⁻¹):: 3253, 1738, 1361, 1262, 1165
- NMR (DMSO-d₆, ppm):: 3.81 (2H, s), 7.38 -7.81 (4H, m), 8.85 (1H, bs)

### Step 6

### Preparation of 1-(4-bromobenzo[b]furan-2-yl-sulfonyl) -2-thiohydantoin.

To a suspension of the product obtained in Step 5 (14.4 g) and ammonium thiocyanate (7.2 g) in acetic anhydride (28 ml) was added dropwise pyridine (9.1 ml) and the mixture was heated with stirring for 2 hours at 60 - 70°C. After cooling to room temperature, the resulting solution was poured into ice-water (500 ml) and the formed precipitate was separated and washed with ethanol to give 10.7 g of the objective compound.
Melting point: 253.3°C
- IR (KBr, cm⁻¹):: 3140, 1756, 1391, 1248, 1166
- NMR (DMSO-d₆, ppm):: 4.77 (2H, s), 7.45 - 7.88 (3H, m), 7.95 (1H, s), 12.86 (1H, bs)

### Step 7

### Preparation of 1-(4-bromobenzo[b]furan-2-yl-sulfonyl)hydantoin.

Starting from the product obtained in step 6 (10.7 g), the objective compound (4.3 g) was obtained in a manner similar to Step 5 of Example 61.
Melting point: 291.7 - 293.5°C
- IR (KBr, cm⁻¹):: 3240, 1741, 1390, 1355, 1167
- NMR (DMSO-d₆, ppm):: 4.48 (2H, s), 7.45 - 7.90 (4H, m), 11.78 (1H, bs)

### Example 63

### Preparation of 1-(7-fluorobenzo[b]furan-2-yl-sulfonyl)hydantoin (compound 33).

### Step 1

### Preparation of 7-fluorobenzo[b]furan-2-yl-sulfonyl chloride.

Starting from 7-fluorobenzo[b]furan (10.4 g), the objective compound (5.7 g) was obtained in a manner similar to step 1 of Example 61.
Melting point: 114°C
- IR (KBr, cm⁻¹):: 1596, 1546, 1372, 1267, 1178
- NMR (CDCl₃, ppm):: 7.24 - 7.69 (4H, m)

### Step 2

### Preparation of N-(7-fluorobenzo[b]furan-2-yl-sulfonyl)glycine ethyl ester.

Starting from the product obtained in step 1 (5.7 g), the objective compound (6.45 g) was obtained in a manner similar to Step 2 of Example 61.
Melting point: 84.5°C
- IR (KBr, cm⁻¹):: 3238, 1734, 1376, 1232, 1165
- NMR (DMSO-d₆, ppm):: 1.03 (3H, t, J = 7.1 Hz), 3.89 (2H, d, J = 6.3 Hz), 3.92 (2H, q, J = 7.1 Hz), 7.32 - 7.66 (4H, m), 9.05 (1H, t, J = 6.3 Hz)

### Step 3

### Preparation of N-(7-fluorobenzo[b]furan-2-yl-sulfonyl)glycine.

Starting from the product obtained in Step 2 (6.4 g), the objective compound (5.42 g) was obtained in a manner similar to Step 3 of Example 61.
Melting point: 140.1°C (decomposition)
- IR (KBr, cm⁻¹):: 3303, 1734, 1349, 1262, 1160
- NMR (DMSO-d₆, ppm):: 3.80 (2H, d, J = 5.0 Hz), 7.28 - 7.66 (4H, m), 8.90 (1H, t, J = 5.0 Hz)

### Step 4

### Preparation of 1-(7-fluorobenzo[b]furen-2-yl-sulfonyl)-2-thiohydantoin.

To a suspension of the product obtained in Step 3 (5.4 g) and ammonium thiocyanate (3.32 g) in acetic anhydride (12.7 ml) was added dropwise pyridine (4.16 ml) under ice-cooling and nitrogen atmosphere. The mixture was heated with stirring for 2 hours at 70°C. After cooling to room temperature, the resulting solution was poured into ice-water (200 ml) and added small amount of ethanol and the formed precipitate was separated and dissolved in ethyl acetate (200 ml) and the solution was washed with successive water and saturated aqueous NaCl solution. After drying over anhydrous sodium sulfate, ethyl acetate was removed in vacuo and the residue was washed with ethanol to give 2.83 g of the objective compound.
Melting point: 229.9 - 232.0°C
- IR (KBr, cm⁻¹):: 3258, 1765, 1744, 1448, 1177
- NMR (DMSO-d₆, ppm):: 4.73 (2H, s), 7.39 - 7.77 (3H, m), 8.13 (1H, d, J = 2.6 Hz), 12.83 (1H, bs)

### Step 5

### Preparation of 1-(7-fluorobenzo[b]furan-2-yl-sulfonyl)hydantoin.

Starting from the product obtained in Step 4 (2.8 g), the objective compound (1.1 g) was obtained in a manner similar to Step 5 of Example 61.
Melting point: >300°C
- IR (KBr, cm⁻¹):: 3381, 1735, 1610, 1383, 1166
- NMR (DMSO-d₆, ppm):: 3.98 (2H, s), 7.34 - 7.71 (3H, m), 7.78 (1H, d, J = 3.0 Hz)

### Example 64

### Preparation of 1-(4,5-dichlorobenzo[b]furan-2-yl-sulfonyl)hydantoin (compound 29).

### Step 1

### Preparation of (3,4-dichlorophenyloxy)acetaldehyde dimethyl acetal.

Starting from 3,4-dichlorophenol (200 g), the objective compound (218.8 g) was obtained in a manner similar to Step 1 of Example 62.
- IR (neat, cm⁻¹):: 2940, 2830, 1595, 1475, 1297, 1235
- NMR (CDCl₃, ppm):: 3.45 (6H, s), 3.96 (2H, d, J = 5.3 Hz), 4.69 (1H, t, J = 5.3 Hz), 6.78 (1H, dd, J = 8.9, 3.0 Hz), 7.02 (1H, d, J = 3.0 Hz), 7.31 (1H, d, J = 8.9 Hz)

### Step 2

### Preparation of mixture of 4,5-dichlorobenzo[b]-furan and 5,6-dichlorobenzo[b]furan.

Starting from the product obtained in Step 1 (218.8 g), the mixture of the objective compounds (102.1 g) was obtained in a manner similar to Step 2 of Example 62.

### Step 3

### Preparation of 4,5-dichlorobenzo[b]furan-2-yl-sulfonyl chloride and 5,6-dichlorobenzo[b]furan-2-ylsulfonyl chloride.

To a solution of the mixture obtained in step 2 (100 g) in anhydrous ether (440 ml) was added dropwise 1.5 M lithium diisopropylamide mono(tetrahydrofuran) in cyclohexane (440 ml) under nitrogen atmosphere at -70°C over 1 hour, then into the solution was bubbled sulfur dioxide for 1.5 hours at -70°C. After stirring for 1 hour at room temperature, the solvent was removed in vacuo and ether was added to the residue. The formed precipitate was separated by filtration to give a mixture of lithium 4,5-dichlorobenzo[b]furan-2-sulfinate and lithium 5,6-dichlorobenzo[b]furan-2-sulfinate. To the suspension of the products in dichloromethane (1.8 l) was added N-chlorosuccinimide (92.1 g) at -50°C and stirred for 1.5 hours. At room temperature, insoluble matters were filtered off and dichloromethane was removed in vacuo and the residue was purified by silica gel column chromatography to give 17.4 g of 4,5-dichlorobenzo[b]furan-2-ylsulfonyl chloride and 7.4 g of 5,6-dichlorobenzo[b]furan-2-yl-sulfonyl chloride, respectively.
4,5-dichlorobenzo[b]furan-2-ylsulfonyl chloride
Melting point: 114.6°C
- IR (KBr, cm⁻¹):: 1529, 1444, 1401, 1191
- NMR (DMSO-d₆, ppm):: 6.87 (1H, d, J = 1.0 Hz), 7.55 (1H, d, J = 8.9 Hz), 7.69 (1H, dd, J = 8.9, 1.0 Hz)
5,6-dichlorobenzo[b]furan-2-ylsulfonyl chloride
Melting point: 159.8°C
- IR (KBr, cm⁻¹):: 1537, 1390, 1163, 1081
- NMR (DMSO-d₆, ppm):: 6.87 (1H, d, J = 1.0 Hz), 7.92 (1H, s), 8.02 (1H, d, J =1.0 Hz)

### Step 4

### Preparation of N-(4,5-dichlorobenzo[b]furan-2-yl-sulfonyl)glycine ethyl ester.

Starting from 4,5-dichlorobenzo[b]furan-2-yl-sulfonyl chloride obtained in Step 3 (17 g), the objective compound (18.2 g) was obtained in a manner similar to Step 2 of Example 61.
Melting point: 155.2 - 155.5°C
- IR (KBr, cm⁻¹):: 3199, 1737, 1225, 1160
- NMR (CDCl₃, ppm):: 1.05 (3H, t, J = 7.1 Hz), 3.92 (2H, s), 3.95 (2H, q, J = 7.1 Hz), 7.56 (1H, s), 7.78 (2H, s), 9.09 (1H, bs)

### Step 5

### Preparation of N-(4,5-dichlorobenzo[b]furan-2-yl-sulfonyl)glycine.

Starting from the product obtained in Step 4 (18 g), the objective compound (16.2 g) was obtained in a manner similar to Step 3 of Example 61.
Melting point: 189.8 - 194.7°C
- IR (KBr, cm⁻¹):: 3320, 1719, 1366, 1256, 1162
- NMR (DMSO-d₆, ppm):: 3.83 (2H, d, J = 6.3 Hz), 7.56 (1H, S), 7.76 (2H, s), 8.97 (1H, t, J = 6.3 Hz)

### Step 6

### Preparation of 1-(4,5-dichlorobenzo[b]furan-2-yl-sulfonyl)-2-thiohydantoin.

Starting from the product obtained in Step 5 (16 g), the objective compound (7.4 g) was obtained in a manner similar to Step 4 of Example 61.
Melting point: 214.6 - 217.5°C
- IR (KBr, cm⁻¹):: 1793, 1762, 1445, 1167
- NMR (DMSO-d₆, ppm):: 4.77 (2H, s), 7.85 (2H, s), 8.11 (1H, s), 12.95 (1H, bs)

### Step 7

### Preparation of 1-(4,5-dichlorobenzo[b]furan-2-yl-sulfonyl)hydantoin.

To a suspension of iodine monochloride (6.3 ml) in 1 M hydrochloric acid (150 ml) were added successively the product obtained in Step 6 (7.3 g) and dropwise dichloromethane (150 ml) over 10 minutes. The mixture was stirred for 2.5 hours at room temperature. Under ice-cooling, to the solution was added saturated aqueous sodium sulfite solution and stirred for a while. The formed precipitate was separated by filtration and washed with successive water, ethanol and ether to give 4.8 g of the objective compound.
Melting point: 290.7 - 292.0°C (decomposition)
- IR (KBr, cm⁻¹):: 3256, 1742, 1391, 1356,1168
- NMR (DMSO-d₆, ppm):: 4.47 (2H, s), 7.85 (2H, s), 7.98 (1H, s), 11.80 (1H, bs)

### Example 65

### Preparation of 1-(5,6-dichlorobenzo[b]furan-2-yl-sulfonyl)hydantoin (compound 30).

### Step 1

### Preparation of N-(5,6-dichlorobenzo[b]furan-2-yl-sulfonyl)glycine ethyl ester.

To a solution of 5,6-dichlorobenzo[b]furan-2-yl-sulfonyl chloride obtained in Step 3 of Example 64 (7.4 g) in dichloromethane (60 ml) was added glycine ethyl ester hydrochloride (7.95 g) and added slowly triethylamine (7.89 ml) under ice-cooling and nitrogen atmosphere. The resulting solution was poured into water (100 ml) and acidified with 1 M hydrochloric acid and extracted with ethyl acetate. The organic layer was washed with saturated aqueous NaCl solution and dried over anhydrous sodium sulfate. Ethyl acetate was removed in vacuo and the residue was washed with hexane to give 8.7 g of the objective compound.
Melting point: 132.7 - 133.5°C
- IR (KBr, cm⁻¹):: 3227, 1735, 1360, 1225, 1158
- NMR (DMSO-d₆, ppm):: 1.06 (3H, t, J = 6.9 Hz), 3.90 (2H, s), 3.95 (2H, q, J = 6.9 Hz), 7.52 (1H, s), 8.08 (1H, s), 8.20 (1H, s), 9.05 (1H, bs)

### Step 2

### Preparation of N-(5,6-dichlorobenzo[b]furan-2-yl-sulfonyl)glycine.

Starting from the product obtained in Step 1 (8.6 g), the objective compound (7.8 g) was obtained in a manner similar to Stop 3 of Example 61.
Melting point: 192.6 - 201.8°C
- IR (KBr, cm⁻¹):: 3367, 1719, 1359, 1248, 1159
- NMR (DMSO-d₆, ppm):: 3.80 (2H, d, J - 5.9 Hz), 7.51 (1H, d, J = 1.0 Hz), 8.08 (1H, s), 8.19 (1H, d, J = 1.0 Hz), 8.92 (1H, t, J = 5.9 Hz)

### Step 3

### preparation of 1-(5,6-dichlorobenzo[b]furan-2-yl-sulfonyl)-2-thiohydantoin.

Starting from the product obtained in Step 2 (7.7 g), the objective compound (3.7 g) was obtained in a manner similar to Step 4 of Example 61.
Melting point: >246.0°C (decomposition)
- IR (KBr, cm⁻¹):: 3100, 1743, 1449, 1246, 1166
- NMR (DMSO-d₆, ppm):: 4.73 (2H, s), 8.02 (1H, d, J = 1.0 Hz), 8.20 (1H, s), 8.26 (1H, d, J = 1.0 Hz), 12.82 (1H, bs)

### Step 4

### Preparation of 1-(5,6-dichlorobenzo[b]furan-2-yl-sulfonyl)hydantoin.

Starting from the product obtained in Step 3 (3.7 g), the objective compound (2.7 g) was obtained in a manner similar to Step 5 of Example 61.
Melting point: >300°C (decomposition)
- IR (KBr, cm⁻¹):: 1732, 1389, 1186, 1167
- NMR (DMSO-d₆, ppm):: 4.31 (2H, s), 7.82 (1H, d, J = 0.7 Hz), 8.16 (1H, s), 8.27 (1H, d, J = 0.7 Hz)

### Example 66

### Preparation of 1-(3-bromo-7-fluorobenzo[b]furan-2-ylsulfonyl)hydantoin (compound 34).

### Step 1

### Preparation of 2,3-dibromo-2,3-dihydro-7-fluorobenzo[b]furan.

To a solution of 7-fluorobenzo[b]furan (16 g) in carbon tetrachloride (40 ml) was added dropwise a solution of bromine (22 g) in carbon disulfide (40 ml) at -30°C and the solution was stirred for 1 hour. At room temperature, the formed precipitate was separated by filtration to give 34.4 g of the objective compound.
- IR (KBr, cm⁻¹):: 1634, 1601, 1489, 1459, 1279, 1179
- NMR (CDCl₃, ppm):: 5.74 (1H, d, J =1.3 Hz), 6.93 (1H, s), 7.11 - 7.35 (3H, m)

### Step 2

### Preparation of 3-bromo-7-fluorobenzo[b]furan.

To a solution of potassium hydroxide (12.7 g) in ethanol (180 ml) was slowly added the product obtained in Step 1 (34 g) and stirred for 3 hours. The resulting solution was neutralized by acetic acid, then extracted with ether. The organic layer was washed with successive water and saturated aqueous NaCl solution. After drying over anhydrous sodium sulfate, ether was removed in vacuo to give 24.1 g of the objective compound.
- IR (neat, cm⁻¹):: 3150, 1636, 1595, 1494, 1434, 1322
- NMR (CDCl₃, ppm):: 6.98 - 7.36 (3H, m), 7.68 (1H, s)

### Step 3

### Preparation of 3-bromo-7-fluorobenzo[b]furan-2-ylsulfonyl chloride.

Starting from the product obtained in Step 2 (24.1 g), the objective compound (12.2 g) was obtained in a manner similar to Step 1 of Example 61.
- IR (KBr, cm⁻¹):: 1602, 1533, 1385, 1168
- NMR (DMSO-d₆, ppm):: 7.33 - 7.39 (3H, m)

### Step 4

### Preparation of N-(3-bromo-7-fluorobenzo[b]furan-2-ylsulfonyl)glycine ethyl ester.

Starting from the product obtained in Step 3 (12.2 g), the objective compound (10.2 g) was obtained in a manner similar to Step 2 of Example 61.
Melting point: 126.2 - 126.4°C
- IR (KBr, cm⁻¹):: 3200, 1731, 1366, 1237, 1142
- NMR (DMSO-d₆, ppm):: 1.01 (3H, t, J = 7.1 Hz), 3.89 (2H, q, J = 7.1 Hz), 3.96 (2H, d, J = 5.6 Hz), 7.47 - 7.66 (3H, m), 9.32 (1H, t, J = 5.6 Hz)

### Step 5

### Preparation of N-(3-bromo-7-fluorobenzo[b]furan-2-ylsulfonyl)glycine.

Starting from the product obtained in Step 4 (10.2 g), the objective compound (7.25 g) was obtained in a manner similar to Step 3 of Example 61.
Melting point: 148.5 - 159.6°C
- IR (KBr, cm⁻¹):: 3223, 1716, 1373, 1246, 1163
- NMR (DMSO-d₆, ppm):: 3.86 (2H, s), 7.46 - 7.58 (3H, m), 9.18 (1H, bs)

### Step 6

### Preparation of 1-(3-bromo-7-fluorobenzo[b]furan-2-ylsulfonyl)-2-thiohydantoin.

Starting from the product obtained in Step 5 (7.2 g), the objective compound (5.07 g) was obtained in a manner similar to Step 4 of Example 61.
Melting point: 224.3 - 224.7°C (decomposition)
- IR (KBr, cm⁻¹):: 3290, 1793, 1765, 1235, 1141
- NMR (DMSO-d₆, ppm):: 4.83 (2H, s), 7.57 - 7.72 (3H, m), 12.93 (1H, bs)

### Step 7

### Preparation of 1-(3-bromo-7-fluorobenzo[b]furan-2-ylsulfonyl)hydantoin.

To a suspension of iodine monochloride (3.3 ml) in 1 M hydrochloric acid (110 ml) was added the product obtained in Step 6 (5 g) and dropwise dichloromethane (140 ml). The mixture was stirred for 6 hours at room temperature and then additive iodine monochloride (1.7 ml) was added to the mixture and the resulting mixture was stirred for 1 hour. To the resulting solution was added saturated aqueous sodium sulfite solution and formed precipitate was separated by filtration. The organic layer was washed with saturated aqueous NaCl solution and dried over anhydrous magnesium sulfate. Formed precipitate was suspended in 1 M hydrochloric acid (100 ml) and the suspension was extracted with ethyl acetate. The organic layer was washed with saturated aqueous NaCl solution and dried over anhydrous magnesium sulfate. Both extracts were combined and the solvent was removed in vacuo. The resulting residue was washed with successive ethanol and ether to give 1.66 g of the objective compound.
Melting point: 266.6 - 270.6°C
- IR (KBr, cm⁻¹):: 3160, 1725, 1393, 1184, 1149
- NMR (DMSO-d₆, ppm):: 4.50 (2H, s), 7.53 - 7.77 (3H, m), 11.85 (1H, bs)

Compounds of Example 67 to 72 prepared in a manner similar to Example 61 are summarized in the following table 5 together with corresponding IR and NMR data and melting points.

Intermediate compounds of Example 3 to 39, 41 to 44, 51, 52 and 67 to 72 are summarized in the following table 6 to 10 together with corresponding IR and NMR data and melting points.

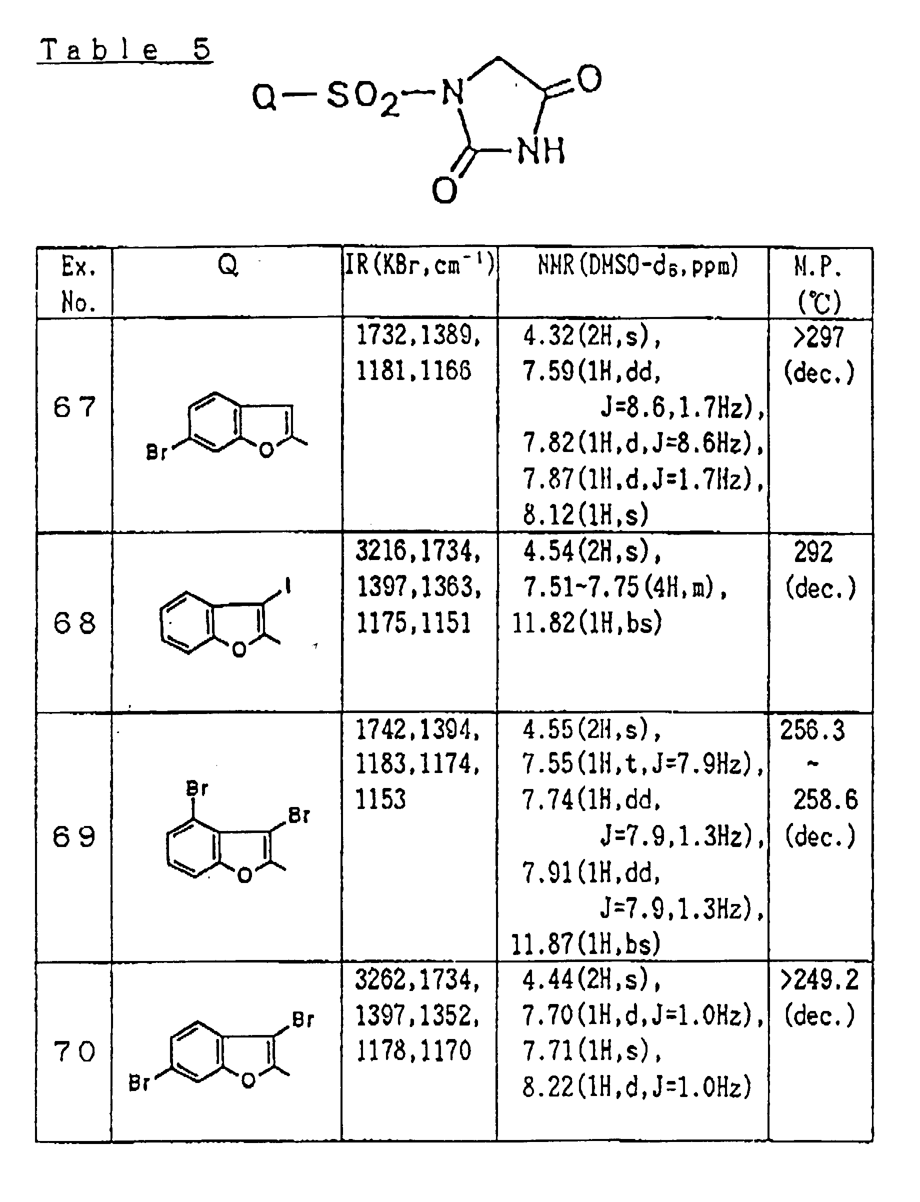

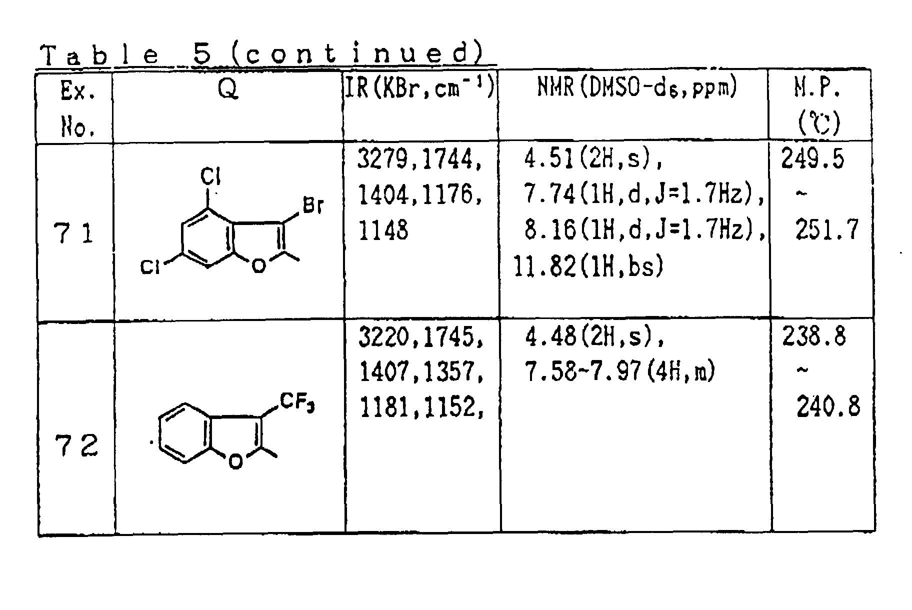

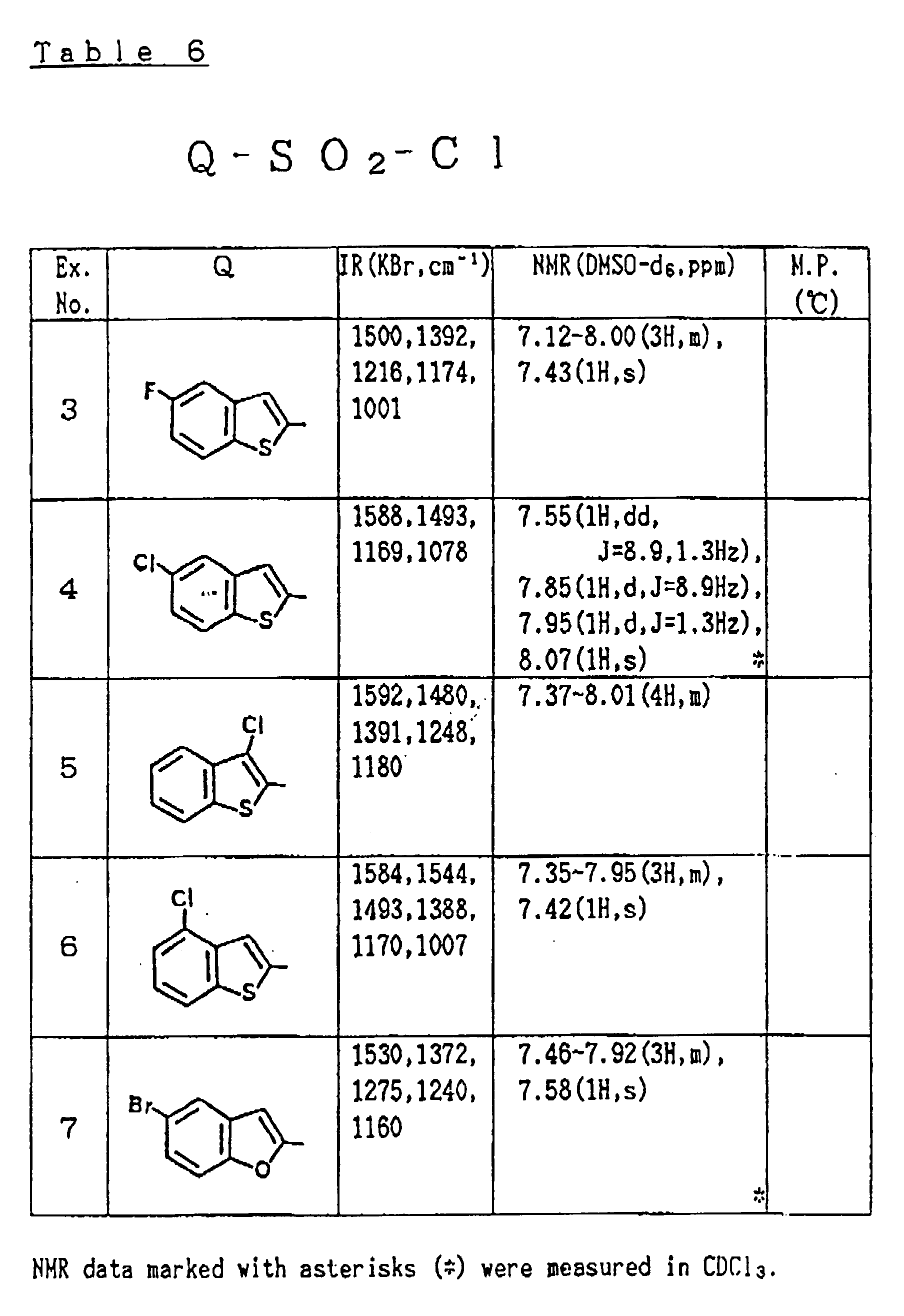

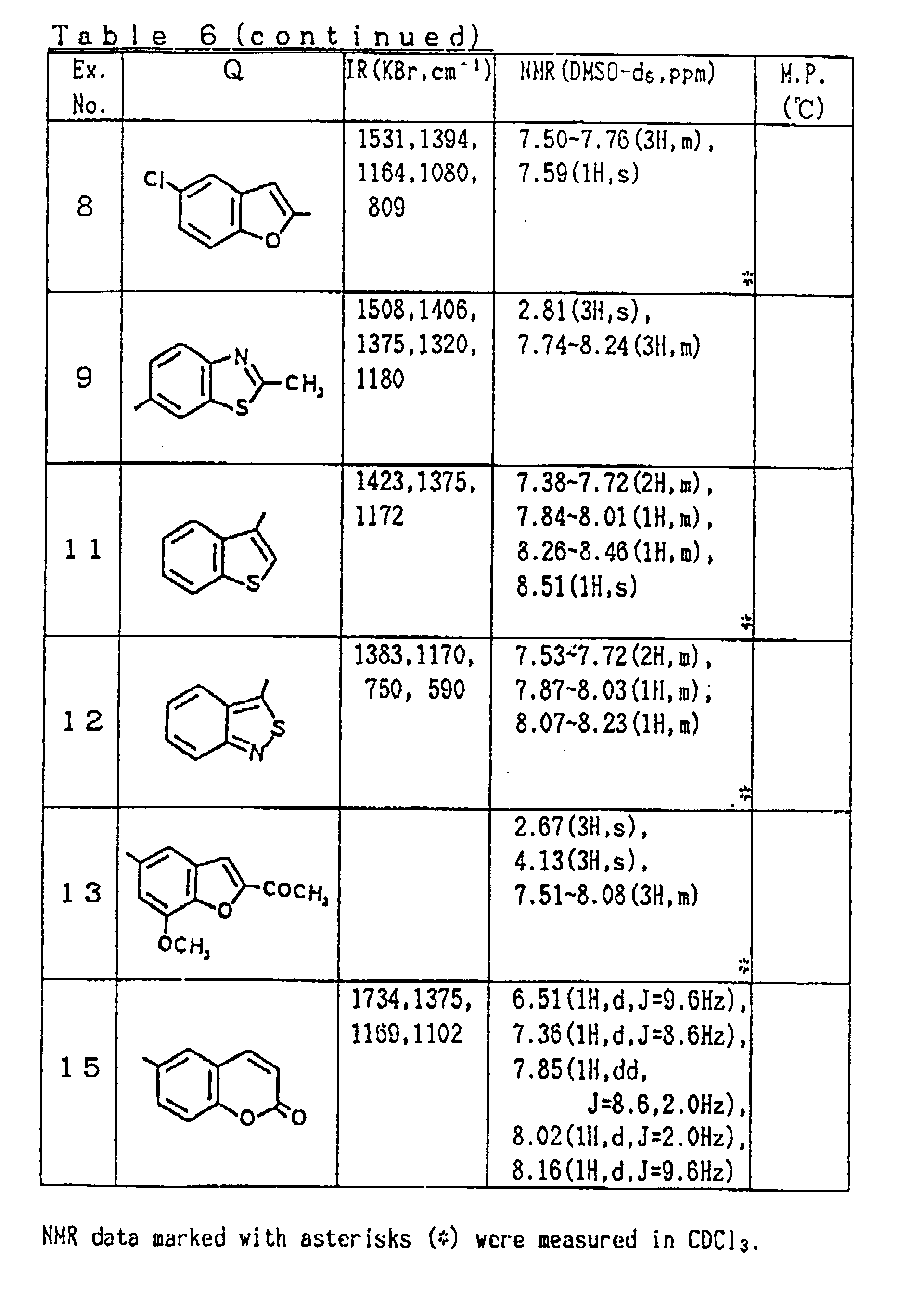

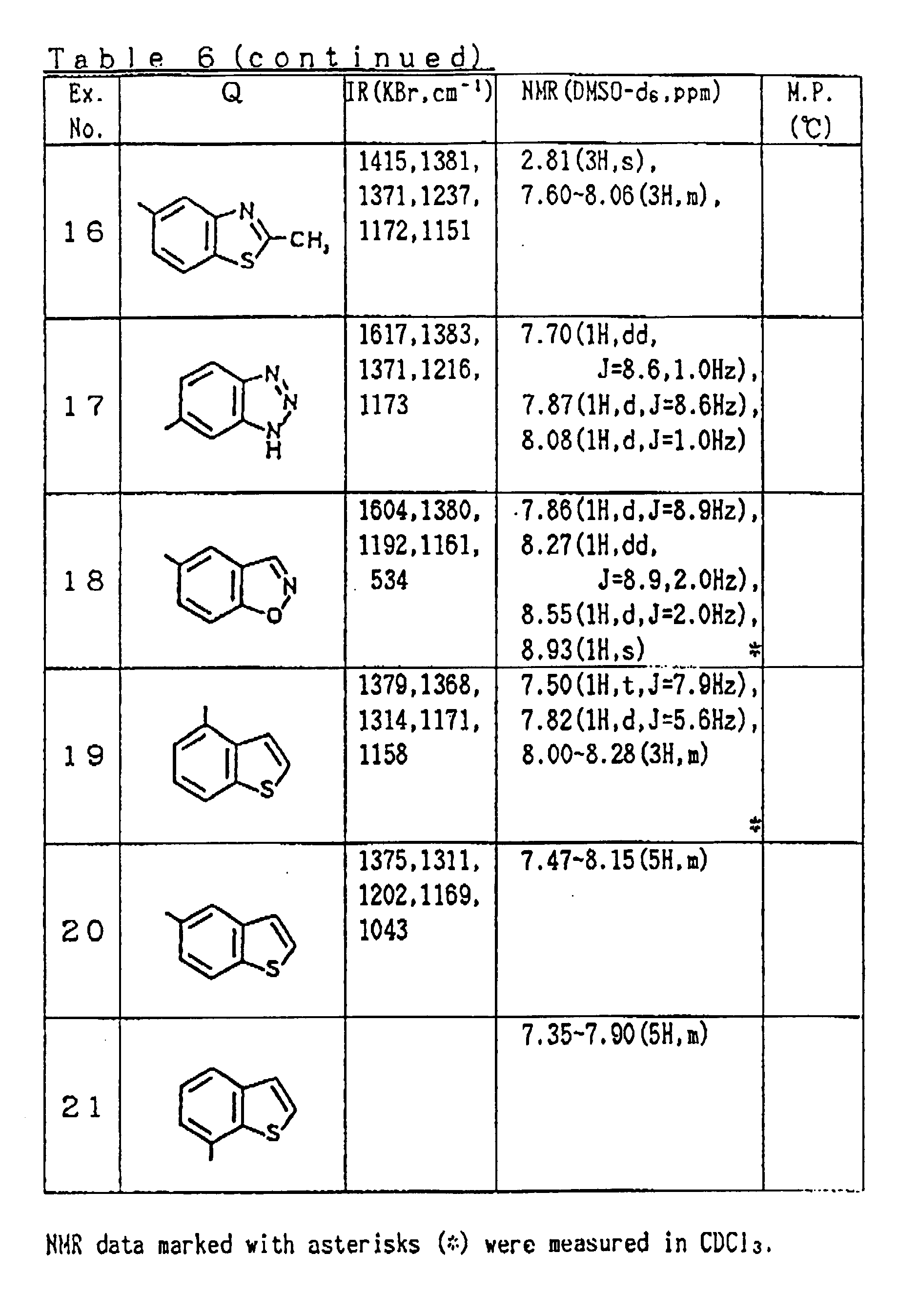

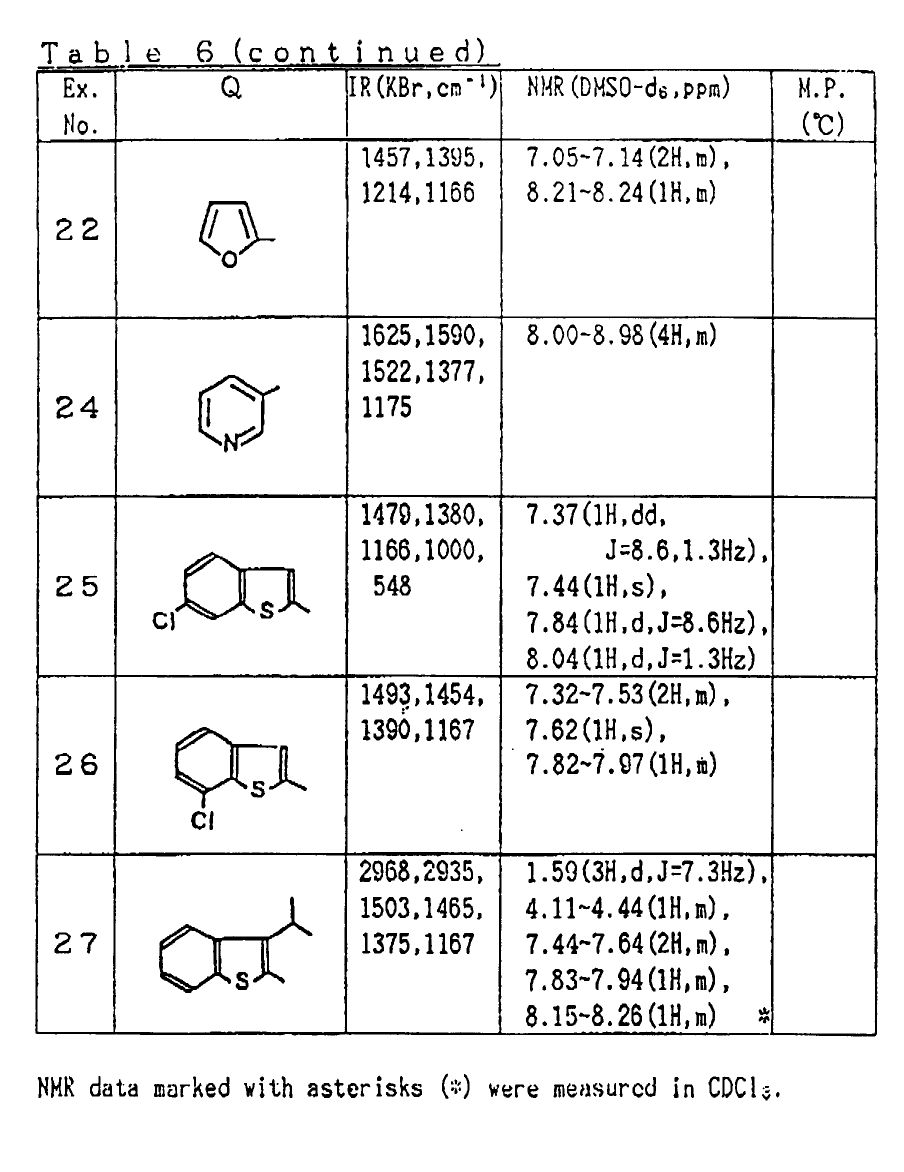

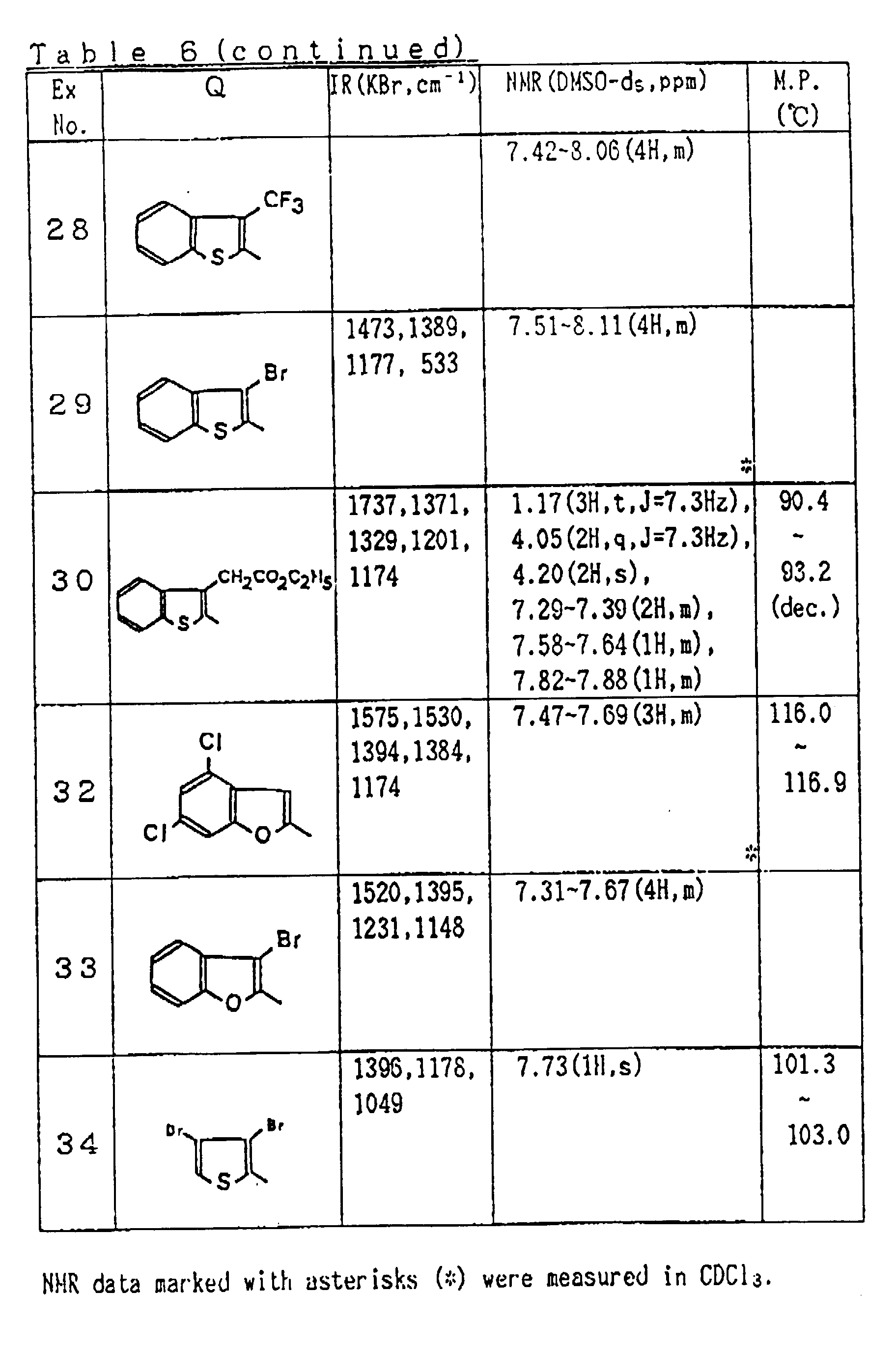

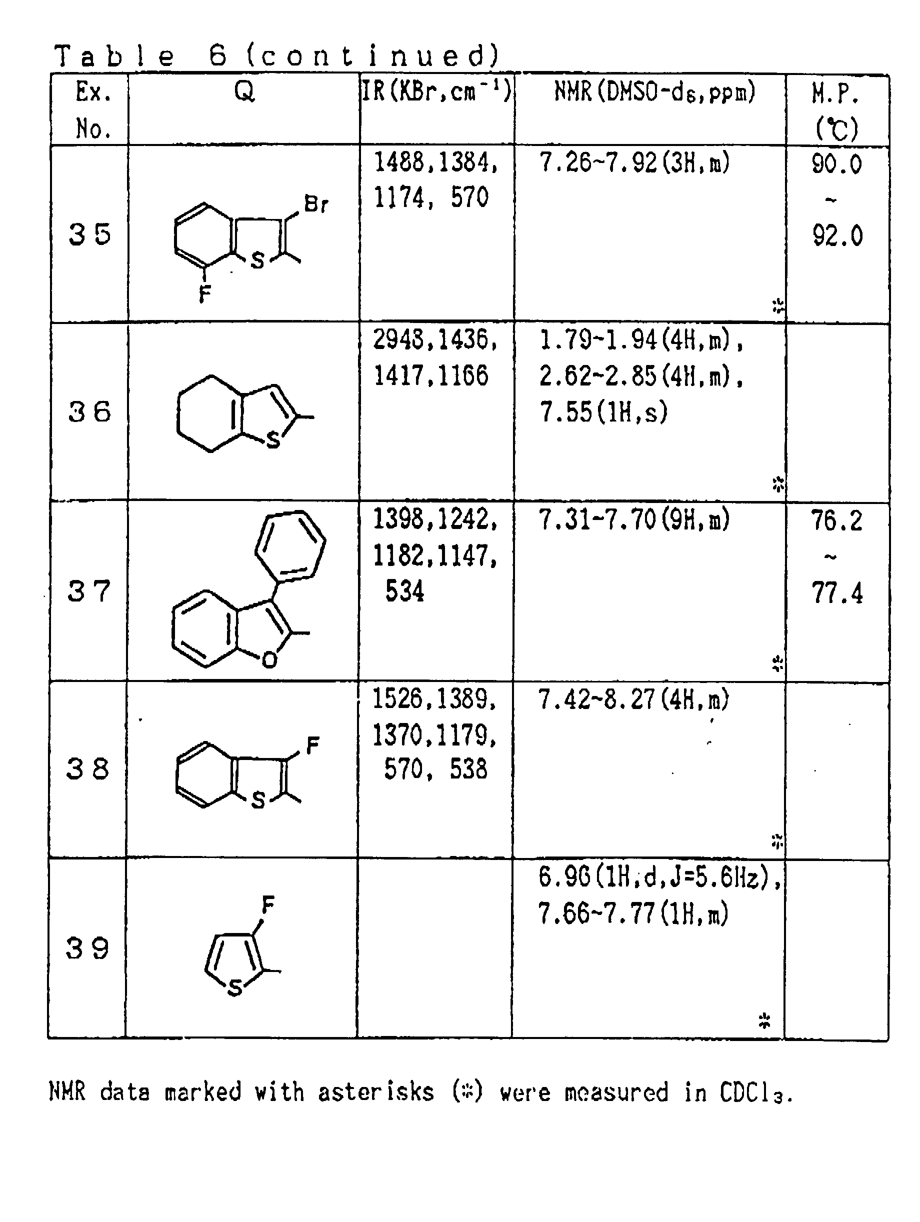

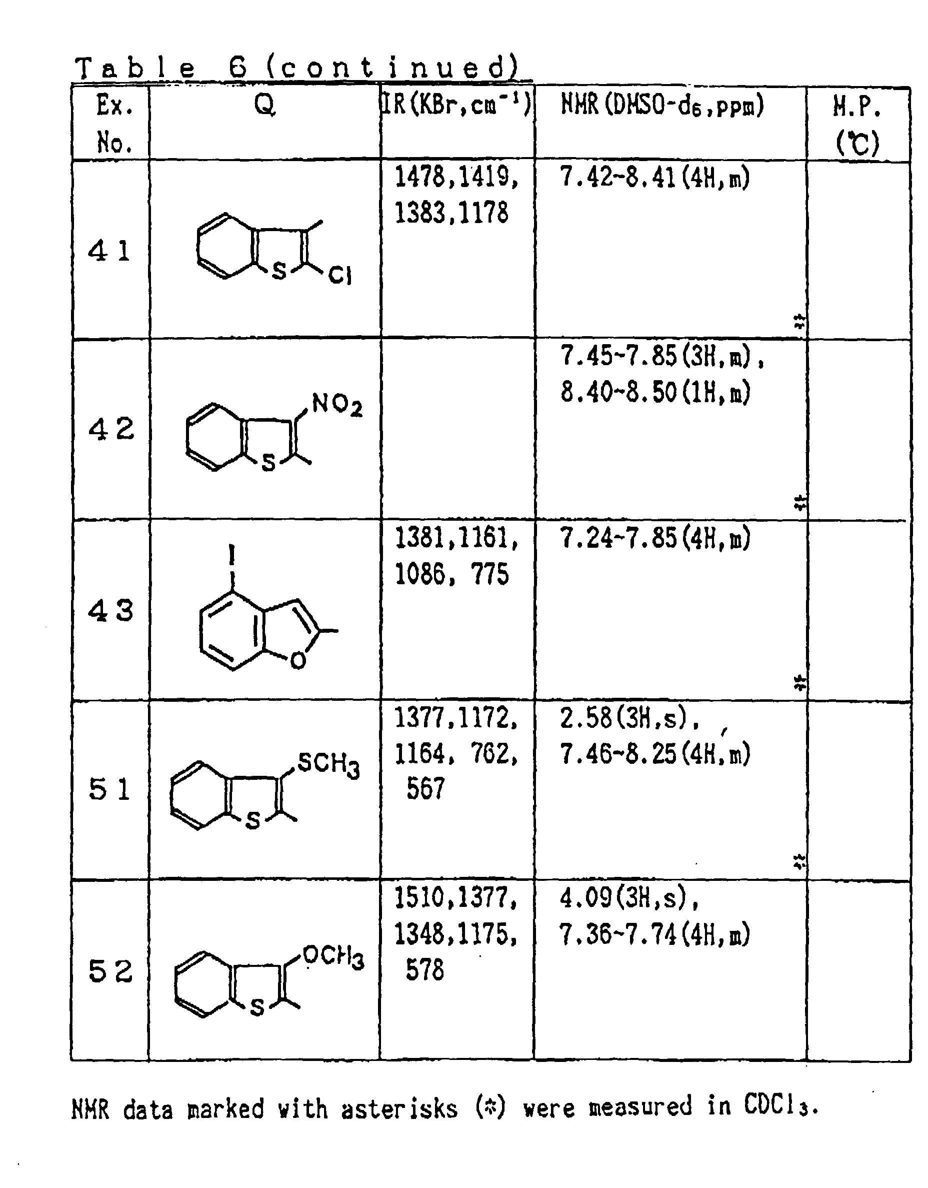

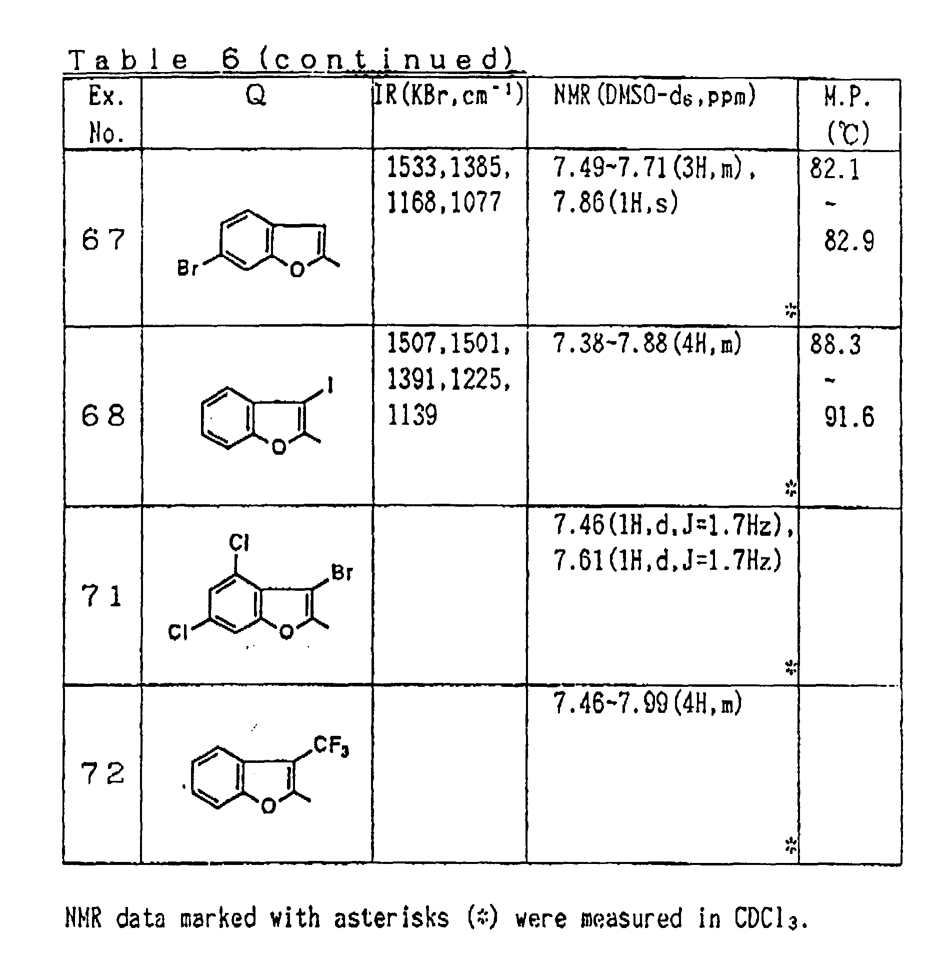

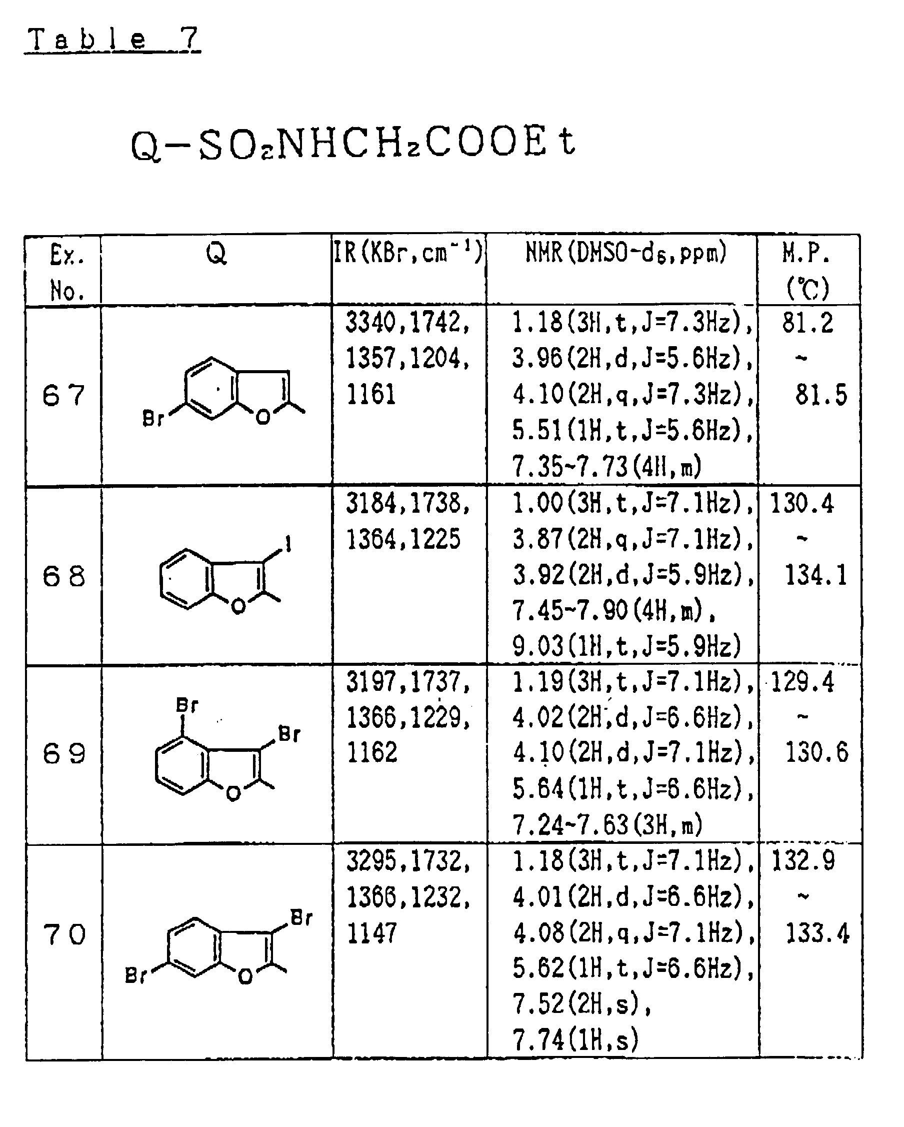

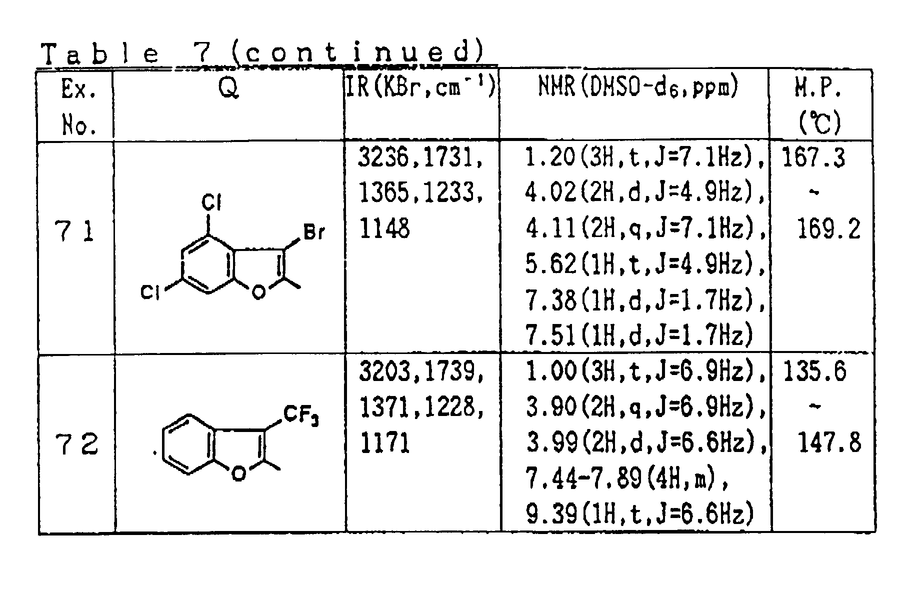

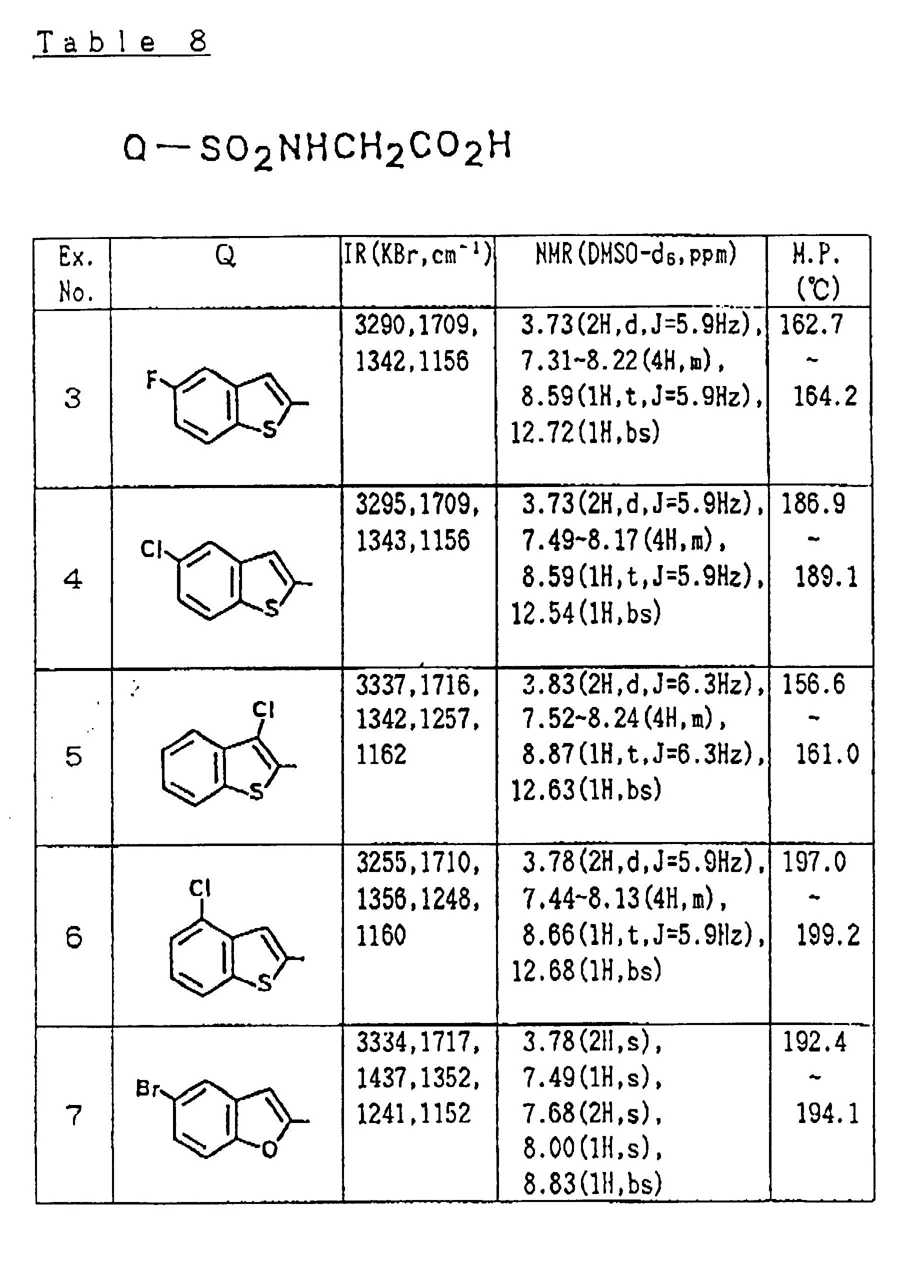

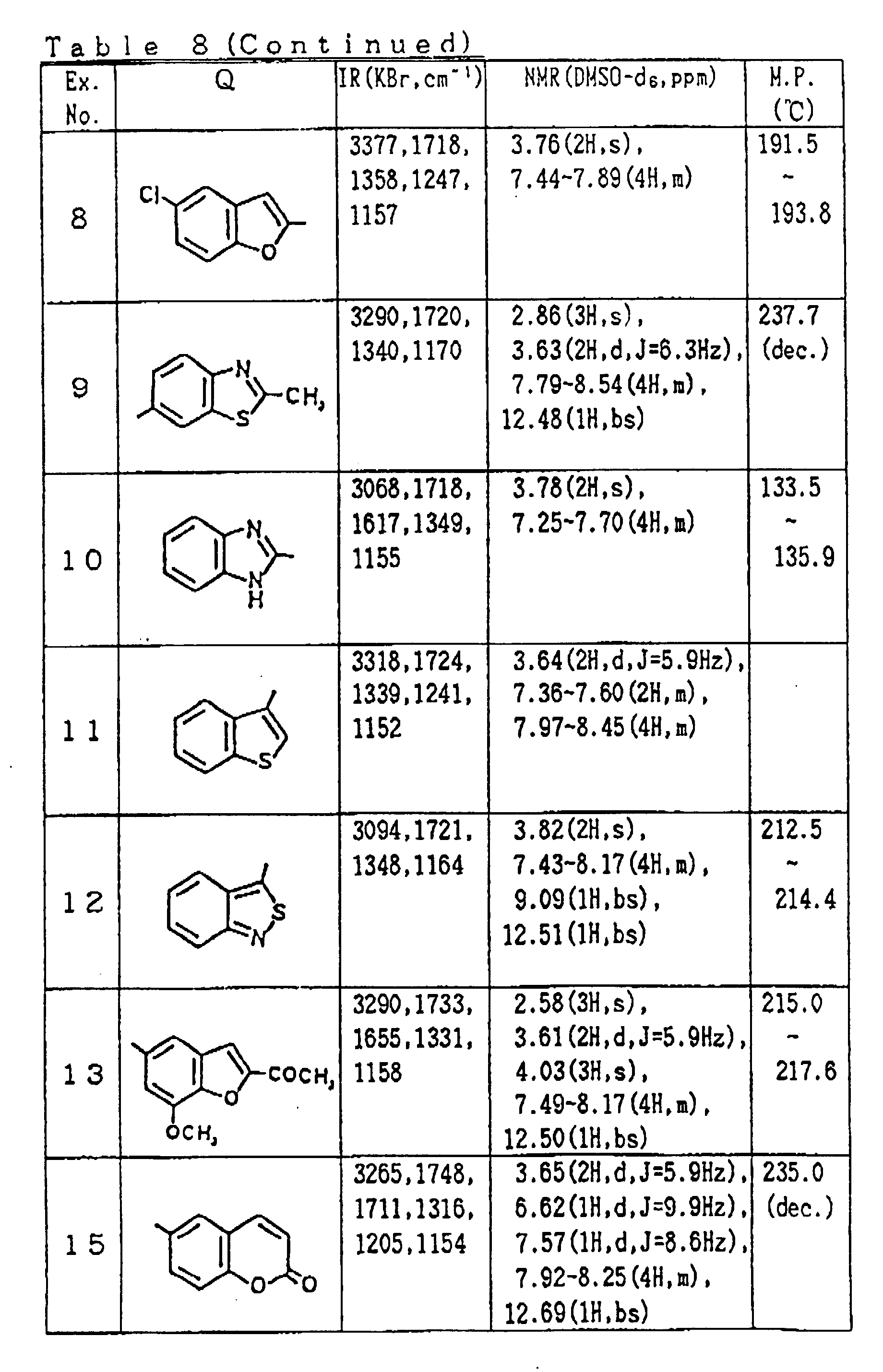

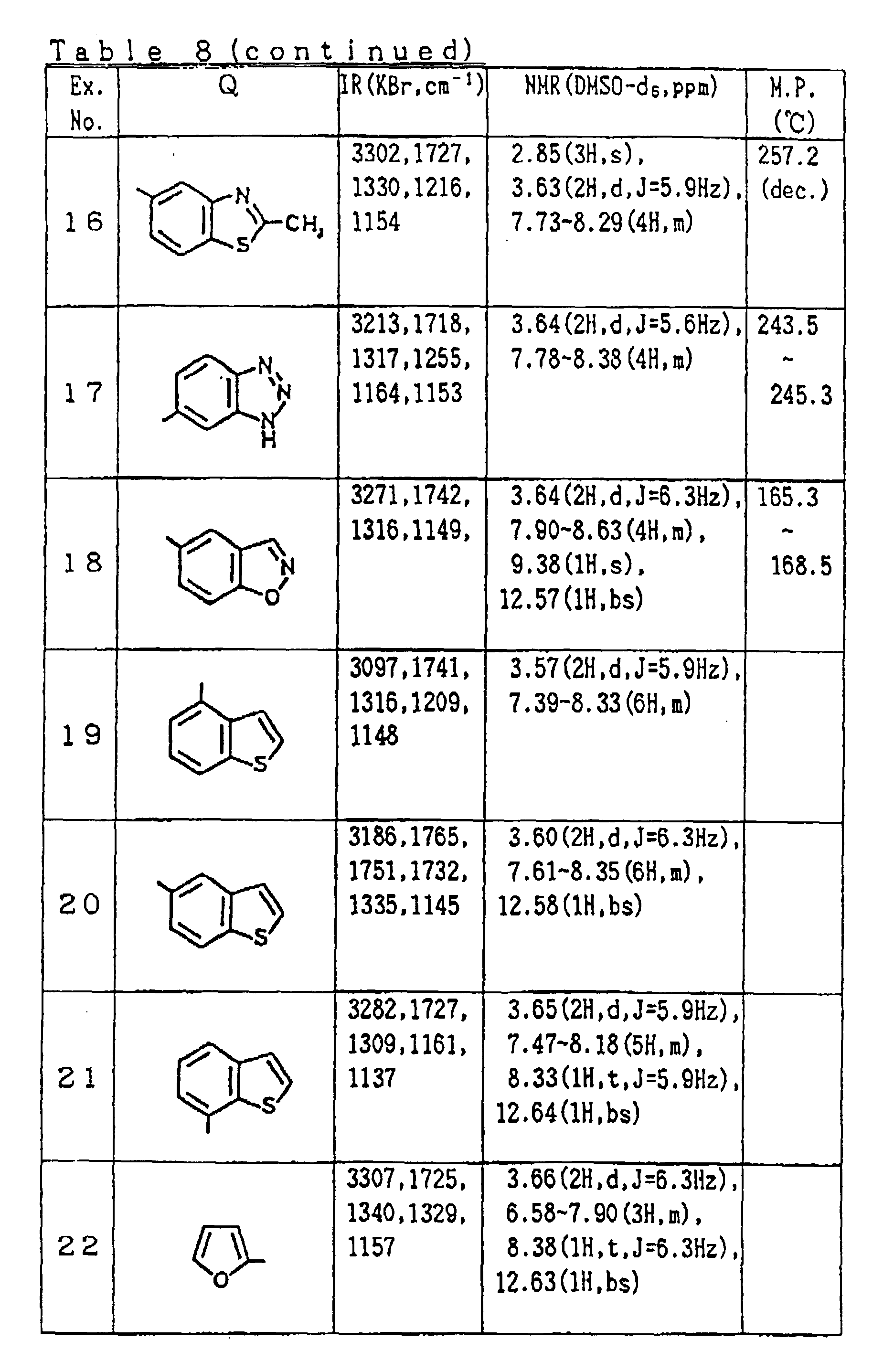

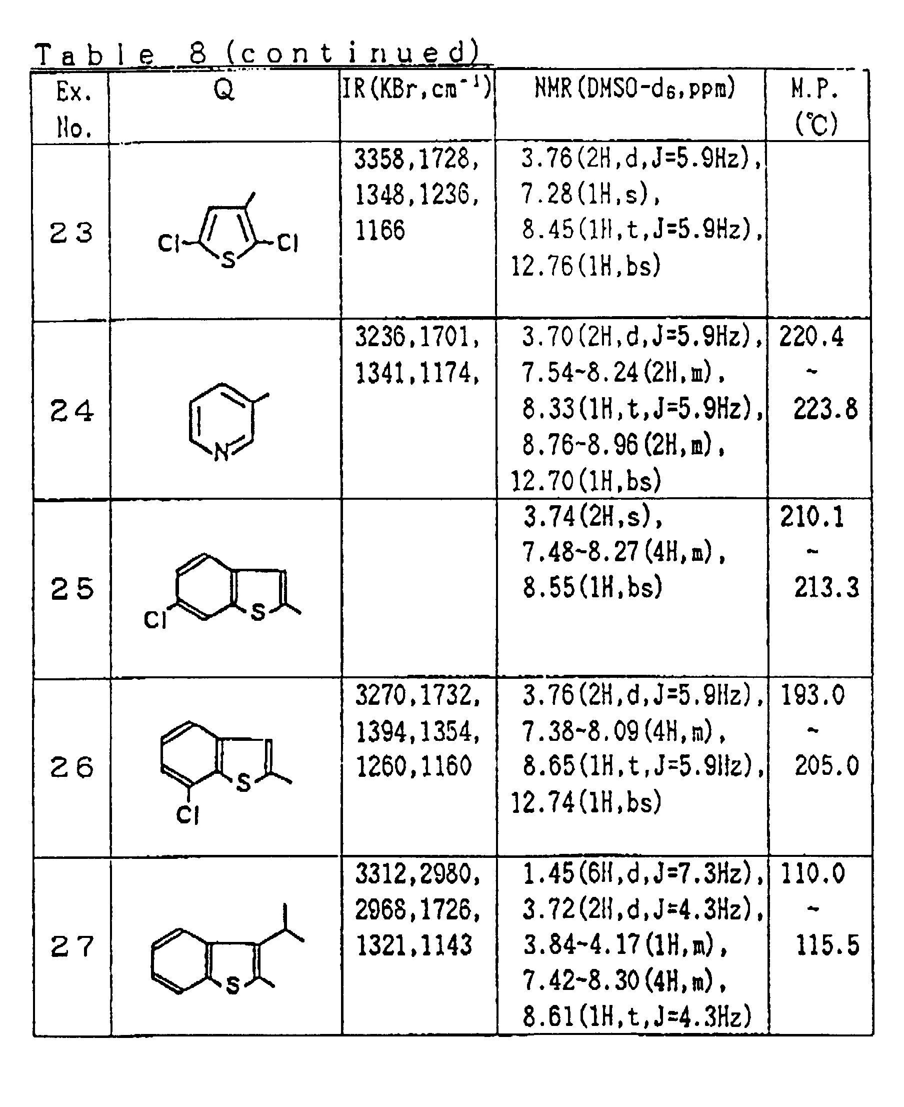

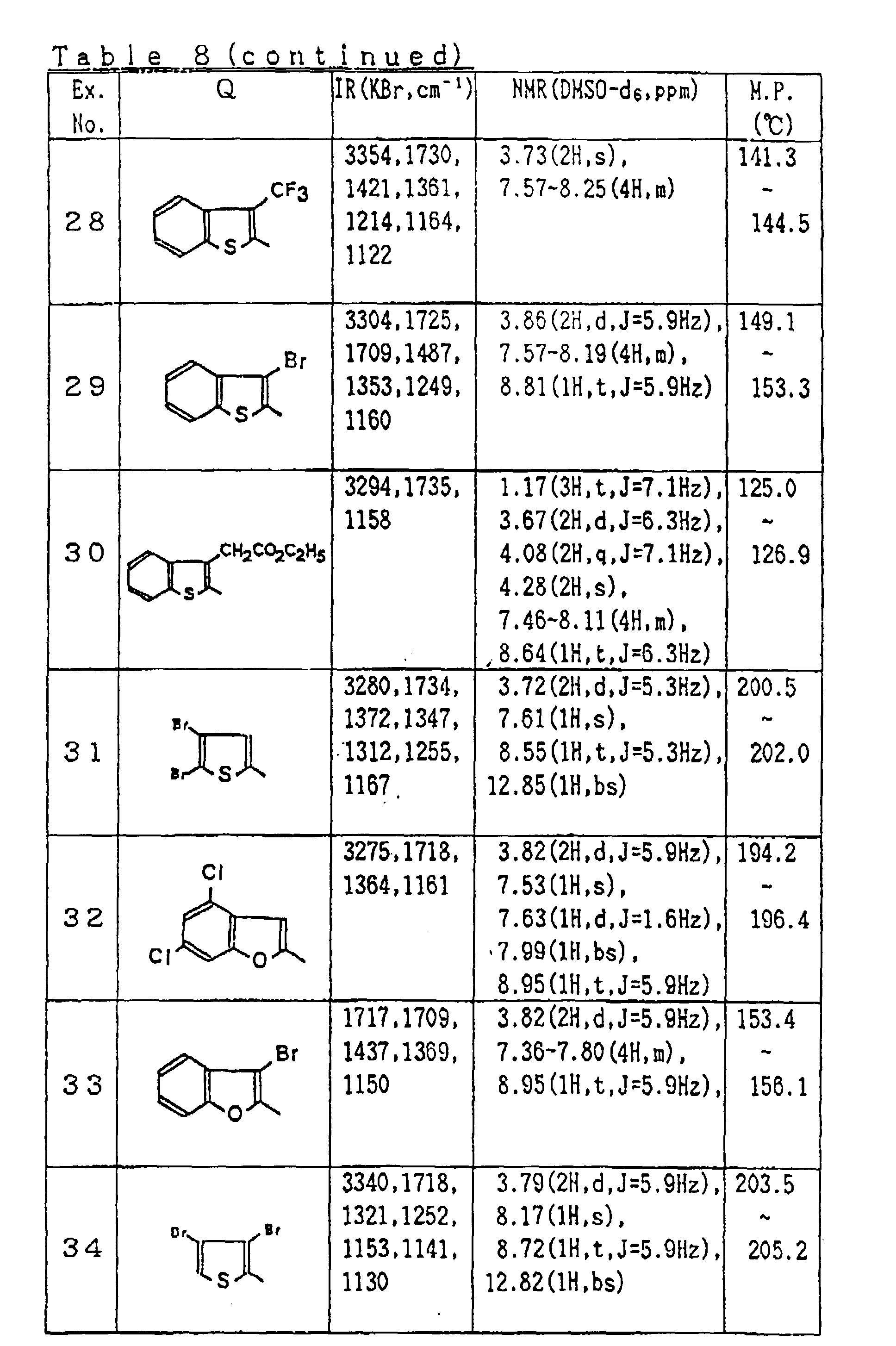

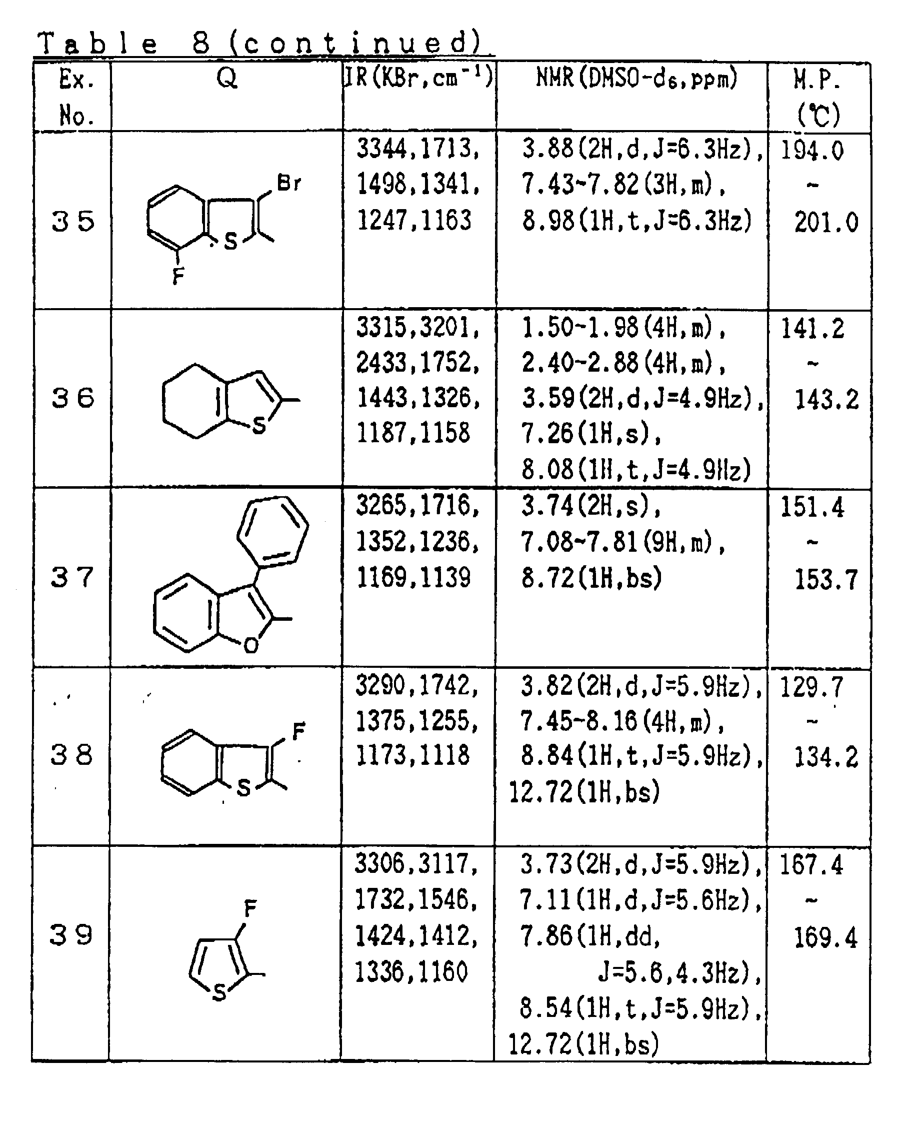

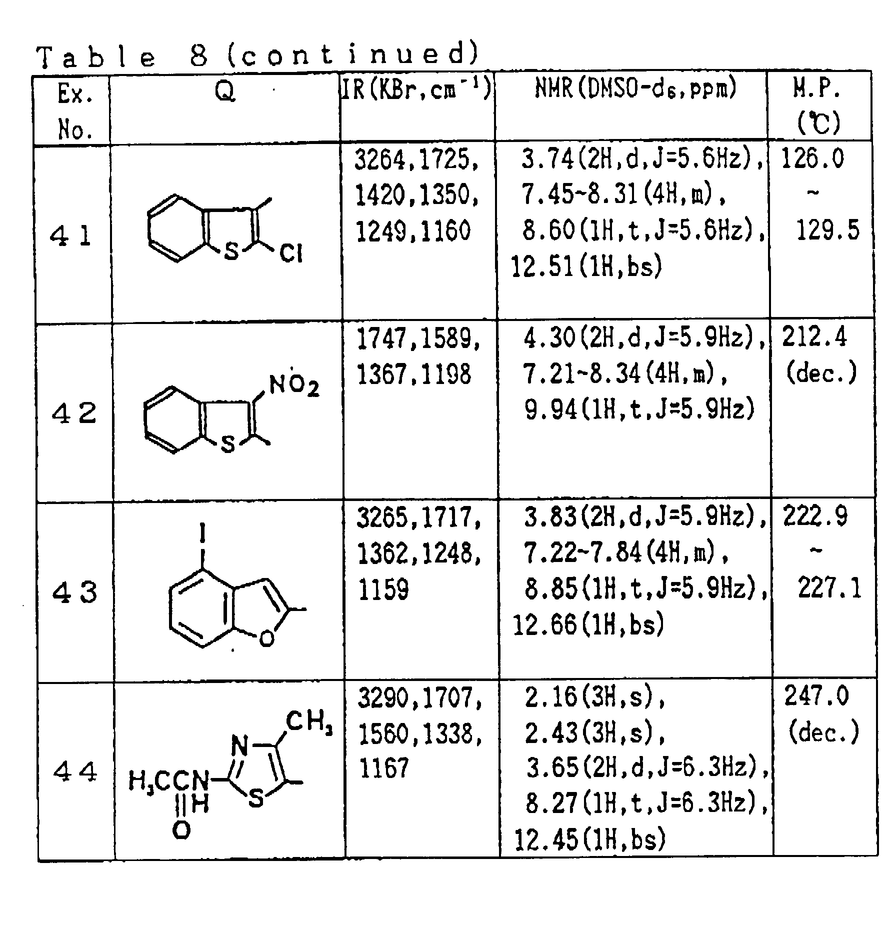

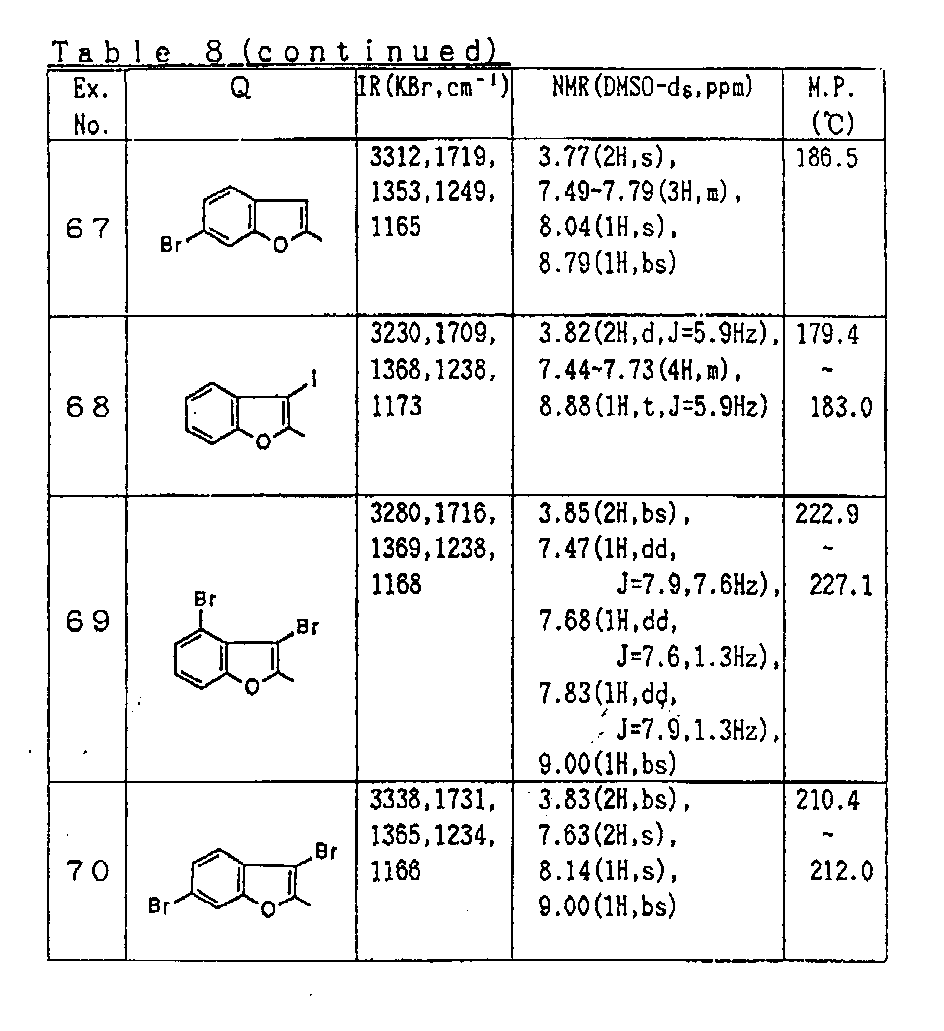

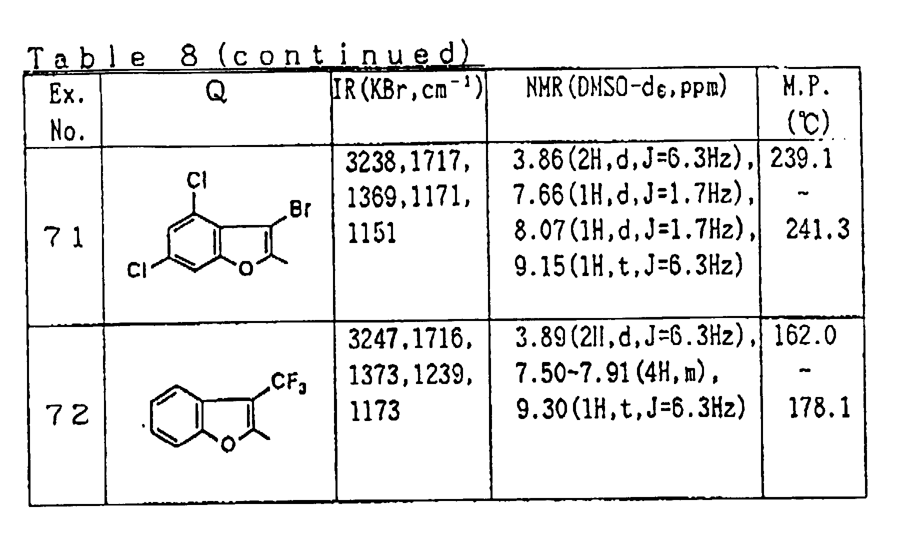

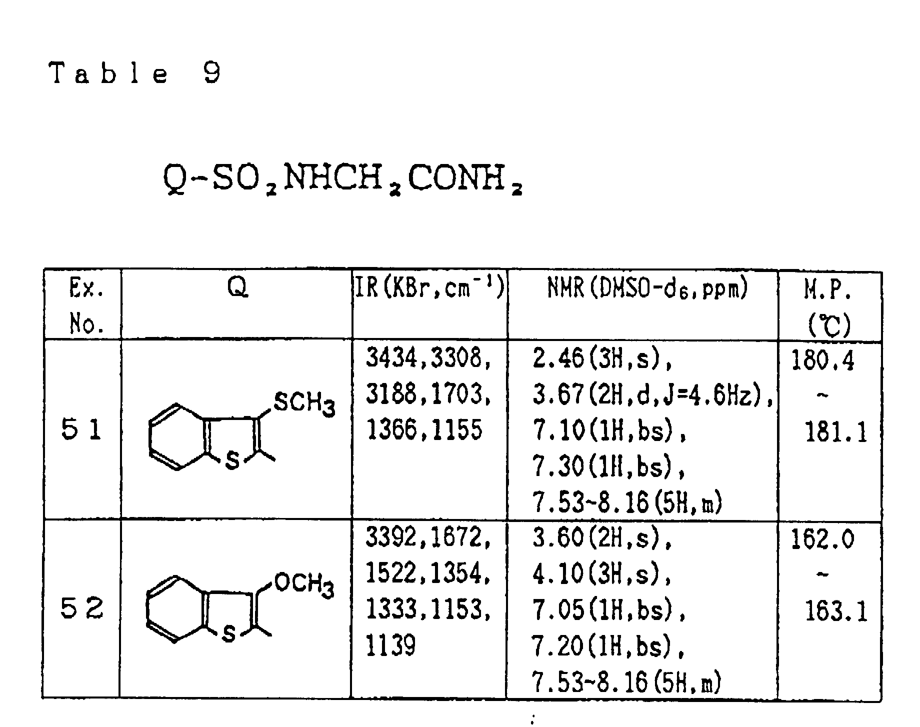

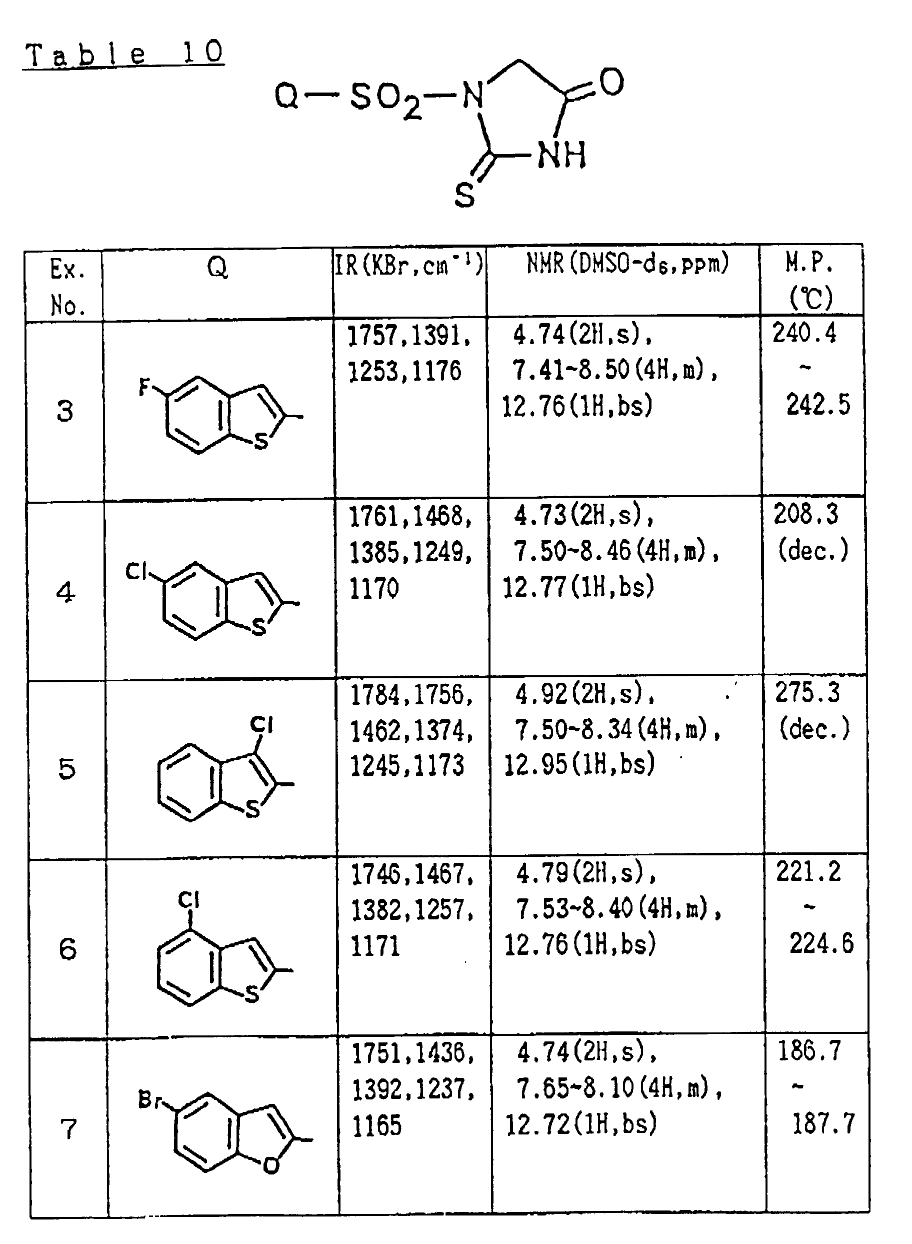

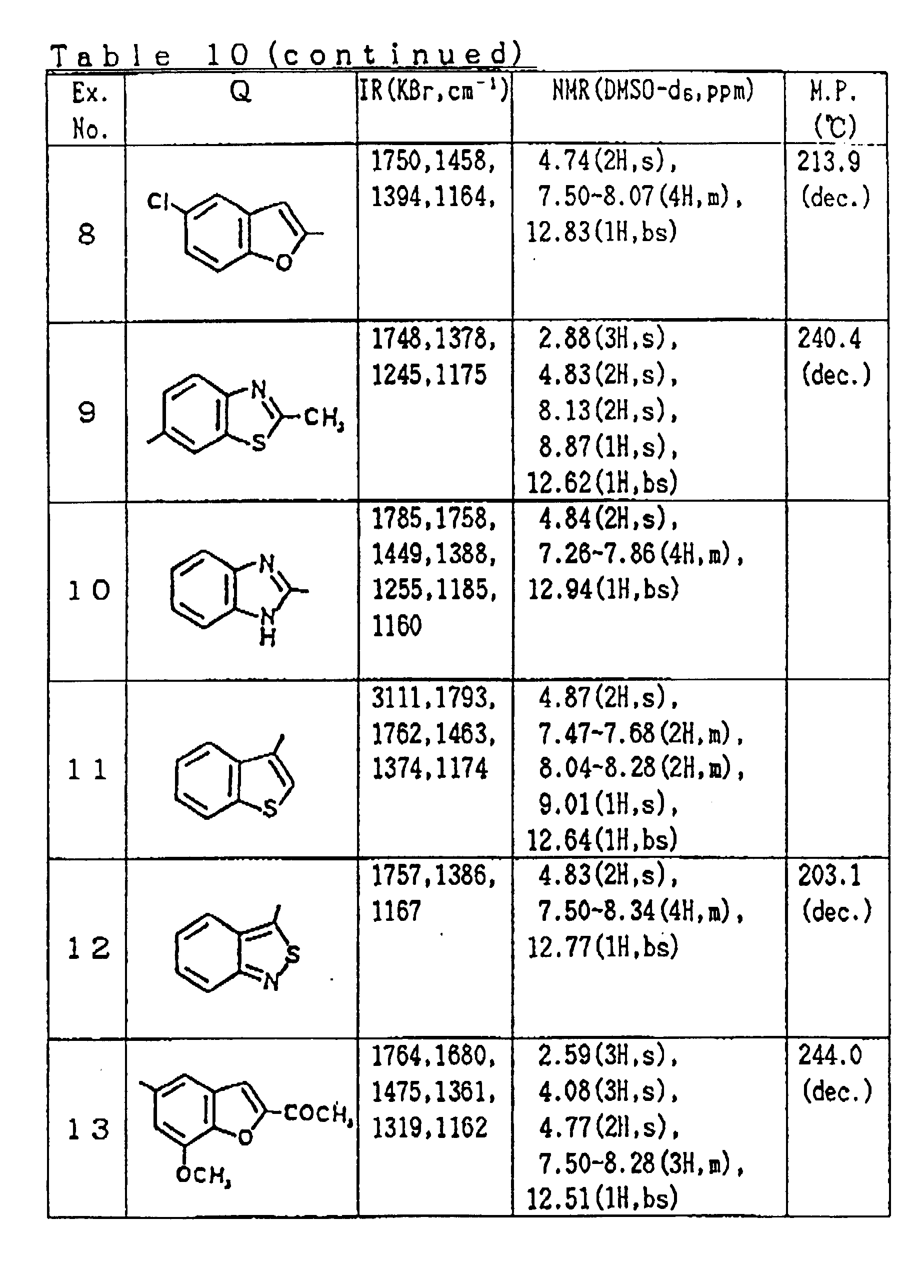

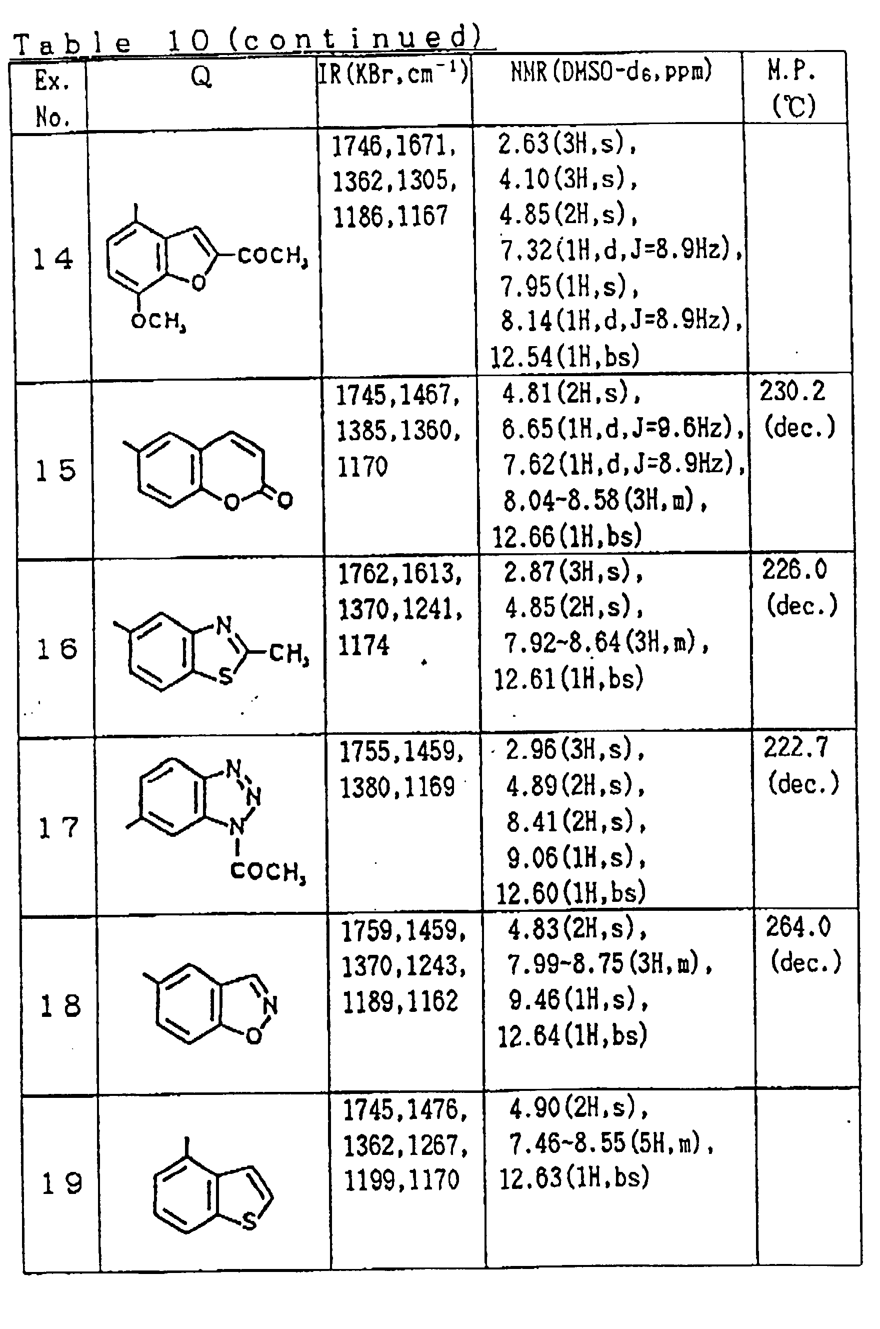

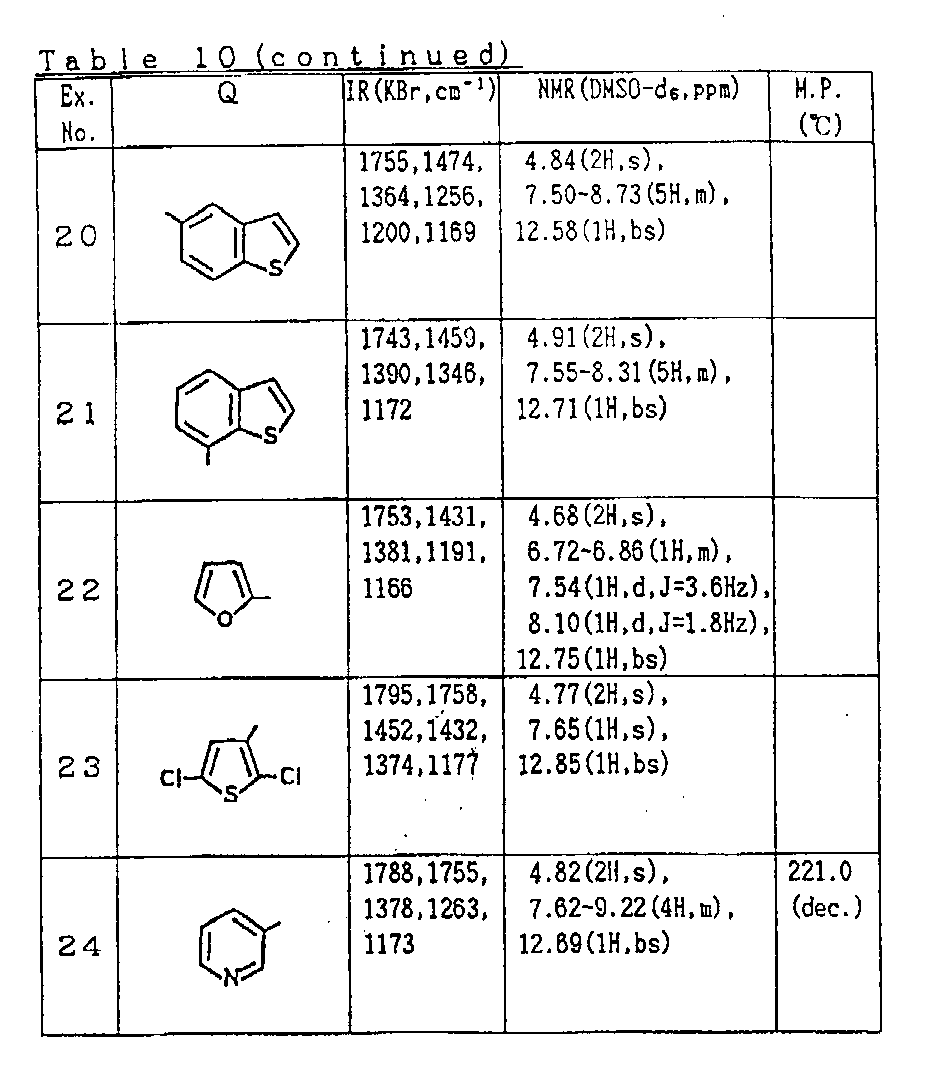

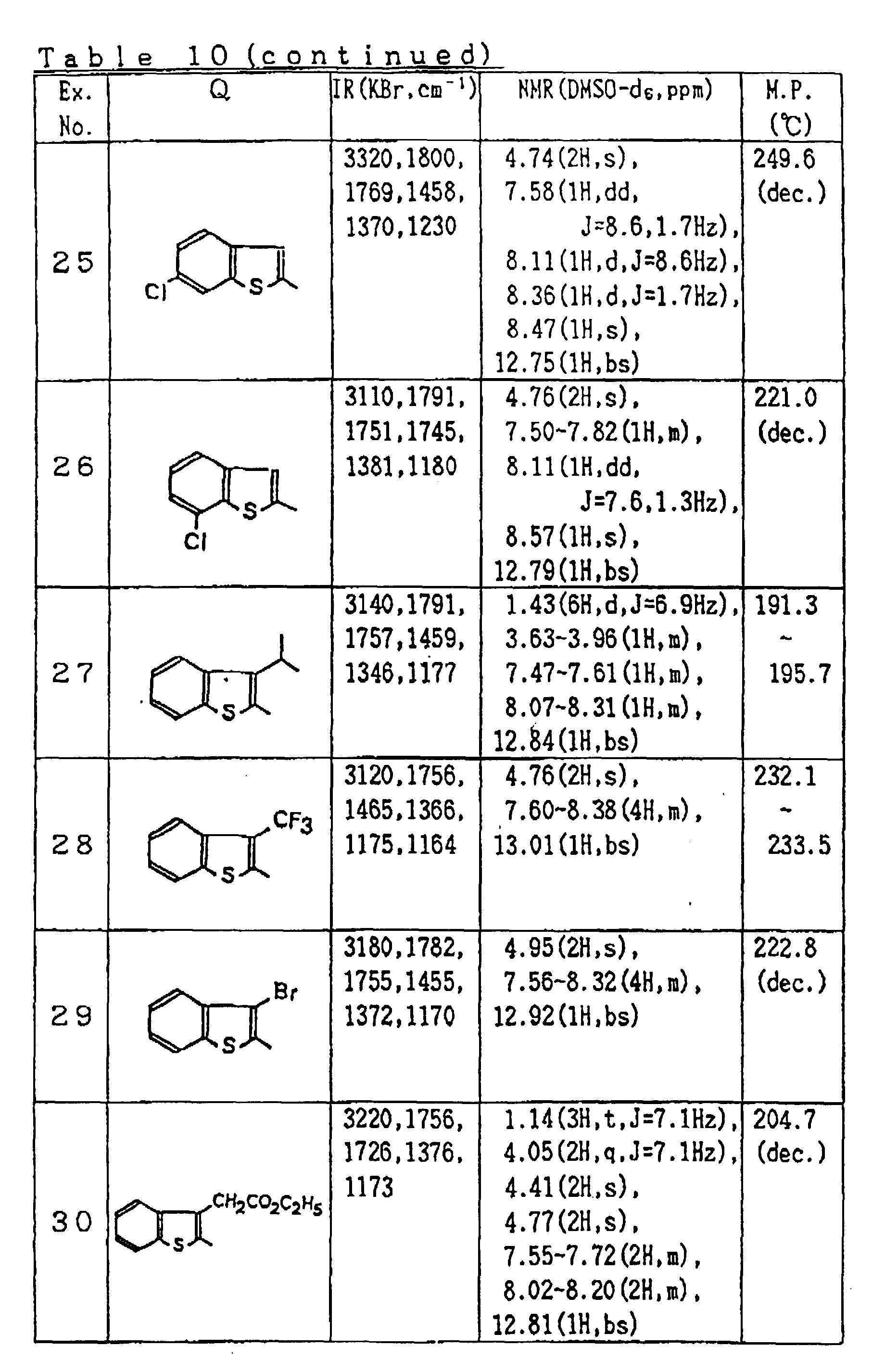

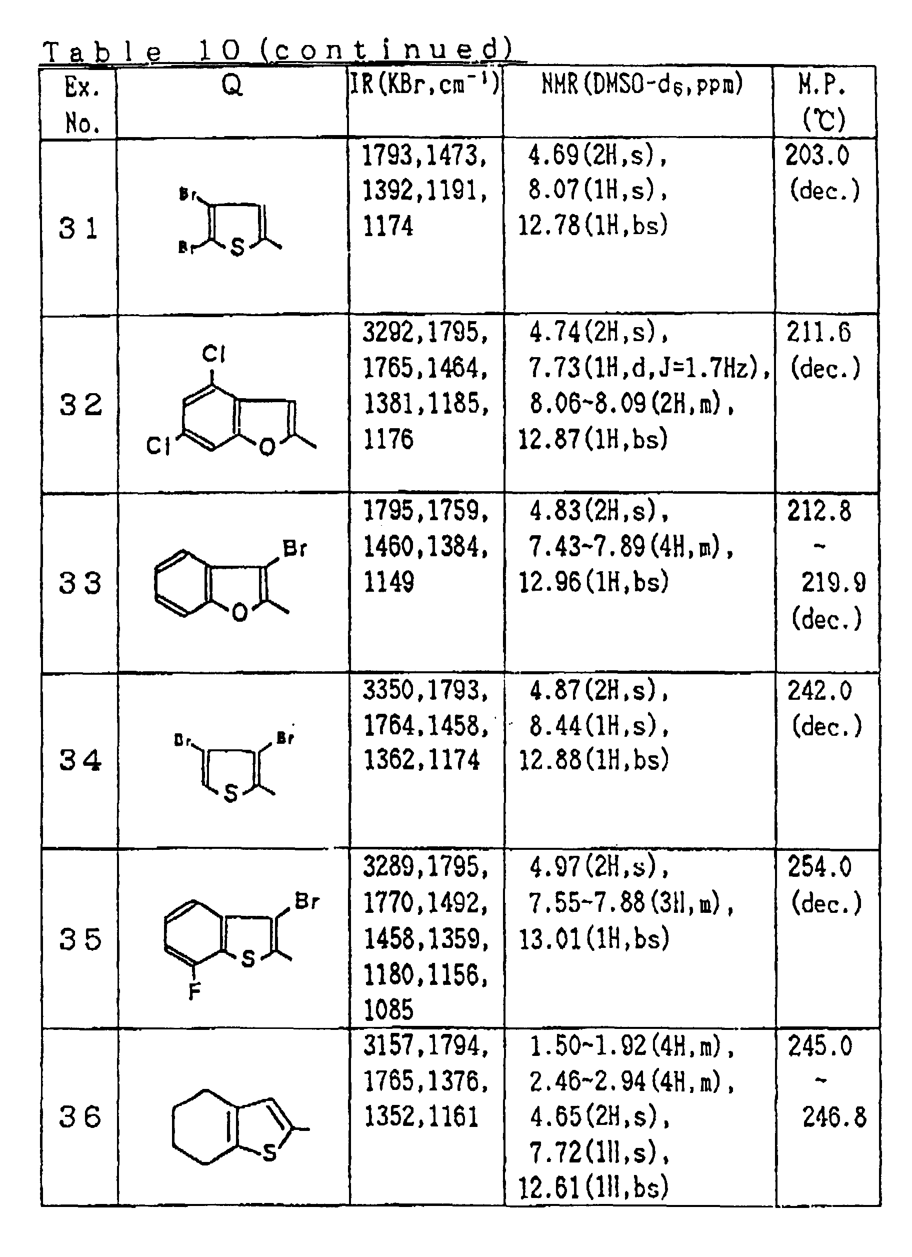

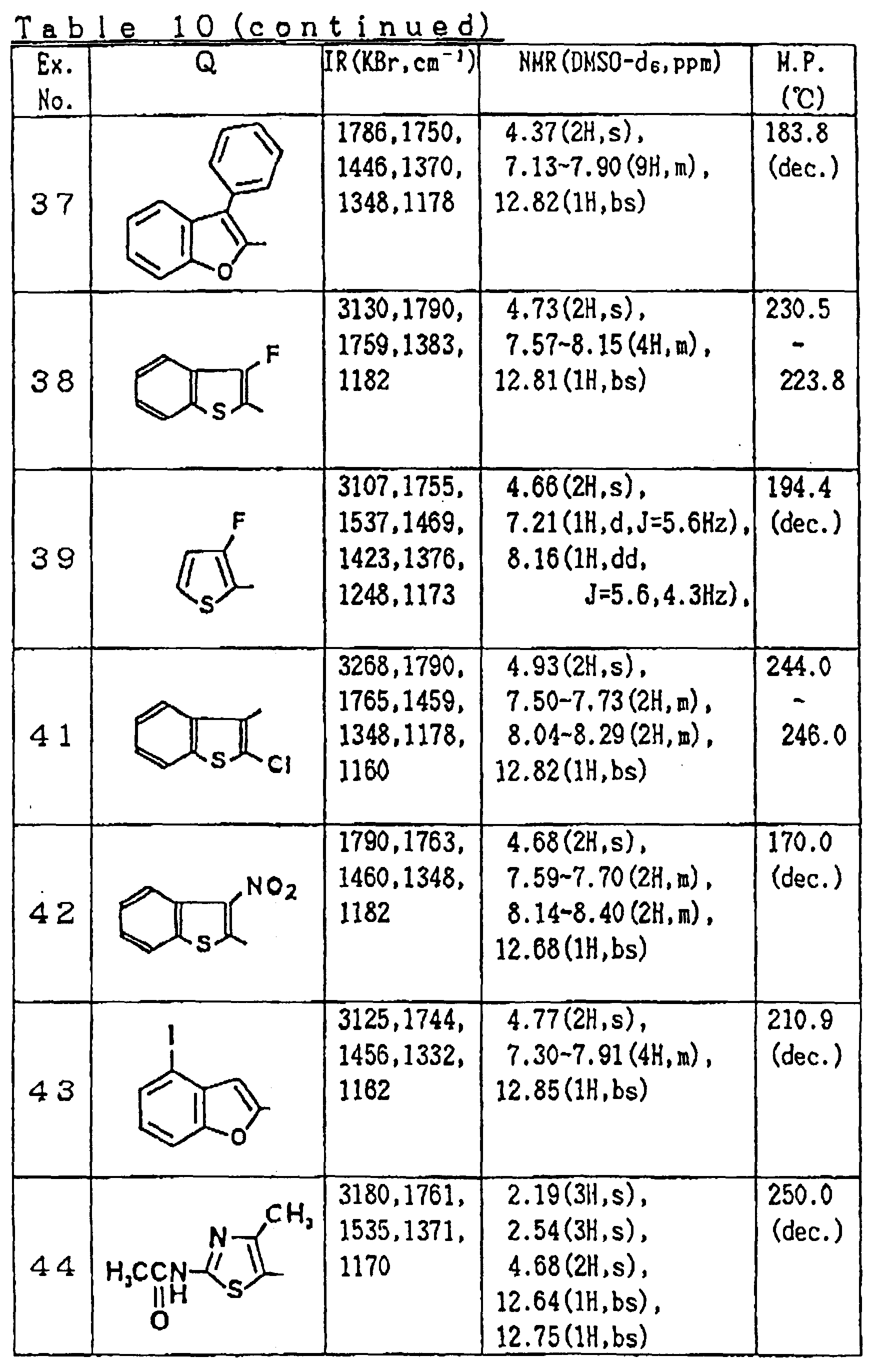

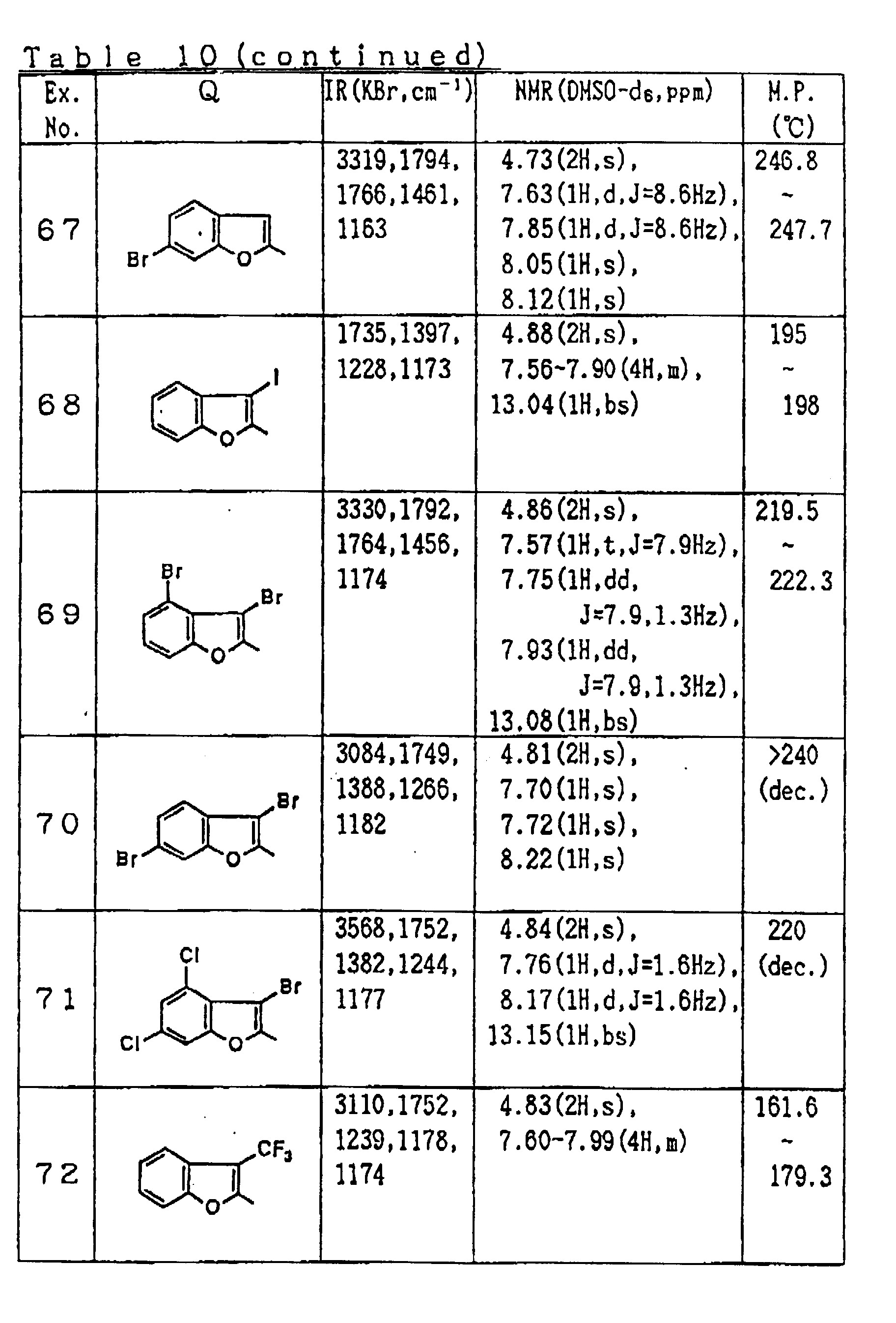

Now, typical but non-limiting examples of formulations of the compound of this invention will be shown below.

### Formulation A (Capsules)

Compound 1, 300 g of weight, 685 g of lactose and 15 g of magnesium stearate were weighed and mixed until the mixture became homogeneous. The mixture was then filled in No. 1 hard gelatin capsule at 200 mg each to obtain capsule preparation.

### Formulation B (Capsules)

Compound 23, 250 g of weight, 730 g of lactose and 20 g of magnesium stearate were weighed and mixed until the mixture became homogeneous. The mixture was then filled in No. 1 hard gelatin capsule at 200 mg each to obtain capsule preparation.

### Formulation C (Tablets)

Compound 19, 300 g of weight, 550 g of lactose, 120 g of potato starch, 15 g of polyvinyl alcohol and 15 g of magnesium stearate were weighed. The weighed amount of compound 19, lactose and potato starch were mixed until accomplishing homogeneity. Then aqueous solution of polyvinylalcohol was added to the mixture and granulated by wet process. The granules were then dried, mixed with magnesium stearate and pressed into tablets, each weighing 200 mg.

### Formulation D (Powder)

Compound 31, 200 g of weight, 790 g of lactose and 10 g of magnesium stearate were weighed and mixed until the mixture became homogeneous to obtain 20% powder preparation.

### Formulation E (Suppositories)

Compound 29, 100 g of weight were weighed and ground by a mortar until the compound became fine powder. Then 180 g of polyethylene glycol 1500 and 720 g of polyethylene glycol 4000 were added to the compound and melted. The mixture was then pressed at 1 g each to obtain suppository preparation.

## Claims (Claims for the following Contracting State(s): DE, GB, FR, IT, NL, SE, LI, CH, BE, AT, LU)

1. Hydantoin derivatives represented by the formula (I) : 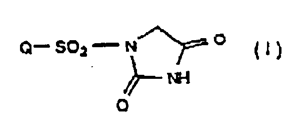 and non-toxic salts, solvates and solvates of non-toxic salts thereof; wherein Q represents a thienyl group, a benzothienyl group or a benzofuranyl group anyone of which may be substituted by one or more substituents which are same or different and selected from a group consisting at a halogen atom, a C1-C4 alkyl group, a nitro group, a cyano group, an optionally protected carboxy group, an optionally protected carboxymethyl group, a halogenated C1-C4 alkyl group, a C1-C4 alkylthio group, a C1-C4 alkylcarbonyl group, a C1-C4 alkoxy group, a C1-C4 alkylsulfinyl group, a C1-C4 alkylsulfonyl group, an optionally protected hydroxy group, an optionally protected amino group, a carbamoyl group and a phenyl group.

2. Hydantoin derivatives as claimed in claim 1 wherein Q represents a benzo[b]thien-2-yl group which may be substituted, and non-toxic salts, solvates and solvates of non-toxic salts thereof.

3. Hydantoin derivatives as claimed in claim 1 wherein Q represents a benzo[b]furan-2-yl group which may be substituted, and non-toxic salts, solvates and solvates of non-toxic salts thereof.

4. Hydantoin derivatives as claimed in claim 3 wherein the said substituents are 1 or 2 halogen atoms.

5. A process for producing hydantoin derivatives represented by the formula (I): 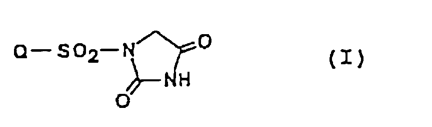 wherein Q has the same significance as defined in claim 1, by cyclization of a sulfonylglycine derivative represented by the formula (V) :
Q-SO₂NHCH₂CONH₂ (V)
wherein Q has the same significance as defined above, with a haloformic acid ester.

6. A process for producing hydantoin derivatives represented by the formula (I): 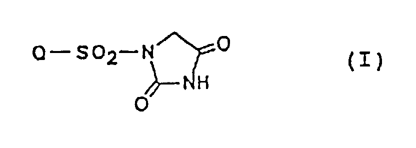 wherein Q has the same significance as defined in claim 1, by cyclizing a sulfonylglycine derivative represented by the formula (VI):
Q-SO₂NHCH₂CO-R¹ (VI)
wherein Q has the same significance as defined above, and R¹ represents a hydroxy group or an alkoxy group, with a thiocyanate derivative, then oxidizing the cyclized product.

7. A pharmaceutical composition which comprises at least one of hydantoin derivatives represented by the formula (I): 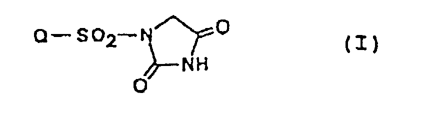 and non-toxic salts, solvates and solvates of non-toxic salts thereof; wherein Q has the same significance as defined in claim 1.

8. A pharmaceutical composition as claimed in claim 7 wherein Q represents a benzo[b]thien-2-yl group which may be substituted.

9. A pharmaceutical composition as claimed in claim 7 wherein Q represents a benzo[b]furan-2-yl group which may be substituted.

10. A pharmaceutical composition as claimed in claim 9 wherein the said substituents are 1 or 2 halogen atoms.

11. A sulfonylglycine derivative represented by the formula (IV):
Q-SO₂NHCH₂CO-R (IV)
wherein R represents a hydroxy group, an alkoxy group or an amino group which may be substituted with an alkoxycarbonyl group, O represents a thienyl group, a furyl group, a benzothienyl group or a benzofuranyl group anyone of which may be substituted by one or more substituents which are same or different and selected from a group consisting of a halogen atom, a C1-C4 alkyl group, a nitro group, a cyano group, an optionally protected carboxy group, an optionally protected carboxymethyl group, a halogenated C1-C4 alkyl group, a C1-C4 alkylthio group, a C1-C4 alkylcarbonyl group, a C1-C4 alkoxy group, a C1-C4 alkylsulfinyl group, a C1-C4 alkylsulfonyl group, an optionally protected hydroxy group, an optionally protected amino group, a carbamoyl group and a phenyl group; provided that Q does not represent an unsubstituted thienyl group, an optionally protected carboxy substituted thienyl group or an optionally protected carboxy substituted benzo[b]thienyl group, and that in case Q is a benzofuranyl group, Q represents a benzo[b]furan-2-yl group.

12. Intermediate compounds as claimed in claim 11 wherein Q represents a benzo[b]thien-2-yl group which may be substituted.

13. Intermediate compounds as claimed in claim 11 wherein Q represents a benzo[b]furan-2-yl group which may be substituted.

14. Interrnediate compounds as claimed in claim 13 wherein the said substituents are 1 to 3 halogen atoms.

## Claims (Claims for the following Contracting State(s): ES, GR)

1. A process for producing hydantoin derivatives represented by the formula (I): 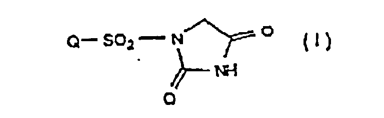 and non-toxic salts, solvates and solvates of non-toxic salts thereof; wherein Q represents a thienyl group, a benzothienyl group or a benzofuranyl group anyone of which may be substituted by one or more substituents which are same or different and selected from a group consisting of a halogen atom, a C1-C4 alkyl group, a nitro group, a cyano group, an optionally protected carboxy group, an optionally protected carboxymethyl group, a halogenated C1-C4 alkylgroup, a C1-C4 alkylthio group, a C1-C4 alkylcarbonyl group, a C1-C4 alkoxy group; a C1-C4 alkylsulfinyl group, a C1-C4 alkylsulfonyl group, an optionally protected hydroxy group, an optionally protected amino group, a carbamoyl group and a phenyl group,
comprising the cyclization of a sulfonylglycine derivative represented by the formula (V):
Q-SO₂NHCH₂CONH₂ (V)
wherein Q is defined as above with a haloformic acid ester.

2. Process for producing hydantoin derivatives represented by the formula (I): 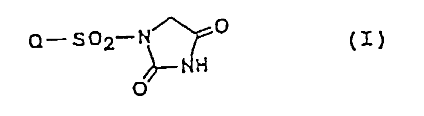 wherein Q is defined as in claim 1, comprising the cyclization of a sulfonylglycine derivative represented by formula (VI):
Q-SO₂NHCH₂-CO-R¹ (VI)
wherein Q is defined as above and R¹ represents a hydroxy group or an alkoxy group with a thiocyanate derivative and subsequently oxidizing the cyclized product.

3. Process according to claims 1 or 2, wherein Q represents a benzo[b]thien-2-yl group which may be substituted, and non-toxic salts, solvates and solvates of non-toxic salts thereof.

4. Process according to claim 1 or 2, wherein Q represents a benzo[b]furan-2-yl group which may be substituted, and non-toxic salts, solvates and solvates of non-toxic salts thereof.

5. Process according to claim 1 or 2, wherein the said substituents are 1 or 2 halogen atoms.

6. Use of a hydantoin derivative or a pharmaceutically acceptable salt thereof obtainable a by a process according to any one of the preceding claims for the manufacture of a medicament for inhibiting aldose reductase activity.

## Patentansprüche (Patentansprüche für folgende(n) Vertragsstaat(en): DE, GB, FR, IT, NL, SE, LI, CH, BE, AT, LU)

1. Hydantoin-Derivate mit der Formel (I): 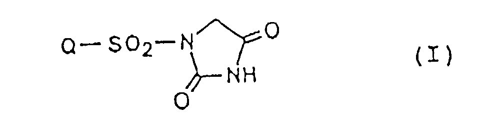 und nicht-toxische Salze, Solvate und Solvate von nicht-toxischen Salzen davon, worin Q eine Thienyl-Gruppe, eine Benzothienyl-Gruppe oder eine Benzofuranyl-Gruppe ist, die jeweils einen oder mehrere gleiche oder verschiedene Substituenten tragen können, ausgewählt aus der Gruppe bestehend aus Halogenatomen, C1-C4-Alkyl-Gruppen, Nitro-Gruppen, Cyano-Gruppen, wahlweise geschützten Carboxy-Gruppen, wahlweise geschützten Carboxymethyl-Gruppen, halogenierten C1-C4-Alkyl-Gruppen, C1-C4-Alkylthio-Gruppen, C1-C4-Alkylcarbonyl-Gruppen, C1-C4-Alkoxy-Gruppen, C1-C4-Alkylsulfinyl-Gruppen, C1-C4-Alkylsulfonyl-Gruppen, wahlweise geschützten Hydroxy-Gruppen, wahlweise geschützten Amino-Gruppen, Carbamoyl-Gruppen und Phenyl-Gruppen.

2. Hydantoin-Derivate gemäß Anspruch 1, in denen Q eine Benzo[b]tienyl-2-yl-Gruppe ist, die substituiert sein kann und nicht-toxische Salze, Solvate und Solvate von nicht-toxischen Salzen davon.

3. Hydantoin-Derivate gemäß Anspruch 1, in denen Q eine Benzo[b]furan-2-yl-Gruppe ist, die substituiert sein kann und nicht-toxische Salze, Solvate und Solvate von nicht-toxischen Salzen davon.

4. ydantoin-Derivate gemäß Anspruch 3, in denen die Substituenten 1 oder 2 Halogenatome sind.

5. Verfahren zur Herstellung von Hydantoin-Derivaten mit der Formel (I): 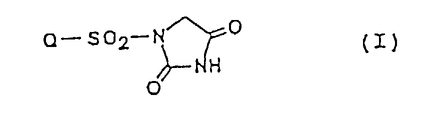 worin Q die gleiche Bedeutung wie in Anspruch 1 hat durch Cyclisierung von Sulfonylglycin-Derivaten mit der Formel (V):
Q-SO₂NHCH₂CONH₂ (V)
worin Q die gleiche Bedeutung wie oben hat mit einem halogenierten Ameisensäureester.

6. Verfahren zur Herstellung von Hydantoin-Derivatn mit der Formel (I): 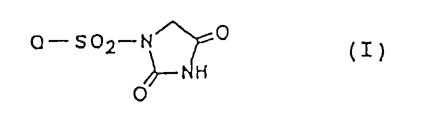 worin Q die gleiche Bedeutung wie in Anspruch 1 hat, durch Cyclisierung von Sulfonylglycin-Derivaten mit der Formel (VI):
Q-SO₂NHCH₂CO-R¹ (VI)
worin Q die gleiche Bedeutung wie oben hat und R¹ eine Hydroxy-Gruppe oder eine Alkoxy-Gruppe bedeutet mit einem Thiocyanat-Derivat und anschließendem Oxidieren des cyclisierten Produkts.

7. Pharmazeutische Zusammensetzung, umfassend wenigstens ein Hydantoin-Derivat mit der Formel (I): 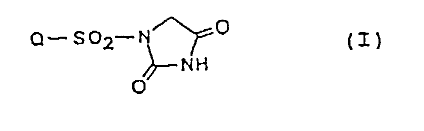 und nicht-toxische Salze, Solvate und Solvate von nicht-toxischen Salzen davon, worin Q die gleiche Bedeutung wie Anspruch 1 hat.

8. Pharmazeutische Zusammensetzung gemäß Anspruch 7, in der Q eine Benzo[b]thien-2-yl-Gruppe ist, die substituiert sein kann.

9. Pharmazeutische Zusammensetzung gemäß Anspruch 7, in der Q eine Benzo[b]furan-2-yl-Gruppe ist, die substituiert sein kann.

10. Pharmazeutische Zusammensetzung gemäß Anspruch 9, in der die Substituenten 1 oder 2 Halogenatome sind.

11. Sulfonylglycin-Derivate mit der Formel (IV)
Q-SO₂NHCH₂CO-R (IV)
worin R eine Hydroxy-Gruppe, eine Alkoxy-Gruppe oder eine Amino-Gruppe ist, die mit einer Alkoxycarbonyl-Gruppe substituiert sein kann, Q eine Thienyl-Gruppe, eine Furyl-Gruppe, eine Benzothienyl-Gruppe oder eine Benzofuranyl-Gruppe ist, die mit einem oder mehreren gleich oder verschiedenen Substituenten substituiert sein kann, ausgewählt aus der Gruppe bestehend aus Halogenatomen, C1-4-Alkyl-Gruppen, Nitro-Gruppen, Cyano-Gruppen, wahlweise geschützten Carboxy-Gruppen, wahlweise geschützten Carboxymethyl-Gruppen, halogenierten C1-C4-Alkyl-Gruppen, C1-C4-Alkylthio-Gruppen, C1-C4-Alkylcarbonyl-Gruppen, C1-C4-Alkoxy-Gruppen, C1-C4-Alkylsulfinyl-Gruppen, C1-C4-Alkylsulfonyl-Gruppen, wahlweise geschützten Hydroxy-Gruppen, wahlweise geschützten Amino-Gruppen, Carbamoyl-Gruppen und Phenyl-Gruppen, vorausgesetzt daß Q keine unsubstituierte Thienyl-Gruppe, wahlweise geschützte Carboxy-substituierte Thienyl-Gruppe oder wahlweise geschützt Carboxy-substituierte Benzo[b]thienyl-Gruppe ist und daß in dem Falle, daß Q eine Benzofuranyl-Gruppe ist, Q für Benzo[b]furan-2-yl steht.

12. Zwischenprodukt gemäß Anspruch 11, in dem Q eine Benzo[b]thien-2-yl-Gruppe ist, die substituiert sein kann.

13. Zwischenprodukt gemäß Anspruch 11, in dem Q eine Benzo[b]furan-2-yl-Gruppe ist, die substituiert sein kann.

14. Zwischenprodukt gemäß Anspruch 13, in dem die Substituenten 1 bis 3 Halogenatome sind.

## Patentansprüche (Patentansprüche für folgende(n) Vertragsstaat(en): ES, GR)

1. Verfahren zur Herstellung von Hydantoin-Derivaten mit der Formel (I): 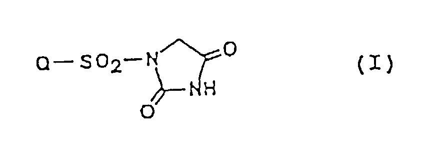 und nicht-toxischen Salzen, Solvaten und Solvaten von nicht-toxischen Salzen davon, worin Q eine Thienyl-Gruppe, eine Benzothienyl-Gruppe oder eine Benzofuranyl-Gruppe ist, die jeweils einen oder mehrere gleiche oder verschiedene Substituenten tragen kann, ausgewählt aus der Gruppe bestehend aus Halogenatomen, C1-C4-Alkyl-Gruppen, Nitro-Gruppen, Cyano-Gruppen, wahlweise geschützten Carboxy-Gruppen, wahlweise geschützten Carboxymethyl-Gruppen, halogenierten C1-C4-Alkyl-Gruppen, C1-C4-Alkylthio-Gruppen, C1-C4-Alkylcarbonyl-Gruppen, C1-C4-Alkoxy-Gruppen, C1-C4-Alkylsulfinyl-Gruppen, C1-C4-Alkylsulfonyl-Gruppen, wahlweise geschützten Hydroxy-Gruppen, wahlweise geschützten Amino-Gruppen, Carbamoyl-Gruppen und Phenyl-Gruppen, umfassen die Cyclisierung von Sulfonylglycin-Derivaten mit der Formel (V):
Q-SO₂NHCH₂CONH₂ (V)
worin Q wie oben definiert ist mit einem halogenierten Ameisensäureester.

2. Verfahren zur Herstellung von Hydantoin-Derivaten mit der Formel (I): 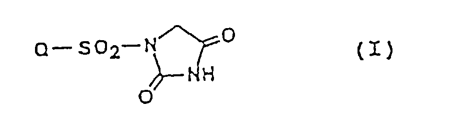 worin Q wie in Anspruch 1 definiert ist, umfassend die Cyclisierung von Sulfonylglycin-Derivaten mit der Formel (VI)
Q-SO₂NHCH₂-CO-R¹ (VI)
worin Q wie oben definiert ist und R¹ eine Hydroxy- oder Alkoxy-Gruppe ist, mit einem Thiocyanat-Derivat und anschließender Oxidierung des cyclisierten Produkts.

3. Verfahren gemäß einem der Ansprüche 1 oder 2, in dem Q eine Benzo[b]thien-2-yl-Gruppe ist, die substituiert sein kann und nicht-toxische Salze, Solvate und Solvate von nicht-toxischen Salzen davon.

4. Verfahren gemäß Anspruch 1 oder 2, in dem Q eine Benzo[b]furan-2-yl-Gruppe ist, die substituiert sein kann und nicht-toxische Salze, Solvate und Solvate von nicht-toxischen Salzen davon.

5. Verfahren gemäß Anspruch 1 oder 2, in dem die Substituenten 1 oder 2 Halogenatome sind.

6. Verwendung eines Hydantoin-Derivats oder eines pharmazeutisch verträglichen Salzes davon, erhältlich nach einem Verfahren gemäß der einem der vorstehenden Ansprüche zur Herstellung eines Medikaments zur Inhibierung von Aldosereduktase-Aktivität.

## Revendications (Revendications pour l'(les) Etat(s) contractant(s) suivant(s): DE, GB, FR, IT, NL, SE, LI, CH, BE, AT, LU)

1. Dérivés d'hydantoïne représentés par la formule (I) : 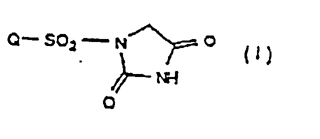 et sels, produits de solvatation non toxiques et produits de solvatation des sels non toxiques de ceux-ci, où Q représente un groupe thiényle, un groupe benzothiényle ou un groupe benzofuranyle, dont l'un quelconque peut être substitué par un ou plusieurs substituants qui sont identiques ou différents et sont choisis dans un groupe constitué d'un halogène, un groupe alkyle en C1 à C4, un groupe nitro, un groupe cyano, un groupe carboxyle facultativement protégé, un groupe carboxyméthyle facultativement protégé, un groupe alkyle en C1 à C4 halogéné, un groupe alkylthio en C1 à C4, un groupe alkylcarbonylé en C1 à C4, un groupe alcoxyle en C1 à C4, un groupe alkylsulfinyle en C1 à C4, un groupe alkylsulfonyle en C1 à C4, un groupe hydroxyle facultativement protégé, un groupe amino facultativement protégé, un groupe carbamoyle et un groupe phényle.

2. Dérivés d'hydantoïne selon la revendication 1, dans lesquels Q représente un groupe benzo[b]thién-2-yle qui peut être substitué et les sels, produits de solvatation non toxiques et produits de solvatation des sels non toxiques de ceux-ci.

3. Dérivés d'hydantoïne selon la revendication 1, dans lesquels Q représente un groupe benzo[b]furan-2-yle qui peut être substitué et les sels, produits de solvatation non toxiques et produits de solvatation des sels non toxiques de ceux-ci.

4. Dérivés d'hydantoïne selon la revendication 3, dans lesquels lesdits substituants sont 1 ou 2 halogènes.

5. Procédé de production de dérivés d'hydantoïne représentés par la formule (I) : 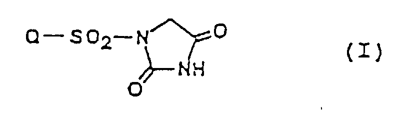 où Q a la même signification que définie dans la revendication 1, par cyclisation d'un dérivé de la sulfonylglycine représenté par la formule (V) :
Q-SO₂NHCH₂CONH₂ (V)
où Q a la même signification que précédemment définie avec un ester d'acide halogénoformique.

6. Procédé de production des dérivés d'hydantoïne représentés par la formule (I) : 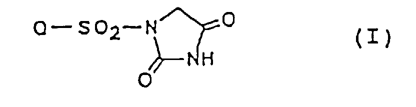 où Q a la même signification que définie dans la revendication 1, par les étapes de cyclisation d'un dérivé de la sulfonylglycine représenté par la formule (VI) :
Q-SO₂NHCH₂CO-R¹ (VI)
où Q a la même signification que précédemment définie et R¹ représente un groupe hydroxyle ou un groupe alcoxyle avec un dérivé du thiocyanate, et d'oxydation du produit cyclisé.

7. Composition pharmaceutique qui comprend au moins l'un des dérivés d'hydantoïne représentés par la formule (I) : 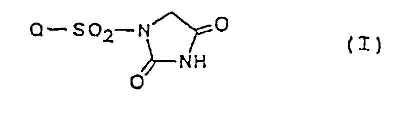 et les sels, les produits de solvatation non toxiques et les produits de solvatation des sels non toxiques de ceux-ci; où Q a la même signification que définie à la revendication 1.

8. Composition pharmaceutique selon la revendication 7, dans laquelle Q représente un groupe benzo[b]thién-2-yle qui peut être substitué.

9. Composition pharmaceutique selon la revendication 7, dans laquelle Q représente un groupe benzo[b]furan-2-yle qui peut être substitué.

10. Composition pharmaceutique selon la revendication 9, dans laquelle lesdits substituants sont 1 ou 2 halogènes.

11. Dérivé de la sulfonylglycine représenté par la formule (IV) :
Q-SO₂NHCH₂CO-R (IV)
où R représente un groupe hydroxyle, un groupe alcoxyle ou un groupe amino qui peut être substitué par un groupe alcoxycarbonyle, Q représente un groupe thiényle, un groupe furyle, un groupe benzothiényle ou un groupe benzofuranyle, dont l'un quelconque peut être substitué par un ou plusieurs substituants qui sont identiques ou différents et sont choisis dans un groupe constitué d'un halogène, un groupe alkyle en C1 à C4, un groupe nitro, un groupe cyano, un groupe carboxyle facultativement protégé, un groupe carboxyméthyle facultativement protégé, un groupe alkyle en C1 à C4 halogéné, un groupe alkylthio en C1 à C4, un groupe alkylcarbonyle en C1 à C4, un groupe alcoxyle en C1 à C4, un groupe alkylsulfinyle en C1 à C4, un groupe alkylsulfonyle en C1 à C4, un groupe hydroxyle facultativement protégé, un groupe amino facultativement protégé, un groupe carbamoyle et un groupe phényle, à la condition que Q ne représente pas un groupe thiényle non substitué, un groupe thiényle substitué par un carboxyle facultativement protégé ni un groupe benzo[b]thiényle substitué par un carboxyle facultativement protégé et que, dans le cas où Q est un groupe benzofuranyle, Q représente un groupe benzo[b]furan-2-yle.

12. Composés intermédiaires selon la revendication 11, dans lesquels Q représente un groupe benzo[b]thién-2-yle qui peut être substitué.

13. Composés intermédiaires selon la revendication 11, dans lesquels Q représente un groupe benzo[b]furan-2-yle qui peut être substitué.

14. Composés intermédiaires selon la revendication 13, dans lesquels lesdits substituants sont 1 à 3 halogènes.

## Revendications (Revendications pour l'(les) Etat(s) contractant(s) suivant(s): ES, GR)

1. Procédé pour la production de dérivés d'hydantoïne représentés par la formule (I) : 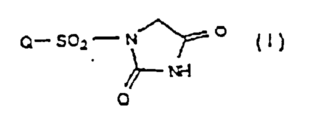 et de sels, de produits de solvatation non toxiques et de produits de solvatation des sels non toxiques de ceux-ci ; où Q représente un groupe thiényle, un groupe benzothiényle ou un groupe benzofuranyle dont l'un quelconque peut être substitué par un ou plusieurs substituants qui sont identiques ou différents et sont choisis dans un groupe constitué d'un halogène, un groupe alkyle en C1 à C4, un groupe nitro, un groupe cyano, un groupe carboxyle facultativement protégé, un groupe carboxyméthyle facultativement protégé, un groupe alkyle en C1 à C4 halogéné, un groupe alkylthio en C1 à C4, un groupe alkylcarbonyle en C1 à C4, un groupe alcoxyle en C1 à C4, un groupe alkylsulfinyle en C1 à C4, un groupe alkylsulfonyle en C1 à C4, un groupe hydroxyle facultativement protégé, un groupe amino facultativement protégé, un groupe carbamoyle et un groupe phényle, comprenant l'étape de cyclisation d'un dérivé de la sulfonylglycine représenté par la formule (V) :
Q-SO₂NHCH₂CONH₂ (V)
où Q est défini comme précédemment avec un ester d'acide halogénoformique.

2. Procédé de production des dérivés d'hydantoïne représentés par la formule (I) : 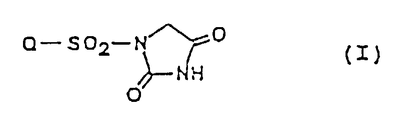 où Q est défini selon la revendication 1, comprenant l'étape de cyclisation d'un dérivé de la sulfonylglycine représenté par la formule (VI) :
Q-SO₂NHCH₂CO-R¹ (VI)
où Q est défini comme précédemment et R¹ représente un groupe hydroxyle ou un groupe alcoxyle avec un dérivé du thiocyanate et une étape ultérieure d'oxydation du produit cyclisé.

3. Procédé selon la revendication 1 ou 2, dans lequel Q représente un groupe benzo[b]thién-2-yle qui peut être substitué et les sels, les produits de solvatation non toxiques et les produits de solvatation des sels non toxiques de ceux-ci.

4. Procédé selon la revendication 1 ou 2, dans lequel Q représente un groupe benzo[b]furan-2-yle qui peut être substitué et les sels, les produits de solvatation non toxiques et les produits de solvatation des sels non toxiques de ceux-ci.

5. Procédé selon la revendication 1 ou 2, dans lequel lesdits substituants sont 1 ou 2 halogènes.

6. Utilisation d'un dérivé d'hydantoïne ou de l'un de ses sels pharmaceutiquement acceptables qui peut être obtenu par un procédé selon l'une quelconque des revendications précédentes pour la fabrication d'un médicament destiné à inhiber l'action de l'aldose réductase.
